# EUROPEAN PATENT SPECIFICATION

(11) **EP 1 453 539 B1**
(45) Date of publication and mention of the grant of the patent: **19.11.2008**
(21) Application number: 02783303.7
(22) Date of filing: 05.12.2002
(51) Int. Cl.: A61K 39/35

(54) **IMMUNOTHERAPEUTIC METHODS AND SYSTEMS**
IMMUNOTHERAPEUTISCHE METHODEN UND SYSTEME
PROCEDES ET SYSTEMES IMMUNOTHERAPEUTIQUES

(30) Priority: 05.12.2001 US 338385 P
(43) Date of publication of application: 08.09.2004
(73) Proprietor: Circassia Limited, Oxford, Oxfordshire OX4 4GA (GB)
(72) Inventor: LARCHE, Mark, Surrey KT4 8JJ (GB); LEDGER, Philip William, Bedford MK43 7LU (GB)
(74) Representative: Woods, Geoffrey Corlett
(86) International application number: PCT/GB2002/005548
(87) International publication number: WO 2003/047618

(56) References cited:
- WO-A-94/24281
- WO-A-95/26980
- WO-A-99/34826
- VAN NEERVEN R J J ET AL: "CHARACTERIZATION OF CAT DANDER-SPECIFIC T LYMPHOCYTES FROM ATOPIC PATIENTS" JOURNAL OF IMMUNOLOGY, THE WILLIAMS AND WILKINS CO. BALTIMORE, US, vol. 152, 1994, pages 4203-4210, XP002104747 ISSN: 0022-1767

## Description

### Field of the Invention

The present invention relates to immunotherapeutic methods and systems and, in particular, it relates to methods of desensitising an individual to polypeptide antigens, particularly to polypeptide allergens.

### Background of the Invention

The ability of the immune system to elicit a response to a particular molecule depends critically upon its ability to recognise the presence of an antigen. Classically, the term antigen has been associated with the ability of a molecule to be an antibody generator via induction of B-cells. It is now known, however, that T cells also possess the ability to recognise antigens. T-cell antigen recognition requires antigen presenting cells (APCs) to present antigen fragments (peptides) on their cell surface in association with molecules of the major histocompatibility complex (MHC). T cells use their antigen specific T-cell receptors (TCRs) to recognise the antigen fragments presented by the APC. Such recognition acts as a trigger to the immune system to generate a range of responses to eradicate the antigen which has been recognised.

T lymphocytes have been implicated in the pathogenesis of a wide variety of diseases involving immune recognition of antigens derived both from the internal (host) and external environments. Autoimmune diseases such as autoimmune thyroiditis, rheumatoid arthritis and lupus erythrematosus arise from the recognition by the immune system of host, or self, antigens.

Recognition of external antigens by the immune system of an organism, such as man, can in some cases result in diseases, known as atopic conditions. An example of the latter are the allergic diseases including asthma, atopic dermatitis and allergic rhinitis. In this group of diseases, B lymphocytes generate antibodies of the IgE class (in humans) which bind externally derived antigens, which are referred to in this context as allergens, since these molecules elicit an allergic response. Production of allergen-specific IgE is dependent upon T lymphocytes which are also activated by (are specific for) the allergen. Allergen-specific IgE antibodies bind to the surface of cells such as basophils and mast cells by virtue of the expression by these cells of surface receptors for IgE. Crosslinking of surface bound IgE molecules by allergen results in degranulation of these effector cells causing release of inflammatory mediators such as histamine, 5-hydroxtryptamine and lipid mediators such as the sulphidoleukotrienes. In addition to IgE-dependent events, certain allergic diseases such as asthma are characterised by IgE-independent events. It has been demonstrated that the induction of the late phase reaction is at least in part, an IgE-independent event which is dependent upon the activation of allergen-specific T lymphocytes.

Allergic IgE-mediated diseases are currently normally treated with agents which provide symptomatic relief, or by prevention. Examples of such agents are antihistamines, β₂ agonists, and glucocorticosteroids. In addition, some IgE-mediated diseases are treated by desensitisation procedures that involve the periodic injection of allergen components or extracts. Desensitisation treatments may induce an IgG response that competes with IgE for allergen, or they may induce specific suppressor T cells that block the synthesis of IgE directed against allergen. This form of treatment is not always effective and poses the risk of provoking serious side effects, particularly general anaphylactic shock. This can be fatal unless recognised immediately and treated with adrenaline.

WO 99/34826 discloses a method of desensitising a patient to a polypeptide allergen comprising administering a peptide derived from the allergen wherein restriction to a MHC Class II molecule possessed by the patient can be demonstrated by the peptide and the peptide is able to induce a late phase response in an individual who possesses the said MHC Class II molecule.

WO 94/24281 discloses T cell epitopes of the major allergens from Dermatophagoides (house dust mite).

### Summary of the Invention

The invention provides use of
(i) a polypeptide comprising a whole first polypeptide antigen sequence which comprises a T cell epitope or a polypeptide comprising a portion of the first polypeptide antigen sequence, which portion comprises a T cell epitope, or
(ii) a polypeptide comprising a whole second polypeptide antigen sequence or a polypeptide comprising a portion of the second polypeptide antigen sequence, which portion contains a T cell epitope, for the manufacture of a medicament for desensitising an individual to the second polypeptide antigen by a method comprising:
   (a) administering a primary composition comprising the polypeptide comprising the whole first polypeptide antigen sequence or the polypeptide comprising the portion of the first polypeptide antigen sequence to the individual in a manner sufficient to generate a hyporesponsive state against the first polypeptide antigen sequence; and
   (b) administering a secondary composition to the individual, wherein said secondary composition comprises the polypeptide comprising the whole second antigen sequence or the polypeptide comprising the portion of the second polypeptide sequence whereby the hyporesponsive state generated in step (a) and administration of the secondary composition are sufficient to desensitise the individual to the second polypeptide antigen, and wherein the first polypeptide antigen is different from the second polypeptide antigen, and wherein the individual has previously been exposed to the first or second polypeptide antigen.

The invention also provides products containing
(i) a polypeptide comprising a whole first antigen sequence which comprises a T cell epitope or a polypeptide comprising a portion of the first polypeptide antigen sequence, which portion comprises a T cell epitope, and
(ii) a polypeptide comprising a whole second antigen sequence or a polypeptide comprising a portion of the second polypeptide antigen sequence, which portion contains a T cell epitope, as a combined preparation for simultaneous, separate or sequential use in a method of desensitising the individual to the second polypeptide antigen comprising steps (a) and (b) as defined above, and wherein the first polypeptide antigen is different from the second polypeptide antigen, and wherein the individual has previously been exposed to the first or second polypeptide antigen.

In certain aspects, the individual is allergic to the first polypeptide antigen. In other aspects, the first polypeptide antigen is an aero allergen. In a particularly preferred embodiment, the primary composition comprises one or more peptides the primary composition comprises one or more Fel d 1 peptides selected from the group consisting of EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12), and peptides substantially homologous to any one or more of SEQ ID NOs 1-12.

The second polypeptide antigen can be an allergen obtained or derived from any one or more of the following sources: latex; plants such as grass, tree, and ragweed; pollens; fungi and moulds; foods; stinging insects including wasps; the chironomidae (non-biting midges); spiders and mites; flies such as housefly, fruit fly, sheep blow fly and screw worm fly; grain weevil; silkworm; honeybee; non-biting midge larvae; bee moth larvae; mealworm; cockroach; larvae of *Tenibrio molitor* beetle; and mammals such as cat, dog, horse, cow, pig, sheep, rabbit, rat, guinea pig, mice and gerbil.

In some embodiments, the second polypeptide antigen is an allergen and is selected from the group consisting of Der p1; Der p 2; Der p 3; Der p 4; Der p 5; Der p 6; Der p 7; Der p 9; Der f 1; Der f 2; Der f 3; Der f 4; Der f 7; Fel d 1 chain 1 or 2; Hev b 1; Hev b 3; Lol p 1; Lol p 2a; Lol p 3; Lol p 5a; Lol p 5b; Lol p isoform 9; Lol p 11; Ole e 1; Par j P2; Par j P5; Par j P8; Par j P9; Par j 1; Phl p 1; Phl p 2; Phl p 5; Phl p 5b; Phl p 5a; VES V 5; VES M 1; VES V 1; VES V 2; VES VI; Bet v 1; Bet v 2; Bet v 3; Bet v 4; Que a I; Car b I; Aln g I; Rubisco; Ara h 1; Amb a 1; Amb a 2; Amb a 1.3; Amb a 1.2; Amb a 1.1; Cry j IB precursor; Cry j IA precursor; Cry j II precursor; Cry j II protein; Cry j I precursor; Can f 1; Can f 2; serum albumin fragment; Equ c1; Equ c 2; Eur m 1; POA P 9; Cr p1; Cr p2; Bla g 2; Bla g 4; and Bla g 5.

In other embodiments, the second polypeptide antigen is obtained or derived from an autoantigen. In certain embodiments, the autoantigen is associated with any one of the following autoimmune disorders: Multiple Sclerosis; Diabetes; Rheumatoid Arthritis; Thyroiditis; Systemic Lupus Erthromatosus; Behcet's Disease; Coeliac Disease; or Myasthenia gravis. In particular preferred embodiments, the autoantigen is selected from the group consisting of: myelin basic protein (MBP); proteolipid protein (PLP); myelin oligodendrocyte glycoprotein (MOG); glutamic acid decarboxylase (GAD); insulin; IA-2 (a protein phosphatase-like molecule); collagen; heat shock proteins (HSP's); thyroglobulin; histone proteins; immunoglobulin heavy chain; S antigen from the eye (Sag); HLA-B44; HLA B51; HSP65; gliadin; and acetyl choline receptor. In still further embodiments, the second polypeptide antigen is obtained or derived from a transplant antigen.

The compositions administered in the practice of the invention can be administered in a slow release formulation or in an oil and water emulsion. In addition, the compositions can be administered using intradermal injection, subcutaenous injection, intramuscular injection, intravenous injection, transdermal, intranasal, oral, intraocular, or intrathecal administration technique. In a preferred embodiment, one or more of the compositions is provided in a dry, powdered form and is administered using a transdermal particle injection technique.

It is an advantage of the present invention that individuals who are multiply allergic to different allergens can be treated in a single, time-saving method. It is also an advantage of the present invention that a mild antigen (e.g., an aero allergen) can be used to establish a tolergeneic environment that then enables a more severe antigen (e.g., a nut allergen) to be administered and take advantage of the environment in a desensitisation to that more severe antigen.

### Brief Description of the Figures

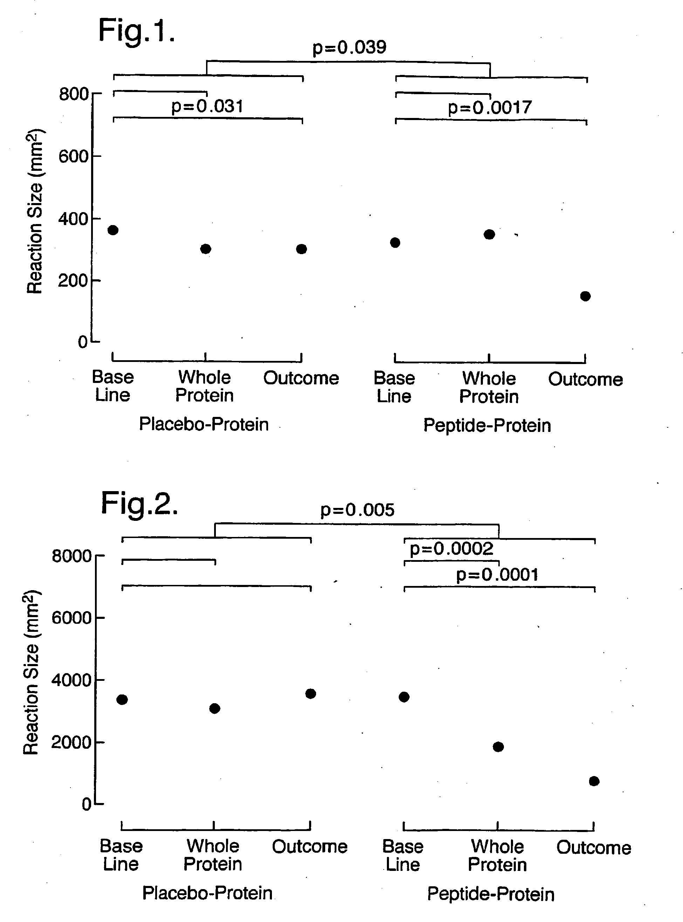

**Figure 1****.** *Administration of peptides followed by whole protein reduces the cutaneous early phase reaction to Fel d1*. In a placebo-controlled, double blind clinical trial, subjects were injected intradermally with whole Fel d 1 protein and the size of the reaction at 15 minutes measured (baseline). A series of injections were administered to test subjects, wherein the injections were carried out using compositions of peptides derived from the amino acid sequence of Fel d 1. The dose of peptides started at 5 µg of each peptide and increased until a cumulative dose of 90 µg had been administered. Approximately 2-4 weeks later the whole Fel d 1 protein was injected (whole protein). 3-6 months later whole protein challenge was performed and the magnitude of the skin reaction at 15 minutes defined (outcome).
**Figure 2****.** *Administration of peptides followed by whole protein reduces the cutaneous late-phase reaction to Fel d 1*. In a placebo-controlled, double blind clinical trial, subjects were injected intradermally with whole Fel d 1 protein and the size of the reaction at 6 hours measured (baseline). A series of injections were administered to test subjects, wherein the injections were carried out using compositions of peptides derived from the amino acid sequence of Fel d 1. The dose of peptides started at 5 µg of each peptide and increased until a cumulative dose of 90 µg had been administered. Approximately 2-4 weeks later the whole Fel d 1 protein was injected (whole protein). 3-6 months later whole protein challenge was performed and the magnitude of the skin reaction at 6 hours defined (outcome).

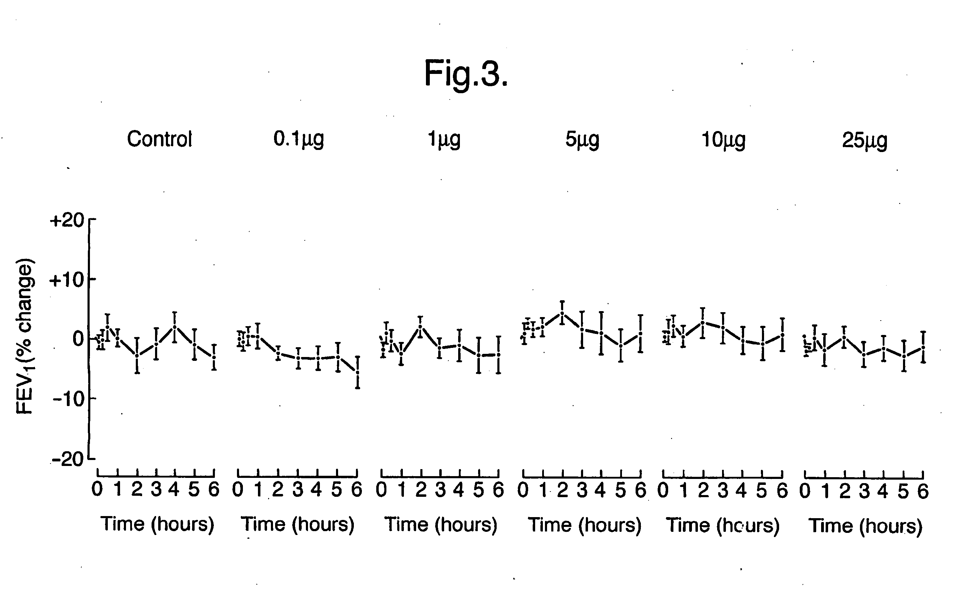

**Figure 3****.** Peptides from the cat allergen Fel d 1 were administered intradermally at 2 weekly intervals in the following dose schedule: 0.1 µg, 1.0 µg, 5.0 µg, 10.0 µg, 25.0 µg. No isolated late asthmatic reactions were observed (n = 8 subjects; pooled data). The Fel d 1 peptides that were administered were: EICPAVKRDVDLFLTGT (SEQ ID NO. 1); LFLTGTPDEYVEQVAQY (SEQ ID NO. 2); EQVAQYKALPVVLENA (SEQ ID NO. 3); KALPVVLENARILKNCV (SEQ ID NO. 4); RILKNCVDAKMTEEDKE (SEQ ID NO. 5); KMTEEDKENALSLLDK (SEQ ID NO. 6); KENALSLLDKIYTSPL (SEQ ID NO. 7); LTKVNATEPERTAMKK (SEQ ID NO. 8); TAMKKIQDCYVENGLI (SEQ ID NO. 9); SRVLDGLVMTTISSSK (SEQ ID NO. 10); ISSSKDCMGEAVQNTV (SEQ ID NO. 11); and AVQNTVEDLKLNTLGR (SEQ ID NO. 12).

### Detailed Description of the Invention

Before describing the present invention in detail, it is to be understood that this invention is not limited to particularly exemplified molecules, methods or process parameters as such may, of course, vary. It is also to be understood that the terminology used herein is for the purpose of describing particular embodiments of the invention only, and is not intended to be limiting. In addition, the practice of the present invention will employ, unless otherwise indicated, conventional methods of virology, microbiology, molecular biology, recombinant DNA techniques and immunology all of which are within the ordinary skill of the art. Such techniques are explained fully in the literature. See, e.g., Sambrook, et al., Molecular Cloning: A Laboratory Manual (2nd Edition, 1989); DNA Cloning: A Practical Approach, vol. I & II (D. Glover, ed.); Oligonucleotide Synthesis (N. Gait, ed., 1984); A Practical Guide to Molecular Cloning (1984); and Fundamental Virology, 2nd Edition, vol. I & II (B.N. Fields and D.M. Knipe, eds.).

It must be noted that, as used in this specification and the appended claims, the singular forms "a," "an." and "the" include plural referents unless the content clearly dictates otherwise.

### A. Definitions

Unless defined otherwise, all technical and scientific terms used herein have the same meaning as commonly understood by one of ordinary skill in the art to which the invention pertains. Although a number of methods and materials similar or equivalent to those described herein can be used in the practice of the present invention, the preferred materials and methods are described herein.

In describing the present invention, the following terms will be employed, and are intended to be defined as indicated below.

By "needleless syringe" is meant an instrument which delivers a particulate (e.g., powdered) composition transdermally without the aid of a conventional needle to pierce the skin. Needleless syringes for use with the present invention are discussed throughout this document, and are used to carry out transdermal particle injection techniques.

The term "transdermal," delivery intends intradermal (e.g., into the dermis or epidermis), transdermal, (e.g., "percutaneous") and transmucosal administration, i.e., delivery by passage of an agent into or through skin or mucosal tissue. *See, e.g.,* Transdermal Drug Delivery: Developmental Issues and Research Initiatives, Hadgraft and Guy (eds.), Marcel Dekker, Inc., (1989); Controlled Drug Delivery: Fundamentals and Applications, Robinson and Lee (eds.), Marcel Dekker Inc., (1987); and Transdermal Delivery of Drugs, Vols. 1-3, Kydonieus and Berner (eds.), CRC Press, (1987). Thus, the term encompasses delivery from a needleless syringe deliver as described in U.S. Patent No. 5,630,796, as well as particle-mediated delivery as described in U.S. Patent No. 5,865,796.

A "peptide," used interchangeably herein with the term "polypeptide," is used in it broadest sense to refer to a composition of two or more subunit amino acids, amino acid analogs, or other peptidomimetics. The subunits may be linked by peptide bonds or by other bonds, for example ester, ether, etc. As used herein, the term "amino acid" refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is typically called a polypeptide or a protein.

An "antigen" refers to any agent, generally a macromolecule, which can elicit an immunological response in an individual. The term may be used to refer to an individual macromolecule or to a homogeneous or heterogeneous population of antigenic macromolecules. Antigens include allergens and autoantigens. As used herein, "antigen" is generally used to refer to a polypeptide molecule or portion thereof which contains one or more epitopes. For purposes of the present invention, antigens can be obtained or derived from any appropriate source. Furthermore, for purposes of the present invention, an "antigen" includes a polypeptide having modifications, such as deletions, additions and substitutions (generally conservative in nature) to the native sequence, so long as the polypeptide maintains sufficient immunogenicity. These modifications may be deliberate, for example through site-directed mutagenesis, or may be accidental, such as through mutations of hosts which produce the antigens.

In various aspects of the invention, an antigen is described as a molecule containing one or more T cell epitopes. A "T cell epitope" refers generally to those features of a peptide structure which are capable of inducing a T cell response. In this regard, it is accepted in the art that T cell epitopes comprise linear peptide determinants that assume extended conformations within the peptide-binding cleft of MHC molecules, (Unanue et al. (1987) Science 236:551-557). As used herein, a T cell epitope is generally a peptide having at least about 3-5 amino acid residues, and preferably at least 5-10 or more amino acid residues. The ability of a particular antigen to stimulate a cell-mediated immunological response may be determined by a number of well-known assays, such as by lymphoproliferation (lymphocyte activation) assays, CTL cytotoxic cell assays, or by assaying for T-lymphocytes specific for the antigen in a sensitized subject. See, e.g., Erickson et al. (1993) J. Immunol. 151:4189-4199; and Doe et al. (1994) Eur. J. Immunol. 24:2369-2376.

An "allergen" is an antigen which can initiate a state of hypersensitivity or which can provoke an immediate hypersensitivity reaction in an individual already sensitized with the allergen. Allergens are commonly proteins or chemicals bound to proteins which have the property of being allergenic; however, allergens can also include organic or inorganic materials derived from a variety of man-made or natural sources such as plant materials, metals, ingredients in cosmetics or detergents, latexes, or the like. Allergens can elicit any type of hypersensitivity reaction in a sensitized individual. For example, penicillin allergies can manifest as all four types (Type I-IV) of hypersensitivity reactions, contact dermatitis can manifest as a Type IV reaction, and gluten allergy can manifest as a Type III reaction. However, allergens typically are associated with Type I immediate hypersensitivity reactions in sensitized individuals.

Protein or peptide allergens are typically molecules that comprise a region derived or obtained from known sources, including but not limited to protein allergens specific for the genus Dermatophagoides; the genus Euroglyphus; the genus Felis; the genus Ambrosia; the genus Lolium; the genus Phleum; the genus Cryptomeria; the genus Alternaria; the genus Alnus; the genus Betula; the genus Carpinus; the genus Quercus: the genus Olea; the genus Artemisia; the genus Plantago; the genus Parietaria; the genus Canine; the genus Blattella, the genus Apis; the genus Rubisco; the genus Vespula; and the genus Periplaneta.

Techniques for determining nucleic acid and amino acid "sequence identity" or "sequence homology" also are known in the art. Typically, such techniques include determining the nucleotide sequence of the mRNA for a gene and/or determining the amino acid sequence encoded thereby, and comparing these sequences to a second nucleotide or amino acid sequence. In general, "identity" refers to an exact nucleotide-to-nucleotide or amino acid-to-amino acid correspondence of two polynucleotides or polypeptide sequences, respectively. Two or more sequences (polynucleotide or amino acid) can be compared by determining their "percent identity." The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman (1981) Advances in Applied Mathematics 2:482-489. This algorithm can be applied to amino acid sequences by using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov (1986) Nucl. Acids Res. 14(6):6745-6763. An exemplary implementation of this algorithm to determine percent identity of a sequence is provided by the Genetics Computer Group (Madison, WI) in the "BestFit" utility application. The default parameters for this method are described in the Wisconsin Sequence Analysis Package Program Manual, Version 8 (1995) (available from Genetics Computer Group, Madison, WI). A preferred method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff= 60; expect =10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-bin/BLAST.

Alternatively, homology can be determined by hybridization of polynucleotides under conditions which form stable duplexes between homologous regions, followed by digestion with single-stranded-specific nuclease(s), and size determination of the digested fragments. Two DNA, or two polypeptide sequences are "substantially homologous" to each other when the sequences exhibit at least about 80%-85%, preferably at least about 90%, and most preferably at least about 95%-98% sequence identity over a defined length of the molecules, as determined using the methods above. As used herein, substantially homologous also refers to sequences showing complete identity to the specified DNA or polypeptide sequence. DNA sequences that are substantially homologous can be identified in a Southern hybridization experiment under, for example, stringent conditions, as defined for that particular system. For example, stringent hybridization conditions can include 50% formamide, 5x Denhardt=s Solution, 5x SSC, 0.1% SDS and 100 µg/ml denatured salmon sperm DNA and the washing conditions can include 2x SSC, 0.1% SDS at 37°C followed by 1x SSC, 0.1% SDS at 68°C. Defining appropriate hybridization conditions is within the skill of the art. See, e.g., Sambrook et al., *supra*; *DNA Cloning, supra; Nucleic Acid Hybridization, supra.*

As used herein, the term "autoimmune disease" means a set of sustained organ-specific or systemic clinical symptoms and signs associated with altered immune homeostasis that is manifested by qualitative and/or quantitative defects of expressed autoimmune repertoires (Autoimmunity Physiology and Disease, Coutinho and Kazatchkine, eds, Wiley-Liss, 1993, Chapter 27, page 433). Autoimmune diseases are characterized by cytotoxic immune responses to epitopes on self antigens natively found in the diseased individual. The immune system of the individual then activates an inflammatory cascade aimed at cells and tissues presenting those specific self antigens. The destruction of the antigen, tissue, cell type, or organ attacked by the individual's own immune system gives rise to the symptoms of the disease. Clinically significant autoimmune diseases include, for example, rheumatoid arthritis, multiple sclerosis, juvenile-onset diabetes, systemic lupus erythematosus, autoimmune uveoretinitis, autoimmune vasculitis, bullous pemphigus, myasthenia gravis, autoimmune thyroiditis or Hashimoto's disease, Sjogren's syndrome, granulomatous orchitis, autoimmune oophoritis, Crohn's disease, sarcoidosis, rheumatic carditis, ankylosing spondylitis, Grave's disease, and autoimmune thrombocytopenic purpura. *See* e.g., Paul, W.E. (1993) Fundamental Immunology, Third Edition, Raven Press, New York, Chapter 30, pp. 1033-1097; and Cohen et al. (1994) Autoimmune Disease Models, A Guidebook, Academic Press, 1994.

The term "self antigen," which is used interchangeably herein with the term "autoantigen," means an antigen, or a molecule capable of being recognized during an immune response, that is normally part of the individual. This is in contrast with antigens which are foreign, or exogenous, which are not normally part of the individual's milieu. Each autoimmune disease is characterized by an immune response directed at a self antigen. Normally, there are no active immune responses to self antigens, and no symptoms appear. With the development of an immune response to a self antigen, autoimmune diseases may appear. Autoimmune diseases present clinically with different symptoms depending upon the specific self antigen against which an immune response is raised. This immune response results in the destruction of the structure containing the self antigen, and it is the loss of that structure with concurrent loss of that structure's normal function which results in symptoms of autoimmune disease.

Compositions that comprise an antigen or allergen are typically prepared as pharmaceutical compositions which can contain one or more added materials such as carriers, vehicles, and/or excipients. "Carriers," "vehicles" and "excipients" generally refer to substantially inert materials which are non-toxic, not pharmacologically active and do not interact with other components of the composition in a deleterious manner. These materials can be used to increase the amount of solids in particulate compositions. Examples of suitable carriers or vehicles include water, silicone, gelatin, waxes, and like materials. Examples of normally employed excipients include pharmaceutical grades of dextrose, sucrose, lactose, trehalose, mannitol, sorbitol, inositol, dextran, starch, cellulose, sodium or calcium phosphates, calcium carbonate, calcium sulfate, sodium citrate, citric acid, tartaric acid, glycine, high molecular weight polyethylene glycols (PEG), and combinations thereof.

The terms "individual" and "subject" are used interchangeably herein to refer to any member of the subphylum cordata, including, without limitation, humans and other primates, including non-human primates such as chimpanzees and other apes and monkey species; mammals including for example farm animals such as cattle, sheep, pigs, goats and horses, domestic mammals such as dogs and cats, and laboratory animals including rodents such as mice, rats and guinea pigs; birds, including domestic, wild and game birds such as chickens, turkeys and other gallinaceous birds, ducks, geese, and the like. The terms do not denote a particular age. Thus, both adult and newborn individuals are intended to be covered. The methods described herein are intended for use in any of the above vertebrate species, since the immune systems of all of these vertebrates operate similarly.

### B. General Overview

There exists a need for alternative therapeutic strategies to desensitise subjects to polypeptide antigens, particularly allergens. In particular, since many individuals are allergic, or may require desensitising to several polypeptide antigens, it would be beneficial to provide systems that allow for desensitisation to multiple polypeptide antigens, and in particular multiple allergens.

More particularly, we have found, surprisingly, that "tolerance" induced in an individual to a first polypeptide antigen or allergen can create in the individual a "tolergeneic environment" wherein inappropriate immune responses to other antigens can be downregulated in order to provide tolerance to other antigens. This surprising finding means that individuals allergic to multiple allergens can be treated in a greatly reduced time period, and that individuals seriously allergic to some allergens (e.g., peanuts) but more mildly allergic to other allergens (e.g., cat dander) can benefit from a therapy wherein tolerance to the milder allergen is established and then this tolergeneic environment is used to provide tolerance to the other, more extreme allergen. In addition, individuals suffering from an autoimmune disorder who are additionally sensitised (or otherwise immune) to an unrelated antigen or allergen can benefit from a treatment regime wherein tolerance to the unrelated antigen or allergen is first established and then this tolergeneic environment is used to provide tolerance to the autoantigen associated with the autoimmune disorder.

Accordingly, in a first aspect of the disclosure we provide a method of desensitising an individual to one or more polypeptide antigens each of which contains a T cell epitope, the method comprising: step (1) administering to the individual a T-cell-epitope-containing peptide, or a course of T-cell-epitope-containing peptides, of a first antigen to which the individual has been previously exposed, in order to generate a state of hyporesponsiveness to the first antigen; then step (2) administering to the individual a compound, that is, a composition that comprises the T cell epitope of a peptide administered in step (1) and optionally further comprising a T cell epitope of the one or more polypeptide antigens to which the individual is to be desensitised; and, if the composition administered in step (2) does not contain a T cell epitope of the said one or more polypeptide antigens, the method further comprises the step of (3) administering to the individual a composition which contains a T cell epitope of the one or more polypeptide antigens to which the individual is to be desensitised, wherein in steps (2) and (3) the composition is substantially localised at the site of administration and if steps (2) and (3) are carried out the compositions are administered to substantially the same site.

Preferably, the first antigen is an allergen. In addition, the individual to be treated is typically sensitised to the antigen in the sense that the individual displays a pathogenic recognition of the first antigen.

Put in another way, in a first aspect of the disclosure, a method is provided for desensitising an individual to one or more polypeptide antigens. The method entails, in a first step, administering to the individual a primary composition comprising a first polypeptide antigen containing a T cell epitope, wherein the individual has been previously exposed to the antigen and the administration is carried out in a manner sufficient to generate a hyporesponsive state against the first polypeptide antigen. Once a hyporesponsive state has been established toward the first polypeptide antigen, or at least a shift toward desensitisation has occurred, the method entails administration of a secondary composition comprising a second, different polypeptide antigen to which the individual is to be sensitised. Administration of the secondary composition is carried out in such a way as to take advantage of the tolergeneic environment established by use of the primary composition, where it is now possible to establish tolerance to the second, different polypeptide antigen. The secondary composition is coadministered with either the first polypeptide antigen or a larger molecule containing the first polypeptide antigen. By "coadministered" it is meant either the simultaneous or concurrent administration of polypeptide antigens, e.g., when the two are present in the same composition or administered in separate compositions at nearly the same time but at different sites, as well as the delivery of polypeptide antigens in separate compositions at different times. For example, the secondary composition may be delivered prior to or subsequent to delivery of the first polypeptide antigen (or a larger molecule comprising the first polypeptide antigen) at the same or a different site. The timing between deliveries can range from about several seconds apart to about several minutes apart, several hours apart, or even several days apart. Furthermore, different delivery methods can be employed.

The first polypeptide antigen is preferably an allergen to which the individual is sensitised to. In addition, in some examples, the second polypeptide antigen is also an allergen, albeit a second, different allergen. Suitable allergens for use in the methods of the invention can of course be obtained and/or produced using known methods. Classes of suitable allergens include, but are not limited to, pollens, animal dander, grasses, molds, dusts, antibiotics, stinging insect venoms, and a variety of environmental (including chemicals and metals), drug and food allergens. Common tree allergens include pollens from cottonwood, popular, ash, birch, maple, oak, elm, hickory, and pecan trees; common plant allergens include those from rye, ragweed, English plantain, sorrel-dock and pigweed; plant contact allergens include those from poison oak, poison ivy and nettles; common grass allergens include Timothy, Johnson, Bermuda, fescue and bluegrass allergens; common allergens can also be obtained from molds or fungi such as Alternaria, Fusarium, Hormodendrum, Aspergillus, Micropolyspora, Mucor and thermophilic actinomycetes; penicillin and tetracycline are common antibiotic allergens; epidermal allergens can be obtained from house or organic dusts (typically fungal in origin), from insects such as house mites (dermatphagoides pterosinyssis), or from animal sources such as feathers, and cat and dog dander; common food allergens include milk and cheese (diary), egg, wheat, nut (e.g., peanut), seafood (e.g., shellfish), pea, bean and gluten allergens; common environmental allergens include metals (nickel and gold), chemicals (formaldehyde, trinitrophenol and turpentine), Latex, rubber, fiber (cotton or wool), burlap, hair dye, cosmetic, detergent and perfume allergens; common drug allergens include local anesthetic and salicylate allergens; antibiotic allergens include penicillin and sulfonamide allergens; and common insect allergens include bee, wasp and ant venom, and cockroach calyx allergens. Particularly well characterized allergens include, but are not limited to, the major and cryptic epitopes of the Der p I allergen (Hoyne et al. (1994) Immunology 83190-195), bee venom phospholipase A2 (PLA) (Akdis et al. (1996) J. Clin. Invest. 98:1676-1683), birch pollen allergen Bet v 1 (Bauer et al. (1997) Clin. Exp. Immunol. 107:536-541), and the multi-epitopic recombinant grass allergen rKBG8:3 (Cao et al. (1997) Immunology 90:46-51). These and other suitable allergens are commercially available and/or can be readily prepared as extracts following known techniques.

In preferred disclosure, the allergen used in the primary composition is a Fel d 1 (the feline skin and salivary gland allergen of the domestic cat *Felis domesticus -* the amino acid sequence of which is disclosed in International Publication WO 91/06571); Der p I, Der p II, Der fI or Der fII (the major protein allergens from the house dust mite dermatophagoides - amino acid sequences disclosed in International Publication WO 94/24281); or allergens present in any of the following: grass, tree and weed (including ragweed) pollens; fungi and moulds; foods (e.g., fish, shellfish, crab, lobster, peanuts, nuts, wheat gluten, eggs and milk); stinging insects e.g., bee, wasp and hornet and the chirnomidae (non-biting midges); spiders and mites, including the house dust mite; allergens found in the dander, urine, saliva, blood or other bodily fluid of mammals such as cat, dog, cows, pigs, sheep, horse, rabbit, rat, guinea pig, mouse and gerbil; airborne particulates in general; latex; and protein detergent additives.

Where the allergen is an insect protein, the peptides may be selected from any of housefly, fruit fly, sheep blow fly, screw worm fly, grain weevil, silkworm, honeybee, non-biting midge larvae, bee moth larvae, mealworm, cockroach and larvae of *Tenibrio molitor* beetle. All these being insect allergens, they are of particular relevance to allergic problems arising in the workplace.

It is preferred that the allergen present in the primary composition is an aero allergen (i.e., an airborne allergen) since it is likely that the individual has previously been exposed to such an allergen even if he or she is not allergic to the allergen. Thus, particularly preferred allergens are those from cat or dog dander, those from pollen, those from fungi and moulds, those from house dust mite or other mites and those from certain foods such as nuts, especially peanuts. Latex can be an aero allergen as can peanut allergen when an aerosol is created, for example when frying in peanut oil.

A particularly preferred allergen for use in the methods described herein, particularly the allergen present in the primary composition, is Fel d 1. Thus, in step (1) it is preferred that one or more T-cell-epitope-containing peptides of Fel d 1 are administered to the individual. It is particularly preferred if one or more of the following peptides of Fel d 1 are administered: EQVAQYKALPVVLENA (SEQ ID NO. 2) or KALPVVLENARILKNCV (SEQ ID NO. 3), both of which are known to be MHC restricted. These peptides are described in more detail in commonly owned International Publication WO 99/34826 (PCT/GB99/00080): Other allergens useful in the present methods can be readily produced using publicly available sequence information for the allergens. The following is a list of known allergens sequences and database accession numbers (NCBI Entrez accession numbers). NCBI is the National Center for Biotechnology information and is a division of the US National Institutes of Health. The NCBI web site, from which access to the database may be sought, www.ncbi.nlm.nih.gov/. The allergens may be used as described above in order to identify MHC-restricted peptides capable of inducing LPR in individuals who possess a particular MHC molecule.

Allergen sequences and database accession numbers (NCBI Entrez accession numbers):

### House dust mite

### Dermatophagoides pteronyssinus

Der p 1
MKIVLAIASLLALSAVYARPSSIKTFEEYKKAFNKSYATFEDEEAARKNFL
ESVKYVQSNGGAINHLSDLSLDEFKNRFLMSAEAFEHLKTQFDLNAETNA
CSINGNAPAEIDLRQMRTVTPIRMQGGCGSCWAFSGVAATESAYLAYRNQ
SLDLAEQELVDCASQHGCHGDTIPRGIEYIQHNGVVQESYYRYVAREQSC
RRPNAQRFGISNYCQIYPPNVNKIREALAQTHSAIAVIIGIKDLDAFRHYDG
RTIIQRDNGYQPNYHAVNIVGYSNAQGVDYWIVRNSWDTNWGDNGYGY
FAANIDLMMIEEYPYVVIL

Der p 2
MMYKILCLSLLVAAVARDQVDVKDCANHEIKKVLVPGCHGSEPCIIHRGK
PFQLEAVFEANQNTKTAKIEIKASIDGLEVDVPGIDPNACHYMKCPLVKGQ
QYDIKYTWNVPKIAPKSENVVVTVKVMGDDGVLACAIATHAKIRD

Der p 3
MIIYNILIVLLLAINTLANPILPASPNATIVGGEKALAGECPYQISLQSSSHFC
GGTILDEYWILTAAHCVAGQTASKLSIRYNSLKHSLGGEKISVAKIFAHEK
YDSYQIDNDIALIKLKSPMKLNQKNAKAVGLPAKGSDVKVGDQVRVSGW
GYLEEGSYSLPSELRRVDIAVVSRKECNELYSKANAEVTDNMICGGDVAN
GGKDSCQGDSGGPVVDVKNNQVVGIVSWGYGCARKGYPGVYTRVGNFI
DWIESKRSQ

Der p 4
KYXNPHFIGXRSVITXLME 
Der p 5
MKFIIAFFVATLAVMTVSGEDKKHDYQNEFDFLLMERIHEQIKKGELALF
YLQEQINHFEEKPTKEMKDKIVAEMDTIIAMIDGVRGVLDRLMQRKDLDI
FEQYNLEMAKKSGDILERDLKKEEARVKKIEV
 Der p 6
AIGXQPAAEAEAPFQISLMK 
Der p 7
MMKLLLIAAAAFVAVSADPIHYDKITEEINKAVDEAVAAIEKSETFDPMK
VPDHSDKFERHIGIIDLKGELDMRNIQVRGLKQMKRVGDANVKSEDGVV
KAHLLVGVHDDVVSMEYDLAYKLGDLHPNTHVISDIQDFVVELSLEVSEE
GNMTLTSFEVRQFANVVNHIGGLSILDPIFAVLSDVLTAIFQDTVRAEMTK
VLAPAFKKELERNNQ
 Der p9
IVGGSNASPGDAVYQIAL

### Dermatophagoides farinae

Der f 1
MKFVLAIASLLVLTVYARPASIKTFEFKKAFNKNYATVEEEEVARKNFLES
LKYVEANKGAINHLSDLSLDEFKNRYLMSAEAFEQLKTQFDLNAETSACR
INSVNVPSELDLRSLRTVTPIRMQGGCGSCWAFSGVAATESAYLAYRNTS
LDLSEQELVDCASQHGCHGDTIPRGIEYIQQNGVVEERSYPYVAREQRCR
RPNSQHYGISNYCQIYPPDVKQIREALTQTHTAIAVIIGIKDLRAFQHYDGR
TIIQHDNGYQPNYHAVNIVGYGSTQGDDYWIVRNSWDTTWGDSGYGYFQ
AGNNLMMIEQYPYVVIM

Der f 2
MISKILCLSLLVAAVVADQVDVKDCANNEIKKVMVDGCHGSDPCIIHRGK
PFTLEALFDANQNTKTAKIEIKASLDGLEIDVPGIDTNACHFMKCPLVKGQ
QYDIKYTWNVPKIAPKSENVVVTVKLIGDNGVLACAIATHGKIRD

Der f 3
MMILTIVVLLAANILATPILPSSPNATIVGGVKAQAGDCPYQISLQSSSHFC
GGSILDEYWILTAAHCVNGQSAKKLSIRYNTLKHASGGEKIQVAEIYQHE
NYDSMTIDNDVALIKLKTPMTLDQTNAKPVPLPAQGSDVKVGDKIRVSG
WGYLQEGSYSLPSELQRVDIDVVSREQCDQLYSKAGADVSENMICGGDV
ANGGVDSCQGDSGGPVVDVATKQIVGIVSWGYGCARKGYPGVYTRVGN
FVDWIESKRSQ

Derf 4
AVGGQDADLAEAPFQISLLK

Der f 7
MMKFLLIAAVAFVAVSADPIHYDKITEEINKAIDDAIAAIEQSETIDPMKVP
DHADKFERHVGIVDFKGELAMRNIEARGLKQMKRQGDANVKGEEGIVK
AHLLIGVHDDIVSMEYDLAYKLGDLHPTTHVISDIQDFVVALSLEISDEGNI
TMTSFEVRQFANVVNHIGGLSILDPIFGVLSDVLTAIFQDTVRKEMTKVLA
PAFKRELEKN

Additional mite allergen sequences (NCBI entrez accession):
1170095; 1359436; 2440053; 666007; 487661; 1545803; 84702; 84699; 625532; 404370; 1091577; 1460058; 7413; 9072; 387592.

### Cat

### Felis sequences

1082946 Fel dI chain 2 precursor - cat
MRGALLVLALLVTQALGVKMAETCPIFYDVFFAVANGNELLLDLSLTKV
NATEPERTAMKKIQDCYVENGLISRVLDGLVMTTISSSKDCMGEAVQNTV
EDLKLNTLGR

1082945 Fel dI chain 1 short form - cat
MLDAALPPCPTVAATADCEICPAVKRDVDLFLTGTPDEYVEQVAQYKALP
VVLENARILKNCVDAKMTEEDKENALSLLDKIYTSPLC

1082944 Fel dI chain 1 long form precursor - cat
MKGARVLVLLWAALLLIWGGNCEICPAVKRDVDLFLTGTPDEYVEQVAQ
YKALPVVLENARILKNCVDAKMTEEDKENALSLLDKIYTSPLC

Additional Felis sequences (NCBI entrez accession):
539716; 539715; 423193; 423192; 423191; 423190; 1364213; 1364212; 395407; 163827; 163823; 163825; 1169665; 232086; 1169666.

### Latex

### Hevea sequences:

Hev b 1
MAEDEDNQQGQGEGLKYLGFVQDAATYAVTTFSNVYLFAKDKSGPLQP
GVDIIEGPVKNVAVPLYNRFSYIPNGALKFVDSTVVASVTIIDRSLPPIVKD
ASIQVVSAIRAAPEAARSLASSLPGQTKILAKVFYGEN

Hev b 3
MAEEVEEERLKYLDFVRAAGVYAVDSFSTLYLYAKDISGPLKPGVDTIEN
VVKTVVTPVYYIPLEAVKFVDKTVDVSVTSLDGVVPPVIKQVSAQTYSVA
QDAPRIVLDVASSVFNTGVQEGAKALYANLEPKAEQYAVITWRALNKLP
LVPQVANVVVPTAVYFSEKYNDVVRGTTEQGYRVSSYLPLLPTEKITKVF
GDEAS

Additional Hevea sequences (NCBI entrez accession):
3319923; 3319921; 3087805; 1493836; 1480457; 1223884; 3452147; 3451147; 1916805; 232267; 123335; 2501578; 3319662; 3288200; 1942537; 2392631; 2392630; 1421554; 1311006; 494093; 3183706; 3172534;.283243; 1170248; 1708278; 1706547; 464775; 266892; 231586; 123337; 116359; 123062; 2213877; 542013; 2144920; 1070656; 2129914; 2129913; 2129912; 100135; 82026; 1076559; 82028; 82027; 282933; 280399; 100138; 1086972;108697;1086976; 1086978; 1086978; 1086976; 1086974; 1086972; 913758; 913757; 913756; 234388; 1092500; 228691; 1177405; 18839; 18837; 18835; 18833; 18831; 1209317; 1184668; 168217; 168215; 168213; 168211; 168209; 348137.

### Rye grass

### Lolium sequences:

126385 Lol p 1
MASSSSVLLVVALFAVFLGSAHGIAKVPPGPNITAEYGDKWLDAKSTWY
GKPTGAGPKDNGGACGYKNVDKAPFNGMTGCGNTPIFKDGRGCGSCFEI
KCTKPESCSGEAVTVTITDDNEEPIAPYHFDLSGHAFGSMAKKGEEQNVRS
AGELELQFRRVKCKYPDDTKPTFHVEKASNPNYLAILVKYVDGDGDVVA
VDIK[?]KGKDKWIELKESWGAVWRIDTPDKLTGPFTVRYTTEGGTKSEFED
VIPEGWKADTSYSAK

126386 Lol p 2a
AAPVEFTVEKGSDEKNLALSIKYNKEGDSMAEVELKEHGSNEWLALKKN
GDGVWEIKSDKPLKGPFNFRFVSEKGMRNVFDDVVPADFKVGTTYKPE

126387 Lol p 3
TKVDLTVEKGSDAKTLVLNIKYTRPGDTLAEVELRQHGSEEWEPMTKKG
NLWEVKSAKPLTGPMNFRFLSKGGMKNVFDEVIPTAFTVGKTYTPEYN

2498581 Lol p 5a
MAVQKYTVALFLRRGPRGGPGRSYAADAGYTPAAAATPATPAATPAGG
WREGDDRRAEAAGGRQRLASRQPWPPLPTPLRRTSSRSSRPPSPSPPRASS
PTSAAKAPGLIPKLDTAYDVAYKAAEAHPRGQVRRLRHCPHRSLRVIAGA
LEVHAVKPATEEVLAAKIPTGELQIVDKIDAAFKIAATAANAAPTNDKFTV
FESAFNKALNECTGGAMRPTSSSPPSRPRSSRPTPPPSPAAPEVKYAVFEAA
LTKAITAMTQAQKAGKPAAAAATAAATVATAAATAAAVLPPPLLVVQSL
ISLLIYY

2498582 Lol p 5b

MAVQKHTVALFLAVALVAGPAASYAADAGYAPATPATPAAPATAATPA
TPATPATPAAVPSGKATTEEQKLIEKINAGFKAAVAAAAVVPPADKYKTF
VETFGTATNKAFVEGLASGYADQSKNQLTSKLDAALKLAYEAAQGATPE
AKYDAYVATLTEALRVIAGTLEVHAVKPAAEEVKVGAIPAAEVQLIDKV
DAAYRTAATAANAAPANDKFTVFENTFNNAIKVSLGAAYDSYKFIPTLVA
AVKQAYAAKQATAPEVKYTVSETALKKAVTAMSEAEKEATPAAAATAT
PTPAAATATATPAAAYATATPAAATATATPAAATATPAAAGGYKV

455288 Lol p isoform 9
MAVQKHTVALFLAVALVAGPAASYAADAGYAPATPATPAAPATAATPA
TPATPATPAAVPSGKATTEEQKLIEKINAGFKAAVAAAAVVPPADKYKTF
VETFGTATNKAFVEGLASGYADQSKNQLTSKLDAALKLAYEAAQGATPE
AKYDAYVATLTEALRVIAGTLEVHAVKPAAEEVKVGAIPAAEVQLIDKV
DAAYRTAATAANAAPANDKFTVFENTFNNAIKVSLGAAYDSYKFIPTLVA
AVKQAYAAKQATAPEVKYTVSETALKKAVTAMSEAEKEATPAAAATAT
PTPAAATATATPAAAYATATPAAATATATPAAATATPAAAGGYKV

1582249 Lol p 11
DKGPGFVVTGRVYCDPCRAGFETNVSHNVEGATVAVDCRPFDGGESKLK
AEATTDKDGWYKIEIDQDHQEEICEVVLAKSPDKSCSEIEEFRDRARVPLT
SNXGIKQQGIRYANPIAFFRKEPLKECGGILQAY

Additional Lolium sequences (NCBI entrez accession):
135480; 417103; 687261; 687259; 1771355; 2388662; 631955; 542131; 542130; 542129; 100636; 626029; 542132; 320616; 320615; 320614; 100638; 100634; 82450; 626028; 100639; 283345; 542133; 1771353; 1763163;1040877; 1040875; 250525; 551047; 515377; 510911; 939932; 439950; 2718; 168316; 168314; 485371; 2388664; 2832717; 2828273; 548867.

### Olive tree

### Olive sequences

416610 Ole e 1
EDIPQPPVSQFHIQGQVYCDTCRAGFITELSEFIPGASLRLQCKDKENGDVT
FTEVGYTRAEGLYSMLVE
RDHKNEFCEITLISSGRKDCNEIPTEGWAKPSLKFKLNTVNGTTRTVNPLG
FFKKEALPKCAQVYNKLGM
YPPNM

### Parietaria

### Parietaria sequences:

2497750 Par j P2
MRTVSMAALVVIAAALAWTSSAEPAPAPAPGEEACGKVVQDIMPCLHFV
KGEEKEPSKECCSGTKKLSEEVKTTEQKREACKCIVRATKGISGIKNELVA
EVPKKCDIKTTLPPITADFDCSKIQSTIFRGYY

1352506 Par j P5
MVRALMPCLPFVQGKEKEPSKGCCSGAKRLDGETKTGPQRVHACECIQT
AMKTYSDIDGKLVSEVPKHCGIVDSKLPPIDVNMDCKTVGVVPRQPQLPV
SLRHGPVTGPSDPAHKARLERPQIRVPPPAPEKA

1532056 Par j P8
MRTVSMAALVVIAAALAWTSSAELASAPAPGEGPCGKVVHHIMPCLKFV
KGEEKEPSKSCCSGTKKLSEEVKTTEQKREACKCIVAATKGISGIKNELVA
EVPKKCGITTTLPPITADFDCSKIESTIFRGYY

1532058 Par j P9
MRTVSAPSAVALVVIVAAGLAWTSLASVAPPAPAPGSEETCGTVVRALMP

CLPFVQGKEKEPSKGCCSGAKRLDGETKTGLQRVHACECIQTAMKTYSDI
DGKLVSEVPKHCGIVDSKLPPIDVNMDCKTLGVVPRQPQLPVSLRHGPVT
GPSDPAHKARLERPQIRVPPPAPEKA

2497749 Par j P9
MRTVSARSSVALVVIVAAVLVWTSSASVAPAPAPGSEETCGTVVGALMP
CLPFVQGKEKEPSKGCCSGAKRLDGETKTGPQRVHACECIQTAMKTYSDI
DGKLVSEVPKHCGIVDSKLPPIDVNMDCKTLGVLHYKGN

1086003 Par j 1
MVRALMPCLPFVQGKEKEPSKGCCSGAKRLDGETKTGPQRVHACECIQT
AMKTYSDIDGKLVSEVPKHCGIVDSKLPPIDVNMDCKTVGVVPRQPQLPV
SLRHGPVTGPSRSRPPTKHGWRDPRLEFRPPHRKKPNPAFSTLG

Additional Parietaria sequences (NCBI entrez accession):
543659; 1836011; 1836010; 1311513; 1311512; 1311511; 1311510; 1311509; 240971.

### Timothy grass

### Phleum sequences:

Phl p 1
MASSSSVLLVVVLFAVFLGSAYGIPKVPPGPNITATYGDKWLDAKSTWYG
KPTGAGPKDNGGACGYKDVDKPPFSGMTGCGNTPIFKSGRGCGSCFEIKC
TKPEACSGEPVVVHITDDNEEPIAPYHFDLSGHAFGAMAKKGDEQKLRSA
GELELQFRRVKCKYPEGTKVTFHVEKGSNPNYLALLVKYVNGDGDVVAV
DIKEKGKDKWIELKESWGAIWRIDTPDKLTGPFTVRYTTEGGTKTEAEDVI
PEGWKADTSYESK

Phl p 1
MASSSSVLLVVALFAVFLGSAHGIPKVPPGPNITATYGDKWLDAKSTWYG
KPTAAGPKDNGGACGYKDVDKPPFSGMTGCGNTPIFKSGRGCGSCFEIKC
TKPEACSGEPVVVHITDDNEEPIAAYHFDLSGIAFGSMAKKGDEQKLRSA
GEVEIQFRRVKCKYPEGTKVTFHVEKGSNPNYLALLVKFSGDGDVVAVDI
KEKGKDKWIALKESWGAIWRIDTPEVLKGPFTVRYTTEGGTKARAKDVIP
EGWKADTAYESK

Phlp 2
MSMASSSSSSLLAMAVLAALFAGAWCVPKVTFTVEKGSNEKHLAVLVKY
EGDTMAEVELREHGSDEWVAMTKGEGGVWTFDSEEPLQGPFNFRFLTEK
GMKNVFDDVVPEKYTIGATYAPEE

Phl p 5
ADLGYGGPATPAAPAEAAPAGKATTEEQKLIEKINDGFKAALAAAAGVPP
ADKYKTFVATFGAASNKAFAEGLSAEPKGAAESSSKAALTSKLDAAYKL
AYKTAEGATPEAKYDAYVATLSEALRIIAGTLEVHAVKPAAEEVKVIPAG
ELQVIEKVDSAFKVAATAANAAPANDKFTVFEAAFNNAIKASTGGAYESY
KFIPALEAAVKQAYAATVATAPEVKYTVFETALKKAFTAMSEAQKAAKP
ATEATATATAAVGAATGAATAATGGYKV

Phl p 5
ADLGYGGPATPAAPAEAAPAGKATTEEQKLIEKINDGFKAALAAAAGVPP
ADKYKTFVATFGAASNKAFAEGLSAEPKGAAESSSKAALTSKLDAAYKL
AYKTAEGATPEAKYDAYVATLSEALRIIAGTLEVHAVKPAAEEVKVIPAG
ELQVIEKVDSAFKVAATAANAAPANDKFTVFEAAFNNAIKASTGGAYESY
KFIPALEAAVKQAYAATVATAPEVKYTVFETALKKAITAMSEAQKAAKP
ATEATATATAAVGAATGAATAATGGYKV

Phl p 5b
AAAAVPRRGPRGGPGRSYTADAGYAPATPAAAGAAAGKATTEEQKLIED
INVGFKAAVAAAASVPAADKFKTFEAAFTSSSKAAAAKAPGLVPKLDAA
YSVAYKAAVGATPEAKFDSFVASLTEALRVIAGALEVHAVKPVTEEPGM
AKIPAGELQIIDKIDAAFKVAATAAATAPADDKFTVFEAAFNKAIKESTGG
AYDTYKCIPSLEAAVKQAYAATVAAAPQVKYAVFEAALTKAITAMSEVQ
KVSQPATGAATVAAGAATTAAGAASGAATVAAGGYKV

Phl p 5a
ADLGYGPATPAAPAAGYTPATPAAPAGADAAGKATTEEQKLIEKINAGFK
AALAGAGVQPADKYRTFVATFGPASNKAFAEGLSGEPKGAAESSSKAAL
TSKLDAAYKLAYKTAEGATPEAKYDAYVATLSEALRIIAGTLEVHAVKPA
AEEVKVIPAGELQVIEKVDAAFKVAATAANAAPANDKFTVFEAAFNDEIK
ASTGGAYESYKFIPALEAAVKQAYAATVATAPEVKYTVFETALKKAITAM
SEAQKAAKPAAAATATATAAVGAATGAATAATGGYKV

Phl p 5
MAVQKYTVALFLAVALVAGPAASYAADAGYAPATPAAAGAEAGKATTE
EQKLIEDINVGFKAAVAAAASVPAADKFKTFEAAFTSSSKAATAKAPGLV
PKLDAAYSVSYKAAVGATPEAKFDSFVASLTEALRVIAGALEVHAVKPVT
EEPGMAKIPAGELQIIDKIDAAFKVAATAAATAPADTVFEAAFNKAIKEST
GGAYDTYKCIPSLEAAVKQAYAATVAAAPQVKYAVFEAALTKAITAMSE
VQKVSQPATGAATVAAGAATTAAGAASGAATVAAGGYKV

Phl p 5
MAVQKYTVALFLAVALVAGPAASYAADAGYAPATPAAAGAEAGKATTE
EQKLIEDINVGFKAAVAAAASVPAADKFKTFEAAFTSSSKAATAKAPGLV
PKLDAAYSVAYKAAVGATPEAKFDSFVASLTEALRVIAGALEVHAVKPV
TEDPAWPKIPAGELQIIDKIDAAFKVAATAAATAPADDKFTVFEAAFNKAI

KESTGGAYDTYKCIPSLEAAVKQAYAATVAAAPQVKYAVFEAALTKAIT
AMSEVQKVSQPATGAATVAAGAATTATGAASGAATVAAGGYKV

Phl p 5
ADAGYAPATPAAAGAEAGKATTEEQKLIEDINVGFKAAVAAAASVPAAD
KFKTFEAAFTSSSKAATAKAPGLVPKLDAAYSVAYKAAVGATPEAKFDSF
VASLTEALRVIAGALEVHAVKPVTEEPGMAKIPAGELQIIDKIDAAFKVAA
TAAATAPADDKFTVFEAAFNKAIKESTGGAYDTYKCIPSLEAAVKQAYAA
TVAAAPQVKYAVFEAALTKAITAMSEVQKVSQPATGAATVAAGAATTA
AGAASGAATVAAGGYKV

Phl p 5
SVKRSNGSAEVHRGAVPRRGPRGGPGRSYAADAGYAPATPAAAGAEAG
KATTEEQKLIEDINVGFKAAVAAAASVPAADKFKTFEAAFTSSSKAATAK
APGLVPKLDAAYSVAYKAAVGATPEAKFDSFVASLTEALRVIAGALEVH
AVKPVTEEPGMAKIPAGELQIIDKIDAAFKVAATAAATAPADDKFTVFEA
AFNKAIKESTGGAYDTYKCIPSLEAAVKQAYAATVAAAPQVKYAVFEAA
LTKAITAMSEVQKVSQPATGAATVAAGAATTAAGAASGAATVAAGGYK
V

Phl p 5
MAVHQYTVALFLAVALVAGPAGSYAADLGYGPATPAAPAAGYTPATPA
APAGAEPAGKATTEEQKLIEKINAGFKAALAAAAGVPPADKYRTFVATFG
AASNKAFAEGLSGEPKGAAESSSKAALTSKLDAAYKLAYKTAEGATPEA
KYDAYVATVSEALRIIAGTLEVHAVKPAAEEVKVIPAGELQVIEKVDAAF
KVAATAANAAPANDKFTVFEAAFNDAIKASTGGAYESYKFIPALEAAVK
QAYAATVATAPEVKYTVFETALKKAITAMSEAQKAAKPAAAATATATAA
VGAATGAATAATGGYKV

Phl p 5

ADLGYGGPATPAAPAEAAPAGKATTEEQKLIEKINDGFKAALAAAAGVPP
ADKYKTFVATFGAASNKAFAEGLSAEPKGAAESSSKAALTSKLDAAYKL
AYKTAEGATPEAKYDAYVATLSEALRIIAGTLEVHAVKPAAEEVKVIPAG
ELQVIEKVDSAFKVAATAANAAPANDKFTVFEAAFNNAIKASTGGAYESY
KFIPALEAAVKQAYAATVATAPEVKYTVFETALKKAFTAMSEAQKAAKP
ATEATATATAAVGAATGAATAATGGYKV

Phl p5b
AAAAVPRRGPRGGPGRSYTADAGYAPATPAAAGAAAGKATTEEQKLIED
INVGFKAAVAAAASVPAADKFKTFEAAFTSSSKAAAAKAPGLVPKLDAA
YSVAYKAAVGATPEAKFDSFVASLTEALRVIAGALEVHAVKPVTEEPGM
AKIPAGELQIIDKIDAAFKVAATAAATAPADDKFTVFEAAFNKAIKESTGG
AYDTYKCIPSLEAAVKQAYAATVAAAPQVKYAVFEAALTKAITAMSEVQ
KVSQPATGAATVAAGAATTAAGAASGAATVAAGGYKV

Phl p5a
ADLGYGPATPAAPAAGYTPATPAAPAGADAAGKATTEEQKLIEKINAGFK
AALAGAGVQPADKYRTFVATFGPASNKAFAEGLSGEPKGAAESSSKAAL
TSKLDAAYKLAYKTAEGATPEAKYDAYVATLSEALRIIAGTLEVHAVKPA
AEEVKVIPAGELQVIEKVDAAFKVAATAANAAPANDKFTVFEAAFNDEIK
ASTGGAYESYKFIPALEAAVKQAYAATVATAPEVKYTVFETALKKAITAM
SEAQKAAKPAAAATATATAAVGAATGAATAATGGYKV

Phl p 5
AVPRRGPRGGPGRSYAADAGYAPATPAAAGAEAGKATTEEQKLIEDINV
GFKAAVAAAASVPAGDKFKTFEAAFTSSSKAATAKAPGLVPKLDAAYSV
AYKAAVGATPEAKFDSFVASLTEALRVIAGALEVHAVKPVTEEPGMAKIP
AGELQIIDKIDAAFKVAATAAATAPADDKFTVFEAAFNKAIKESTGGAYD
TYKCIPSLEAAVKQAYAATVAAAPQVKYAVFEAALTKAITAMSEVQKVS
QPATGAATVAAGAATTATGAASGAATVAAGGYKV

Phl p 5b
MAVPRRGPRGGPGRSYTADAGYAPATPAAAGAAAGKATTEEQKLIEDIN
VGFKAAVAARQRPAADKFKTFEAASPRHPRPLRQGAGLVPKLDAAYSVA
YKAAVGATPEAKFDSFVASLTEALRVIAGALEVHAVKPVTEEPGMAKIPA
GELQIIDKIDAAFKVAATAAATAPADDKFTVFEAAFNKAIKESTGGAYDT
YKCIPSLEAAVKQAYAATVAAAAEVKYAVFEAALTKAITAMSEVQKVSQ
PATGAATVAAGAATTAAGAASGAATVAAGGYKV

Phl p 5
MAVHQYTVALFLAVALVAGPAASYAADLGYGPATPAAPAAGYTPATPA
APAEAAPAGKATTEEQKLIEKINAGFKAALAAAAGVQPADKYRTFVATFG
AASNKAFAEGLSGEPKGAAESSSKAALTSKLDAAYKLAYKTAEGATPEA
KYDAYVATLSEALRIIAGTLEVHAVKPAAEEVKVIPAGELQVIEKVDAAF
KVAATAANAAPANDKFTVFEAAFNDAIKASTGGAYESYKFIPALEAAVK
QAYAATVATAPEVKYTVFETALKKAITAMSEAQKAAKPAAAATATATAA
VGAATGAATAATGGYKV

Phl p 5
EAPAGKATTEEQKLIEKINAGFKAALARRLQPADKYRTFVATFGPASNKA
FAEGLSGEPKGAAESSSKAALTSKLDAAYKLAYKTAEGATPEAKYDAYV
ATLSEALRIIAGTLEVHAVKPAAEEVKVIPAAELQVIEKVDAAFKVAATAA
NAAPANDKFTVFEAAFNDEIKASTGGAYESYKFIPALEAAVKQAYAATVA

TAPEVKYTVFETALKKAITAMSEAQKAAKPPPLPPPPQPPPLAATGAATAA
TGGYKV

Phl p 5
MAVHQYTVALFLAVALVAGPAASYAADLGYGPATPAAPAAGYTPATPA
APAEAAPAGKATTEEQKLIEKINAGFKAALAAAAGVQPADKYRTFVATFG
AASNKAFAEGLSGEPKGAAESSSKAALTSKLDAAYKLAYKTAEGATPEA
KYDAYVATLSEALRIIAGTLEVHAVKPAAEEVKVIPAGELQVIEKVDAAF
KVAATAANAAPANDKFTVFEAAFNDAIKASTGGAYESYKFIPALEAAVK
QAYAATVATAPEVKYTVFETALKKAITAMSEAQKAAKPAAAATATATAA
VGAATGAATAATGGYKV

Phl p 5b
MAVPRRGPRGGPGRSYTADAGYAPATPAAAGAAAGKATTEEQKLIEDIN
VGFKAAVAARQRPAADKFKTFEAASPRHPRPLRQGAGLVPKLDAAYSVA
YKAAVGATPEAKFDSFVASLTEALRVIAGALEVHAVKPVTEEPGMAKIPA
GELQIIDKIDAAFKVAATAAATAPADDKFTVFEAAFNKAIKESTGGAYDT
YKCIPSLEAAVKQAYAATVAAAAEVKYAVFEAALTKAITAMSEVQKVSQ
PATGAATVAAGAATTAAGAASGAATVAAGGYKV

Phl p 5a
ADLGYGPATPAAPAAGYTPATPAAPAGADAAGKATTEEQKLIEKINAGFK
AALAGAGVQPADKYRTFVATFGPASNKAFAEGLSGEPKGAAESSSKAAL
TSKLDAAYKLAYKTAEGATPEAKYDAYVATLSEALRIIAGTLEVHAVKPA
AEEVKVIPAGELQVIEKVDAAFKVAATAANAAPANDKFTVFEAAFNDEIK
ASTGGAYESYKFIPALEAAVKQAYAATVATAPEVKYTVFETALKKAITAM
SEAQKAAKPPPLPPPPQPPPLAATGAATAATGGYKV

Phl p 5
MAVHQYTVALFLAVALVAGPAASYAADLGYGPATPAAPAAGYTPATPA

APAEAAPAGKATTEEQKLIEKINAGFKAALAAAAGVQPADKYRTFVATFG
AASNKAFAEGLSGEPKGAAESSSKAALTSKLDAAYKLAYKTAEGATPEA
KYDAYVATLSEALRIIAGTLEVHAVKPAAEEVKVIPAGELQVIEKVDAAF
KVAATAANAAPANDKFTVFEAAFNDAIKASTGGAYESYKFIPALEAAVK
QAYAATVATAPEVKYTVFETALKKAITAMSEAQKAAKPAAAATATATAA
VGAATGAATAATGGYKV

Phl p 6
MAAHKFMVAMFLAVAVVLGLATSPTAEGGKATTEEQKLIEDVNASFRAA
MATTANVPPADKYKTFEAAFTVSSKRNLADAVSKAPQLVPKLDEVYNAA
YNAADHAAPEDKYEAFVLHFSEALRIIAGTPEVHAVKPGA

Phl p 6
SKAPQLVPKLDEVYNAAYNAADHAAPEDKYEAFVLHFSEALHIIAGTPEV
HAVKPGA

Phl p 6
ADKYKTFEAAFTVSSKRNLADAVSKAPQLVPKLDEVYNAAYNAADHAA
PEDKYEAFVLHFSEALHIIAGTPEVHAVKPGA

Phl p 6
TEEQKLIEDVNASFRAAMATTANVPPADKYKTLEAAFTVSSKRNLADAVS
KAPQLVPKLDEVYNAAYNAADHAAPEDKYEAFVLHFSEALRIIAGTPEVH
AVKPGA

Phl p 6
MAAHKFMVAMFLAVAVVLGLATSPTAEGGKATTEEQKLIEDINASFRAA
MATTANVPPADKYKTFEAAFTVSSKRNLADAVSKAPQLVPKLDEVYNAA
YNAADHAAPEDKYEAFVLHFSEALHIIAGTPEVHAVKPGA

Phl p 6
MVAMFLAVAVVLGLATSPTAEGGKATTEEQKLIEDVNASFRAAMATTAN
VPPADKYKTFEAAFTVSSKRNLADAVSKAPQLVPKLDEVYNAAYNAADH
AAPEDKYEAFVLHFSEALRIIAGTPEVHAVKPGA

Phl p 7
MADDMERIFKRFDTNGDGKISLSELTDALRTLGSTSADEVQRMMAEIDTD
GDGFIDFNEFISFCNANPGLMKDVAKVF

Phl p 11
MSWQTYVDEHLMCEIEGHHLASAAILGHDGTVWAQSADFPQFKPEEITGI
MKDFDEPGHLAPTGMFVAGAKYMVIQGEPGRVIRGKKGAGGITIKKTGQ
ALVVGIYDEPMTPGQCNMVVERLGDYLVEQGM

Additional Phleum sequences (NCBI entrez accession):
458878; 548863; 2529314; 2529308; 2415702; 2415700; 2415698; 542168; 542167; 626037; 542169; 541814; 542171; 253337; 253336; 453976; 439960.

### Wasp (and related)

### Vespula sequences:

465054 ALLERGEN VES V 5
MEISGLVYLIIIVTIIDLPYGKANNYCKIKCLKGGVHTACKYGSLKPNCGN
KVVVSYGLTKQEKQDILKEHNDFRQKIARGLETRGNPGPQPPAKNMKNL
VWNDELAYVAQVWANQCQYGHDTCRDVAKYQVGQNVALTGSTAAKY
DDPVKLVKMWEDEVKDYNPKKKFSGNDFLKTGHYTQMVWANTKEVGC
GSIKYIQEKWHKHYLVCNYGPSGNFMNEELYQTK

1709545 ALLERGEN VES M 1

GPKCPFNSDTVSIIIETRENRNRDLYTLQTLQNHPEFKKKTITRPVVFITHGF
TSSASEKNFINLAKALVDKDNYMVISIDWQTAACTNEYPGLKYAYYPTAA
SNTRLVGQYIATITQKLVKDYKISMANIRLIGHSLGAHVSGFAGKRVQELK
LGKYSEIIGLDPARPSFDSNHCSERLCETDAEYVQIIHTSNYLGTEKILGTV
DFYMNNGKNNPGCGRFFSEVCSHTRAVIYMAECIKHECCLIGIPRSKSSQPI
SRCTKQECVCVGLNAKKYPSRGSFYVPVESTAPFCNNKGKII

1352699 ALLERGEN VES V 1
MEENMNLKYLLLFVYFVQVLNCCYGHGDPLSYELDRGPKCPFNSDTVSIII
ETRENRNRDLYTLQTLQNHPEFKKKTITRPVVFITHGFTSSASETNFINLAK
ALVDKDNYMVISIDWQTAACTNEAAGLKYLYYPTAARNTRLVGQYIATI
TQKLVKHYKISMANIRLIGHSLGAHASGFAGKKVQELKLGKYSEIIGLDPA
RPSFDSNHCSERLCETDAEYVQIIHTSNYLGTEKTLGTVDFYMNNGKNQP
GCGRFFSEVCSHSRAVIYMAECIKHECCLIGIPKSKSSQPISSCTKQECVCV
GLNAKKYPSRGSFYVPVESTAPFCNNKGKII

1346323 ALLERGEN VES V 2
SERPKRVFNIYWNVPTFMCHQYDLYFDEVTNFNIKRNSKDDFQGDKIAIF
YDPGEFPALLSLKDGKYKKRNGGVPQEGNITIHLQKFIENLDKIYPNRNFS
GIGVIDFERWRPIFRQNWGNMKIHKNFSIDLVRNEHPTWNKKMIELEASK
RFEKYARFFMEETLKLAKKTRKQADWGYYGYPYCFNMSPNNLVPECDV
TAMHENDKMSWLFNNQNVLLPSVYVRQELTPDQRIGLVQGRVKEAVRIS
NNLKHSPKVLSYWWYVYQDETNTFLTETDVKKTFQEIVINGGDGIIIWGSS
SDVNSLSKCKRLQDYLLTVLGPIAINVTEAVN

549194 ALLERGEN VES VI
5KVNYCKIKCLKGGVHTACKYGTSTKPNCGKMVVKAYGLTEAEKQEILK
VHNDFRQKVAKGLETRGNPGPQPPAKNMNNLVWNDELANIAQVWASQC
NYGHDTCKDTEKYPVGQNIAKRSTTAALFDSPGKLVKMWENEVKDFNPN

IEWSKNNLKKTGHYTQMVWAKTKEIGCGSVKYVKDEWYTHYLVCNYGP
SGNFRNEKLYEKK

Additional vespula sequences (NCBI entrez accession):
549193; 549192; 549191; 549190; 549189; 117414; 126761; 69576; 625255; 627189; 627188; 627187; 482382; 112561; 627186; 627185; 1923233; 897645; 897647; 745570; 225764; 162551.

### Tree allergen sequences (mainly birch) sequences:

114922 Bet v 1
MGVFNYETETTSVIPAARLFKAFILDGDNLFPKVAPQAISSVENIEGNGGPG
TIKKISFPEGFPFKYVKDRVDEVDHTNFKYNYSVIEGGPIGDTLEKISNEIKI
VATPDGGSILKISNKYHTKGDHEVKAEQVKASKEMGETLLRAVESYLLAH
SDAYN

130975 Bet v 2
MSWQTYVDEHLMCDIDGQASNSLASAIVGHDGSVWAQSSSFPQFKPQEIT
GIMKDFEEPGHLAPTGLHLGGIKYMVIQGEAGAVIRGKKGSGGITIKKTGQ
ALVFGIYEEPVTPGQCNMVVERLGDYLIDQGL

1168696 Bet v 3
MPCSTEAMEKAGHGHASTPRKRSLSNSSFRLRSESLNTLRLRRIFDLFDKN
SDGIITVDELSRALNLLGLETDLSELESTVKSFTREGNIGLQFEDFISLHQSL
NDSYFAYGGEDEDDNEEDMRKSILSQEEADSFGGFKVFDEDGDGYISARE
LQMVLGKLGFSEGSEIDRVEKMIVSVDSNRDGRVDFFEFKDMMRSVLVRS
S

809536 Bet v 4

MADDHPQDKAERERIFKRFDANGDGKISAAELGEALKTLGSITPDEVKHM
MAEIDTDGDGFISFQEFTDFGRANRGLLKDVAKIF

543675 Que a I - Quercus alba=oak trees (fragment)
GVFTXESQETSVIAPAXLFKALFL
543509 Car b I - Carpinus betulus=hornbeam trees (fragment)
GVFNYEAETPSVIPAARLFKSYVLDGDKLIPKVAPQAIXK
543491 Aln g I - Alnus glutinosa=alder trees (fragment)
GVFNYEAETPSVIPAARLFKAFILDGDKLLPKVAPEAVSSVENI

1204056 Rubisco
VQCMQVWPPLGLKKFETLSYLPPLSSEQLAKEVDYLLRKNLIPCLEFELEH
GFVYREHNRSPGYYDGRYWTMWKLPMFGCNDSSQVLKELEECKKAYPS
AFIRIIGFDDK

Additional tree allergen sequences (NCBI entrez accession number):
131919; 128193; 585564; 1942360; 2554672; 2392209; 2414158; 1321728; 1321726; 1321724; 1321722; 1321720; 1321718; 1321716; 1321714; 1321712; 3015520; 2935416; 464576; 1705843; 1168701; 1168710; 1168709; 1168708; 1168707; 1168706; 1168705; 1168704; 1168703; 1168702; 1842188; 2564228; 2564226; 2564224; 2564222; 2564220; 2051993; 1813891; 1536889; 534910; 534900; 534898; 1340000; 1339998; 2149808; 66207; 2129477; 1076249; 1076247; 629480; 481805; 81443; 1361968; 1361967; 1361966; 1361965; 1361964; 1361963; 1361962; 1361961; 1361960; 1361959; 320546; 629483 ; 629482; 629481; 541804; 320545; 81444; 541814:; 629484; 474911; 452742; 1834387; 298737; 298736; 1584322; 1584321; 584320; 1542873; 1542871; 1542869; 1542867; 1542865; 1542863; 1542861; 1542859; 1542857; 1483232; 1483230; 1483228; 558561; 551640; 488605; 452746; 452744; 452740; 452738; 452736; 452734; 452732; 452730; 452728; 450885; 17938; 17927; 17925; 17921; 297538; 510951; 289331; 289329; 166953.

### Peanut

### Peanut sequences

1168391 Ara h 1
MRGRVSPLMLLLGILVLASVSATHAKSSPYQKKTENPCAQRCLQSCQQEP
DDLKQKACESRCTKLEYDPRCVYDPRGHTGTTNQRSPPGERTRGRQPGD
YDDDRRQPRREEGGRWGPAGPREREREEDWRQPREDWRRPSHQQPRKIR
PEGREGEQEWGTPGSHVREETSRNNPFYFPSRRFSTRYGNQNGRIRVLQRF
DQRSRQFQNLQNHRIVQIEAKPNTLVLPKHADADNILVIQQGQATVTVAN
GNNRKSFNLDEGHALRIPSGFISYILNRHDNQNLRVAKISMPVNTPGQFED
FFPASSRDQSSYLQGFSRNTLEAAFNAEFNEIRRVLLEENAGGEQEERGQR
RWSTRSSENNEGVIVKVSKEHVEELTKHAKSVSKKGSEEEGDITNPINLRE
GEPDLSNNFGKLFEVKPDKKNPQLQDLDMMLTCVEIKEGALMLPHFNSK
AMVIVVVNKGTGNLELVAVRKEQQQRGRREEEEDEDEEEEGSNREVRRY
TARLKEGDVFIMPAAHPVAINASSELHLLGFGINAENNHRIFLAGDKDNVI
DQIEKQAKDLAFPGSGEQVEKLIKNQKESHFVSARPQSQSQSPSSPEKESPE
KEDQEEENQGGKGPLLSILKAFN

### Ragweed

### Ambrosia sequences

113478 Amb a 1
MGIKHCCYILYFTLALVTLLQPVRSAEDLQQILPSANETRSLTTCGTYNIID
GCWRGKADWAENRKALADCAQGFAKGTIGGKDGDIYTVTSELDDDVAN
PKEGTLRFGAAQNRPLWIIFARDMVIRLDRELAINNDKTIDGRGAKVEIIN
AGFAIYNVKNIIIHNIIMHDIVVNPGGLIKSHDGPPVPRKGSDGDAIGISGGS

QIWIDHCSLSKAVDGLIDAKHGSTHFTVSNCLFTQHQYLLLFWDFDERGM
LCTVAFNKFTDNVDQRMPNLRHGFVQVVNNNYERWGSYALGGSAGPTIL
SQGNRFLASDIKKEVVGRYGESAMSESINWNWRSYMDVFENGAIFVPSGV
DPVLTPEQNAGMIPAEPGEAVLRLTSSAGVLSCQPGAPC

113479 Amb a 2
MGIKHCCYILYFTLALVTLVQAGRLGEEVDILPSPNDTRRSLQGCEAHNIID
KCWRCKPDWAENRQALGNCAQGFGKATHGGKWGDIYMVTSDQDDDVV
NPKEGTLRFGATQDRPLWIIFQRDMIIYLQQEMVVTSDKTIDGRGAKVELV
YGGITLMNVKNVIIHNIDIHDVRVLPGGRIKSNGGPAIPRHQSDGDAIHVTG
SSDIWIDHCTLSKSFDGLVDVNWGSTGVTISNCKFTHHEKAVLLGASDTH
FQDLKMHVTLAYNIFTNTVHERMPRCRFGFFQIVNNFYDRWDKYAIGGSS
NPTILSQGNKFVAPDFIYKKNVCLRTGAQEPEWMTWNWRTQNDVLENGA
IFVASGSDPVLTAEQNAGMMQAEPGDMVPQLTMNAGVLTCSPGAPC

113477 Amb a 1.3
MGIKQCCYILYFTLALVALLQPVRSAEGVGEILPSVNETRSLQACEALNIID
KCWRGKADWENNRQALADCAQGFAKGTYGGKWGDVYTVTSNLDDDV
ANPKEGTLRFAAAQNRPLWIIFKNDMVINLNQELVVNSDKTIDGRGVKVE
IINGGLTLMNVKNIIIHNINIHDVKVLPGGMIKSNDGPPILRQASDGDTINVA
GSSQIWIDHCSLSKSFDGLVDVTLGSTHVTISNCKFTQQSKAILLGADDTH
VQDKGMLATVAFNMFTDNVDQRMPRCRFGFFQVVNNNYDRWGTYAIG
GSSAPTILCQGNRFLAPDDQIKKNVLARTGTGAAESMAWNWRSDKDLLE
NGAIFVTSGSDPVLTPVQSAGMIPAEPGEAAIKLTSSAGVFSCHPGAPC

113476 Amb a 1.2
MGIKHCCYILYFTLALVTLLQPVRSAEDVEEFLPSANETRRSLKACEAHNII
DKCWRCKADWANNRQALADCAQGFAKGTYGGKHGDVYTVTSDKDDD
VANPKEGTLRFAAAQNRPLWIIFKRNMVIHLNQELVVNSDKTIDGRGVKV
NIVNAGLTLMNVKNIIIHNINIHDIKVCPGGMIKSNDGPPILRQQSDGDAIN

VAGSSQIWIDHCSLSKASDGLLDITLGSSHVTVSNCKFTQHQFVLLLGADD
THYQDKGMLATVAFNMFTDHVDQRMPRCRFGFFQVVNNNYDRWGTYAI
GGSSAPTILSQGNRFFAPDDIIKKNVLARTGTGNAESMSWNWRTDRDLLE
NGAIFLPSGSDPVLTPEQKAGMIPAEPGEAVLRLTSSAGVLSCHQGAPC

113475 Amb a 1.1
MGIKHCCYILYFTLALVTLLQPVRSAEDLQEILPVNETRRLTTSGAYNIIDG
CWRGKADWAENRKALADCAQGFGKGTVGGKDGDIYTVTSELDDDVAN
PKEGTLRFGAAQNRPLWIIFERDMVIRLDKEMVVNSDKTIDGRGAKVEIIN
AGFTLNGVKNVIIHNINMHDVKVNPGGLIKSNDGPAAPRAGSDGDAISISG
SSQIWIDHCSLSKSVDGLVDAKLGTTRLTVSNSLFTQHQFVLLFGAGDENI
EDRGMLATVAFNTFTDNVDQRMPRCRHGFFQVVNNNYDKWGSYAIGGS
ASPTILSQGNRFCAPDERSKKNVLGRHGEAAAESMKWNWRTNKDVLENG
AIFVASGVDPVLTPEQSAGMIPAEPGESALSLTSSAGVLSCQPGAPC

### Cedar sequences

493634 Cry j IB precursor
MDSPCLVALLVFSFVIGSCFSDNPIDSCWRGDSNWAQNRMKLADCAVGF
GSSTMGGKGGDLYTVTNSDDDPVNPPGTLRYGATRDRPLWIIFSGNMNIK
LKMPMYIAGYKTFDGRGAQVYIGNGGPCVFIKRVSNVIIHGLYLYGCSTS
VLGNVLINESFGVEPVHPQDGDALTLRTATNIWIDHNSFSNSSDGLVDVTL
TSTGVTISNNLFFNHHKVMSLGHDDAYSDDKSMKVTVAFNQFGPNCGQR
MPRARYGLVHVANNNYDPWTIYAIGGSSNPTILSEGNSFTAPNESYKKQV
TIRIGCKTSSSCSNWVWQSTQDVFYNGAYFVSSGKYEGGNIYTKKEAFNV
ENGNATPHLTQNAGVLTCSLSKRC

493632 Cry j IA precursor
MDSPCLVALLVLSFVIGSCFSDNPIDSCWRGDSNWAQNRMKLADCAVGF
GSSTMGGKGGDLYTVTNSDDDPVNPAPGTLRYGATRDRPLWIIFSGNMNI

KLKMPMYIAGYKTFDGRGAQVYIGNGGPCVFIKRVSNVIIHGLHLYGCST
SVLGNVLINESFGVEPVHPQDGDALTLRTATNIWIDHNSFSNSSDGLVDVT
LSSTGVTISNNLFFNHHKVMLLGHDDAYSDDKSMKVTVAFNQFGPNCGQ
RMPRARYGLVHVANNNYDPWTIYAIGGSSNPTILSEGNSFTAPNESYKKQ
VTIRIGCKTSSSCSNWVWQSTQDVFYNGAYFVSSGKYEGGNIYTKKEAFN
VENGNATPQLTKNAGVLTCSLSKRC

1076242 Cry j II precursor - Japanese cedar
MAMKLIAPMAFLAMQLIIMAAAEDQSAQIMLDSVVEKYLRSNRSLRKVE
HSRHDAINIFNVEKYGAVGDGKHDCTEAFSTAWQAACKNPSAMLLVPGS
KKFVVNNLFFNGPCQPHFTFKVDGIIAAYQNPASWKNNRIWLQFAKLTGF
TLMGKGVIDGQGKQWWAGQCKWVNGREICNDRDRPTAIKFDFSTGLIIQ
GLKLMNSPEFHLVFGNCEGVKIIGISITAPRDSPNTDGIDIFASKNFHLQKNT
IGTGDDCVAIGTGSSNIVIEDLICGPGHGISIGSLGRENSRAEVSYVHVNGA
KFIDTQNGLRIKTWQGGSGMASHIIYENVEMINSENPILINQFYCTSASACQ
NQRSAVQIQDVTYKNIRGTSATAAAIQLKCSDSMPCKDIKLSDISLKLTSG
KIASCLNDNANGYFSGHVIPACKNLSPSAKRKESKSHKHPKTVMVENMR
AYDKGNRTRILLGSRPPNCTNKCHGCSPCKAKLVIVHRIMPQEYYPQRWI
CSCHGKIYHP

1076241 Cry j II protein - Japanese cedar
MAMKFIAPMAFVAMQLIIMAAAEDQSAQIMLDSDIEQYLRSNRSLRKVEH
SRHDAINIFNVEKYGAVGDGKHDCTEAFSTAWQAACKKPSAMLLVPGNK
KFVVNNLFFNGPCQPHFTFKVDGIIAAYQNPASWKNNRIWLQFAKLTGFT
LMGKGVIDGQGKQWWAGQCKWVNGREICNDRDRPTAIKFDFSTGLIIQG
LKLMNSPEFHLVFGNCEGVKIIGISITAPRDSPNTDGIDIFASKNFHLQKNTI
GTGDDCVAIGTGSSNIVIEDLICGPGHGISIGSLGRENSRAEVSYVHVNGAK
FIDTQNGLRIKTWQGGSGMASHIIYENVEMINSENPILINQFYCTSASACQN
QRSAVQIQDVTYKNIRGTSATAAAIQLKCSDSMPCKDIKLSDISLKLTSGKI
ASCLNDNANGYFSGHVIPACKNLSPSAKRKESKSHKHPKTVMVKNMGAY

DKGNRTRILLGSRPPNCTNKCHGCSPCKAKLVIVHRIMPQEYYPQRWMCS
RHGKIYHP

541803 Cry j I precursor - Japanese cedar
MDSPCLVALLVLSFVIGSCFSDNPIDSCWRGDSNWAQNRMKLADCAVGF
GSSTMGGKGGDLYTVTNSDDDPVNPPGTLRYGATRDRPLWIIFSGNMNIK
LKMPMYIAGYKTFDGRGAQVYIGNGGPCVFIKRVSNVIIHGLHLYGCSTS
VLGNVLINESFGVEPVHPQDGDALTLRTATNIWIDHNSFSNSSDGLVDVTL
SSTGVTISNNLFFNHHKVMLLGHDDAYSDDKSMKVTVAFNQFGPNCGQR
MPRARYGLVHVANNNYDPWTIYAIGGSSNPTILSEGNSFTAPNESYKKQV
TIRIGCKTSSSCSNWVWQSTQDVFYNGAYFVSSGKYEGGNIYTKKEAFNV
ENGNATPQLTKNAGVLTCSLSKRC

541802 Cry j I precursor- Japanese cedar
MDSPCLVALLVFSFVIGSCFSDNPIDSCWRGDSNWAQNRMKLADCAVGF
GSSTMGGKGGDLYTVTNSDDDPVNPAPGTLRYGATRDRPLWIIFSGNMNI
KLKMPMYIAGYKTFDGRGAQVYIGNGGPCVFIKRVSNVIIHGLYLYGCST
SVLGNVLINESFGVEPVHPQDGDALTLRTATNIWIDHNSFSNSSDGLVDVT
LTSTGVTISNNLFFNHHKVMSLGHDDAYSDDKSMKVTVAFNQFGPNCGQ
RMPRARYGLVHVANNNYDPWTIYAIGGSSNPTILSEGNSFTAPNESYKKQ
VTIRIGCKTSSSCSNWVWQSTQDVFYNGAYFVSSGKYEGGNIYTKKEAFN
VENGNATPHLTQNAGVLTCSLSKRC

### Dog

### Canis sequences:

Can f 1
MKTLLLTIGFSLIAILQAQDTPALGKDTVAVSGKWYLKAMTADQEVPEKP
DSVTPMILKAQKGGNLEAKITMLTNGQCQNITVVLHKTSEPGKYTAYEGQ

RVVFIQPSPVRDHYILYCEGELHGRQIRMAKLLGRDPEQSQEALEDFREFS
RAKGLNQEILELAQSETCSPGGQ

Serum albumin fragment
EAYKSEIAHRYNDLGEEHFRGLVL

Serum albumin fragment
LSSAKERFKCASLQKFGDRAFKAWSVARLSQRFPKADFAEISKVVTDLTK
VHKECCHGDLLECADDRADLAKYMCENQDSISTKLKECCDKPVLEKSQC
LAEVERDELPGDLPSLAADFVEDKEVCKNYQEAKDVFLGTFLYEYSRRHP
EYSVSLLLRLAKEYEATLEKCCATDDPPTCYAKVLDEFKPLVDEPQNLVK
TNCELFEKLGEYGFQNALLVRYTKKAPQVSTPTLVVEVSRKLGKVGTKCC
KKPESERMSCADDFLS

Can f 2
MQLLLLTVGLALICGLQAQEGNHEEPQGGLEELSGRWHSVALASNKSDLI
KPWGHFRVFIHSMSAKDGNLHGDILIPQDGQCEKVSLTAFKTATSNKFDL
EYWGHNDLYLAEVDPKSYLILYMINQYNDDTSLVAHLMVRDLSRQQDFL
PAFESVCEDIGLHKDQIVVLSDDDRCQGSRD

Additional dog allergen protein (NCBI entrez accession):
1731859

### Horse

### Equus sequences:

1575778 Equ c1
MKLLLLCLGLILVCAQQEENSDVAIRNFDISKISGEWYSIFLASDVKEKIEE
NGSMRVFVDVIRALDNSSLYAEYQTKVNGECTEFPMVFDKTEEDGVYSL

NYDGYNVFRISEFENDEHIILYLVNFDKDRPFQLFEFYAREPDVSPEIKEEF
VKIVQKRGIVKENIIDLTKIDRCFQLRGNGVAQA

3121755 Equ c 2
SQXPQSETDYSQLSGEWNTIYGAASNIXK

### Euroglyphus (mite)

### Euroglyphus sequences:

Eur m 1 (variant)
TYACSINSVSLPSELDLRSLRTVTPIRMQGGCGSCWAFSGVASTESAYLAY
RNMSLDLAEQELVDCASQNGCHGDTIPRGIEYIQQNGVVQEHYYPYVARE
QSCHRPNAQRYGLKNYCQISPPDSNKIRQALTQTHTAVAVIIGIKDLNAFR
HYDGRTIMQHDNGYQPNYHAVNIVGYGNTQGVDYWIVRNSWDTTWGD
NGYGYFAANINL

Eur m 1 (variant)
TYACSINSVSLPSELDLRSLRTVTPIRMQGGCGSCWAFSGVASTESAYLAY
RNMSLDLAEQELVDCASQNGCHGDTIPRGIEYIQQNGVVQEHYYPYVARE
QSCHRPNAQRYGLKNYCQISPPDSNKIRQALTQTHTAVAVIIGIKDLNAFR
HYDGRTIMQHDNGYQPNYHAVNIVGYGNTQGVDYWIVRNSWDTTWGD
NGYGYFAANINL

Eur m 1 (variant)
ETNACSINGNAPAEIDLRQMRTVTPIRMQGGCGSCWAFSGVAATESAYLA
YRNQSLDLAEQELVDCASQHGCHGDTIPRGIEYIQHNGVVQESYYRYVAR
EQSCRRPNAQRFGISNYCQIYPPNANKIREALAQTHSAIAVIIGIKDLDAFR
HYDGRTIIQRDNGYQPNYHAVNIVGYSNAQGVDYWIVRNSWDTNWGDN
GYGYFAANIDL

Eur m 1 (variant)
ETSACRINSVNVPSELDLRSLRTVTPIRMQGGCGSCWAFSGVAATESAYLA
YRNTSLDLSEQELVDCASQHGCHGDTIPRGIEYIQQNGVVEERSYPYVARE
QQCRRPNSQHYGISNYCQIYPPDVKQIREALTQTHTAIAVIIGIKDLRAFQH
YDGRTIIQHDNGYQPNYHAVNIVGYGSTQGVDYWIVRNSWDTTWGDSG
YGYFQAGNNL

### Poa (grass) sequences

113562 POLLEN ALLERGEN POA P 9
MAVQKYTVALFLVALVVGPAASYAADLSYGAPATPAAPAAGYTPAAPA
GAAPKATTDEQKMIEKINVGFKAAVAAAGGVPAANKYKTFVATFGAASN
KAFAEALSTEPKGAAVDSSKAALTSKLDAAYKLAYKSAEGATPEAKYDD
YVATLSEALRIIAGTLEVHGVKPAAEEVKATPAGELQVIDKVDAAFKVAA
TAANAAPANDKFTVFEAAFNDAIKASTGGAYQSYKFIPALEAAVKQSYAA
TVATAPAVKYTVFETALKKAITAMSQAQKAAKPAAAATGTATAAVGAA
TGAATAAAGGYKV

113561 POA P 9
MAVHQYTVALFLAVALVAGPAASYAADVGYGAPATLATPATPAAPAAG
YTPAAPAGAAPKATTDEQKLIEKINAGFKAAVAAAAGVPAVDKYKTFVA
TFGTASNKAFAEALSTEPKGAAAASSNAVLTSKLDAAYKLAYKSAEGATP
EAKYDAYVATLSEALRIIAGTLEVHAVKPAGEEVKAIPAGELQVIDKVDA
AFKVAATAANAAPANDKFTVFEAAFNDAIKASTGGAYQSYKFIPALEAAV
KQSYAATVATAPAVKYTVFETALKKAITAMSQAQKAAKPAAAVTATAT
GAVGAATGAVGAATGAATAAAGGYKTGAATPTAGGYKV

113560 POA P 9

MDKANGAYKTALKAASAVAPAEKFPVFQATFDKNLKEGLSGPDAVGFA
KKLDAFIQTSYLSTKAAEPKEKFDLFVLSLTEVLRFMAGAVKAPPASKFPA
KPAPKVAAYTPAAPAGAAPKATTDEQKLIEKINVGFKAAVAAAAGVPAA
SKYKTFVATFGAASNKAFAEALSTEPKGAAVASSKAVLTSKLDAAYKLA
YKSAEGATPEAKYDAYVATLSEALRIIAGTLEVHGVKPAAEEVKAIPAGE
LQVIDKVDAAFKVAATAANAAPANDKFTVFEAAFNDAIKASTGGAYQSY
KFIPALEAAVKQSYAATVATAPAVKYTVFETALKKAITAMSQAQKAAKP
AAAVTGTATSAVGAATGAATAAAGGYKV

### Cockroach sequences

2833325 Cr p1
MKTALVFAAVVAFVAARFPDHKDYKQLADKQFLAKQRDVLRLFHRVHQ
HNILNDQVEVGIPMTSKQTSATTVPPSGEAVHGVLQEGHARPRGEPFSVN
YEKHREQAIMLYDLLYFANDYDTFYKTACWARDRVNEGMFMYSFSIAVF
HRDDMQGVMLPPPYEVYPYLFVDHDVIHMAQKYWMKNAGSGEHHSHVI
PVNFTLRTQDHLLAYFTSDVNLNAFNTYYRYYYPSWYNTTLYGHNIDRR
GEQFYYTYKQIYARYFLERLSNDLPDVYPFYYSKPVKSAYNPNLRYHNGE
EMPVRPSNMYVTNFDLYYIADIKNYEKRVEDAIDFGYAFDEHMKPHSLY
HDVHGMEYLADMIEGNMDSPNFYFYGSIYHMYHSMIGHIVDPYHKMGL
APSLEHPETVLRDPVFYQLWKRVDHLFQKYKNRLPRYTHDELAFEGVKV
ENVDVGKLYTYFEQYDMSLDMAVYVNNVDQISNVDVQLAVRLNHKPFT
YNIEVSSDKAQDVYVAVFLGPKYDYLGREYDLNDRRHYFVEMDRFPYHV
GAGKTVIERNSHDSNIIAPERDSYRTFYKKVQEAYEGKSQYYVDKGHNYC
GYPENLLIPKGKKGGQAYTFYVIVTPYVKQDEHDFEPYNYKAFSYCGVGS
ERKYPDNKPLGYPFDRKIYSNDFYTPNMYFKDVIIFHKKYDEVGVQGH

2231297 Cr p2
INEIHSIIGLPPFVPPSRRHARRGVGINGLIDDVIAILPVDELKALFQEKLETS
PDFKALYDAIRSPEFQSIISTLNAMQRSEHHQNLRDKGVDVDHFIQLIRALF

GLSRAARNLQDDLNDFLHSLEPISPRHRHGLPRQRRRSARVSAYLHADDF
HKIITTIEALPEFANFYNFLKEHGLDVVDYINEIHSIIGLPPFVPPSRRHARRG
VGINGLIDDVIAILPVDELKALFQEKLETSPDFKALYDAIRSPEFQSIISTLNA
MPEYQELLQNLRDKGVDVDHFIRVDQGTLRTLSSGQRNLQDDLNDFLALI
PTDQILAIAMDYLANDAEVQELVAYLQSDDFHKIITTIEALPEFANFYNFLK
EHGLDVVDYINEIHSIIGLPPFVPPSQRHARRGVGINGLIDDVIAILPVDELK
ALFQEKLETSPDFKALYDAIDLRSSRA

1703445 Bla g 2
MIGLKLVTVLFAVATITHAAELQRVPLYKLVHVFINTQYAGITKIGNQNFL
TVFDSTSCNVVVASQECVGGACVCPNLQKYEKLKPKYISDGNVQVKFFD
TGSAVGRGIEDSLTISNLTTSQQDIVLADELSQEVCILSADVVVGIAAPGCP
NALKGKTVLENFVEENLIAPVFSIHHARFQDGEHFGEIIFGGSDWKYVDGE
FTYVPLVGDDSWKFRLDGVKIGDTTVAPAGTQAIIDTSKAIIVGPKAYVNP
INEAIGCVVEKTTTRRICKLDCSKIPSLPDVTFVINGRNFNISSQYYIQQNGN
LCYSGFQPCGHSDHFFIGDFFVDHYYSEFNWENKTMGFGRSVE
SV

1705483 Bla g 4
AVLALCATDTLANEDCFRHESLVPNLDYERFRGSWIIAAGTSEALTQYKC
WIDRFSYDDALVSKYTDSQGKNRTTIRGRTKFEGNKFTIDYNDKGKAFSA
PYSVLATDYENYAIVEGCPAAANGHVIYVQIRFSVRRFHPKLGDKEMIQH
YTLDQVNQHKKAIEEDLKHFNLKYEDLHSTCH

2326190 Bla g 5
YKLTYCPVKALGEPIRFLLSYGEKDFEDYRFQEGDWPNLKPSMPFGKTPV
LEIDGKQTHQSVAISRYLGKQFGLSGKDDWENLEIDMIVDTISDFRAAIAN
YHYDADENSKQKKWDPLKKETIPYYTKKFDEVVKANGGYLAAGKLTWA
DFYFVAILDYLNHMAKEDLVANQPNLKALREKVLGLPAIKAWVAKRPPT
DL

Additional cockroach sequences (NCBI Entrez accession numbers):
2580504; 1580797; 1580794; 1362590; 544619; 544618; 1531589; 1580792; 1166573; 1176397; 2897849.

Allergen (general) sequences:
NCBI accession numbers
2739154; 3719257; 3703107; 3687326; 3643813; 3087805; 1864024; 1493836; 1480457; 2598976; 2598974; 1575778; 763532; 746485; 163827; 163823; 3080761; 163825; 3608493; 3581965; 2253610; 2231297; 2897849; 3409499; 3409498; 3409497; 3409496; 3409495; 3409494; 3409493; 3409492; 3409491; 3409490; 3409489; 3409488; 3409487; 3409486; 3409485; 3409484; 3409483; 3409482; 3409481; 3409480; 3409479; 3409478; 3409477; 3409476; 3409475; 3409474; 3409473; 3409472; 3409471; 3409470; 3409469; 3409468; 3409467; 3409466; 3409465; 3409464; 3409463; 3409462; 3409461; 3409460; 3409459; 3409458; 3409457; 3409456; 3318885; 3396070 ; 3367732; 1916805; 3337403; 2851457; 2851456; 1351295; 549187; 136467; 1173367; 2499810; 2498582; 2498581; 1346478; 1171009; 126608; 114091; 2506771; 1706660; 1169665; 1169531; 232086; 416898; 114922; 2497701; 1703232; 1703233; 1703233; 1703232; 3287877; 3122132; 3182907; 3121758; 3121756; 3121755; 3121746; 3121745; 3319925; 3319923; 3319921; 3319651; 3318789; 3318779; 3309647; 3309047; 3309045; 3309043; 3309041; 3309039; 3288200; 3288068; 2924494; 3256212; 3256210; 3243234; 3210053; 3210052; 3210051; 3210050; 3210049; 3210048; 3210047; 3210046; 3210045; 3210044; 3210043; 3210042; 3210041; 3210040; 3210039; 3210038; 3210037; 3210036; 3210035; 3210034; 3210033; 3210032; 3210031; 3210030; 3210029; 3210028; 3210027; 3210026; 3210025; 3210024; 3210023; 3210022; 3210021; 3210020; 3210019; 3210018; 3210017; 3210016; 3210015; 3210014; 3210013; 3210012; 3210011; 3210010; 3210009; 3210008; 3210007; 3210006; 3210005; 3210004; 3210003; 3210002; 3210001; 3210000; 3209999; 3201547; 2781152; 2392605; 2392604; 2781014; 1942360; 2554672; 2392209; 3114481; 3114480; 2981657; 3183706; 3152922 ; 3135503; 3135501; 3135499; 3135497; 2414158; 1321733; 1321731; 1321728; 1321726; 1321724; 1321722; 1321720; 1321718; 1321716; 1321714; 1321712; 3095075; 3062795; 3062793; 3062791; 2266625; 2266623; 2182106; 3044216; 2154736; 3021324; 3004467; 3005841; 3005839; 3004485; 3004473; 3004471; 3004469; 3004465; 2440053; 1805730; 2970629 ; 2959898; 2935527 ; 2935416; 809536; 730091; 585279; 584968; 2498195; 2833325; 2498604; 2498317; 2498299; 2493414; 2498586; 2498585; 2498576; 2497749; 2493446; 2493445; 1513216; 729944; 2498099; 548449; 465054; 465053; 465052; 548671; 548670; 548660; 548658; 548657; 2832430; 232084; 2500822; 2498118; 2498119; 2498119; 2498118; 1708296; 1708793; 416607; 416608; 416608; 416607; 2499791; 2498580; 2498579; 2498578; 2498577; 2497750; 1705483; 1703445; 1709542; 1709545; 1710589; 1352699; 1346568; 1346323; 1346322; 2507248; |1352240; 1352239; 1352237; 1352229; 1351935; 1350779; 1346806; 1346804; 1346803; 1170095; 1168701; 1352506; 1171011; 1171008; 1171005; 1171004; 1171002; 1171001; 1168710; 1168709; 1168708; 1168707; 1168706; 1168705; 1168704; 1168703; 1168702; 1168696; 1168391; 1168390; 1168348; 1173075; 1173074; 1173071; 1169290; 1168970; 1168402; 729764; 729320; 729979; 729970; 729315; 730050; 730049; 730048; 549194; 549193; 549192; 549191; 549190; 549189; 549188; 549185; 549184; 549183; 549182; 549181; 549180; 549179; 464471; 585290; 416731; 1169666; 113478; 113479; 113477; 113476; 113475; 130975; 119656; 113562; 113561; 113560; 416610; 126387; 126386; 126385; 132270; 416611; 416612; 416612; 416611; 730035; 127205; 1352238; 125887; 549186; 137395; 730036; 133174; 114090; 131112; 126949; 129293; 124757; 129501; 416636; 2801531; 2796177; 2796175; 2677826; 2735118; 2735116; 2735114; 2735112; 2735110; 2735108; 2735106 ; 2735104; 2735102 ; 2735100; 2735098 ; 2735096 ; 2707295 ; 2154730; 2154728; 1684720; 2580504 ; 2465137; 2465135; 2465133; 2465131; 2465129; 2465127; 2564228; 2564226; 2564224; 2564222; 2564220; 2051993; 1313972; 1313970; 1313968; 1313966; 2443824 ; 2488684; 2488683; 2488682; 2488681; 2488680; 2488679; 2488678; 2326190 ; 2464905; 2415702; 2415700; 2415698; 2398759; 2398757; 2353266 ; 2338288; 1167836; 414703; 2276458 ; 1684718; 2293571 ; 1580797 ; 1580794 ; 2245508 ; 2245060; 1261972; 2190552 ; 1881574; 511953 ; 1532058; 1532056; 1532054; 1359436; 666007; 487661; 217308; 1731859; 217306; 217304; 1545803; 1514943; 577696; 516728; 506858; 493634; 493632; 2154734; 2154732; 543659; 1086046; 1086045; 2147643; 2147642; 1086003; 1086002; 1086001; 543675; 543623; 543509; 543491; 1364099; 2147108; 2147107; 1364001; 1085628; 631913; 631912; 631911; 2147092; 477301; 543482; 345521; 542131; 542130; 542129; 100636; 2146809; 480443; 2114497; 2144915; 72355; 71728; 319828; 1082946; 1082945; 1082944; 539716; 539715; 423193; 423192; 423191; 423190; 1079187; 627190; 627189; 627188; 627187; 482382; 1362656; 627186; 627185; 627182; 482381; 85299; 85298; 2133756; 2133755; 1079186; 627181; 321044; 321043; 112559; 112558; 1362590; 2133564; 1085122; 1078971; 627144; 627143; 627142; 627141; 280576; 102835; 102834; 102833; 102832; 84703; 84702; 84700; 84699; 84698; 84696; 477888; 477505; 102575; 102572; 478272; 2130094; 629813; 629812; 542172; 542168; 542167; 481432; 320620; 280414; 626029; 542132; 320615; 320614; 100638; 100637; 100635; 82449; 320611; 320610; 280409; 320607; 320606; 539051; 539050; 539049; 539048; 322803; 280407; 100501; 100498; 100497; 100496; 1362137; 1362136; 1362135; 1362134; 1362133; 1362132; 1362131; 1362130; 1362129; 1362128; 100478; 2129891; 1076531; 1362049; 1076486; 2129817; 2129816; 2129815; 2129814; 2129813; 2129812; 2129805; 2129804; 2129802; 2129801; 2129800; 2129799; 479902; 479901; 2129477; 1076247; 629480; 1076242; 1076241; 541803; 541802; 280372; 280371; 1361968; 1361967; 1361966; 1361965; 1361964; 1361963; 1361962; 1361961; 1361960; 1361959; 320546; 2119763; 543622; 541804; 478825; 478824; 478823; 421788; 320545; 81444; 626037; 626028; 539056;483123; 481398; 481397;100733; 100732; 100639; 625532; 1083651; 322674; 322673; 81719; 81718; 2118430; 2118429; 2118428; 2118427; 419801; 419800; 419799; 419798; 282991; 100691; 322995; 322994; 101824; 626077; 414553 ; 398830 ; 1311457; 1916292 ; 1911819; 1911818; 1911659; 1911582; 467629; 467627; 467619 ; 467617 ; 915347; 1871507; 1322185; 1322183; 897645 ; 897647 ; 1850544 ; 1850542 ; 1850540 ; 288917; 452742; 1842045 ; 1839305; 1836011; 1836010; 1829900; 1829899; 1829898; 1829897; 1829896; 1829895; 1829894; 1825459 ; 1808987; 159653 ; 1773369 ; 1769849; 1769847; 608690; 1040877 ; 1040875; 1438761; 1311513; 1311512; 1311511; 1311510; 1311509; 1311689; 1246120; 1246119; 1246118; 1246117; 1246116; 1478293; 1478292; 1311642; 1174278; 1174276; 1086972; 1086974; 1086976; 1086978; 1086978; 1086976; 1086974; 1086972; 999009; 999356; 999355; 994866; 994865; 913758; 913757; 913756; 913285; 913283; 926885; 807138; 632782; 601807; 546852; 633938; 544619; 544618; 453094; 451275; 451274; 407610; 407609; 404371; 409328; 299551; 299550; 264742; 261407; 255657; 250902; 250525; 1613674; 1613673; 1613672; 1613671; 1613670; 1613304; 1613303; 1613302; 1613240; 1613239; 1613238; 1612181; 1612180; 1612179; 1612178; 1612177; 1612176; 1612175; 1612174; 1612173; 1612172; 1612171; 1612170; 1612169; 1612168; 1612167; 1612166; 1612165; 1612164; 1612163; 1612162; 1612161; 1612160; 1612159; 1612158; 1612157; 1612156; 1612155; 1612154; 1612153; 1612152; 1612151; 1612150; 1612149; 1612148; 1612147; 1612146; 1612145; 1612144; 1612143; 1612142; 1612141; 1612140; 1612139; 1093120; 447712; 447711; 447710; 1587177; 158542; 1582223; 1582222; 1531589 ; 1580792 ; 886215 ; 1545897; 1545895; 1545893; 1545891; 1545889; 1545887; 1545885; 1545883; 1545881; 1545879; 1545877; 1545875; 166486 ; 1498496 ; 1460058; 972513 ; 1009442 ; 1009440; 1009438 ; 1009436 ; 1009434 ; 7413 ; 1421808 ; 551228 ; 452606 ; 32905 ; 1377859 ; 1364213 ; 1364212 ; 395407 ; 22690 ; 22688 ; 22686 ; 22684 ; 488605 ; 17680 ; 1052817 ; 1008445 ; 1008443 ; 992612; 706811 ; 886683 ; 747852 ; 939932 ; 19003 ; 1247377 ; 1247375; 1247373; 862307 ; 312284 ; 999462 ; 999460 ; 999458 ; 587450 ; 763064; 886209 ; 1176397 ; 1173557 ; 902012 ; 997915; 997914; 997913; 997912; 997911; 997910; 99790; 997908; 997907; 997906; 997905; 997904; 997903; 997902; 997901; 997900; 997899; 997898; 997897; 997896; 997895; 997894; 997893; 997892; 910984; 910983; 910982; 910981; 511604; 169631 ; 169629 ; 169627 ; 168316 ; 168314 ; 607633 ; 555616 ; 293902 ; 485371 ; 455288 ; 166447; 166445 ; 166443 ; 166435 ; 162551 ; 160780 ; 552080 ; 156719 ; 156715 ; 515957 ; 515956 ; 515955 ; 515954 ; 515953 ; 459163 ; 166953 ; 386678 ; 169865.

In other preferred embodiments, the polypeptide antigen is an autoantigen, typically wherein the autoantigen is administered with the secondary composition, that is, is step (2) or (3). Peptide autoantigens useful in the instnat methods include, but are not limited to insulin, glutamic acid decarboxylase (64K), PM-1 and carboxypeptidase in diabetes; myelin basic protein in multiple sclerosis; rh factor in erythroblastosis fetalis; acetylcholine receptors in myasthenia gravis; thyroid receptors in Graves' Disease; basement membrane proteins in Good Pasture's syndrome; and thyroid proteins in thyroiditis.

In any regard, once selected, the peptide allergens used herein can be obtained and/or produced using a variety of methods known to those skilled in the art. In particular, the allergens can be isolated directly from native sources, using standard purification techniques. Alternatively, the allergens can be recombinantly produced using expression systems well known in the art and purified using known techniques. The peptide allergens can also be synthesized, based on described amino acid sequences or amino acid sequences derived from the DNA sequence of a nucleic acid molecule of interest, via chemical polymer syntheses such as solid phase peptide synthesis. Such methods are known to those skilled in the art. See, e.g., J. M. Stewart and J. D. Young, Solid Phase Peptide Synthesis, 2nd Ed., Pierce Chemical Co., Rockford, IL (1984) and G. Barany and R. B. Merrifield, The Peptides: Analysis, Synthesis, Biology, editors E. Gross and J. Meienhofer, Vol. 2, Academic Press, New York, (1980), pp. 3-254, for solid phase peptide synthesis techniques; and M. Bodansky, Principles of Peptide Synthesis, Springer-Verlag, Berlin (1984) and E. Gross and J. Meienhofer, Eds., The Peptides: Analysis, Synthesis, Biology, supra, Vol. 1, for classical solution synthesis.

By "T-cell-epitope-containing peptide of an antigen we mean any suitable peptide of an antigen that contains a T-cell epitope. Typically, the peptide of the antigen is a peptide that contains a single T cell epitope but it may contain two or more T cell epitopes. However, as described in more detail below, a mixture of peptides may be given. It is not necessary for each of the peptides in the mixture to be biologically active within the individual provided that one or more of them are recognised by T cells within the individual. For this to occur, the peptide or peptides will need to be presented by (restricted by) an MHC molecule expressed by the individual. Typically, depending on the antigen (allergen) the mixture of peptides may contain 3 or 4 peptides but may contain 5 or 6 or 7 or 8 or 9 or 10 or 11 or 12, or more different peptides.

It can readily be determined whether a peptide contains a T cell epitope of an antigen by using *in vitro* proliferation assays or cytokine assays, which are known in the art, such as those described in commonly owned WO 99/34826 (PCT/GB99/00080). Preferably, the peptide of the antigen (allergen) administered in step (1), that is, in the primary composition, is one that does not stimulate B cells. Typically, the peptide of the antigen (allergen) is a peptide that has between 6 and 30 amino acids, preferably between 6 and 20 amino acids, more preferably between 7 and 18 amino acids and most preferably between 10 and 14 amino acids. Preferably, the peptide of the antigen (allergen) has the same contiguous amino acid sequence as is found in the relevant portion of the native antigen (allergen).

By "peptide of an antigen" we include the meaning that the peptide is chemically derived from the polypeptide antigen, for example by proteolytic cleavage and we also include the meaning that the peptide is derived in an intellectual sense from the polypeptide antigen, for example by making use of the amino acid sequence of the polypeptide antigen and synthesising peptides based on the sequence.

By "polypeptide antigen from which the peptides in step (1) are derived" we mean a polypeptide antigen that contains the same T cell epitope present in the peptide or peptides administered in step (1), that is, in the primary composition. By "peptide" we include not only molecules in which amino acid residues are joined by peptide (-CO-NH-) linkages but also molecules in which the peptide bond is reversed. Such retro-inverso peptidomimetics may be made using methods known in the art, for example such as those described in Mézière et a/ (1997) J. Immunol. 159:3230-3237. This approach involves making pseudopeptides containing changes involving the backbone, and not the orientation of side chains. It has been shown that, at least for MHC class II and T helper cell responses, these pseudopeptides are useful. Retro-inverse peptides, which contain NH-CO bonds instead of CO-NH peptide bonds, are much more resistant to proteolysis.

Similarly, the peptide bond may be dispensed with altogether provided that an appropriate linker moiety which retains the spacing between the Cα atoms of the amino acid residues is used; it is particularly preferred if the linker moiety has substantially the same charge distribution and substantially the same planarity as a peptide bond.
It will be appreciated that the peptide may conveniently be blocked at its N- or C-terminus so as to help reduce susceptibility to exoproteolytic digestion. It is also possible to modify the structure of peptides for such purposes as increasing solubility, enhancing therapeutic or preventive efficacy, or stability (e.g., shelf life *ex vivo*, and resistance to proteolytic degradation *in vivo*). A modified peptide can be produced in which the amino acid sequence has been altered, such as by amino acid substitution, deletion, or addition, to modify immunogenicity and/or reduce allergenicity, or to which a component has been added for the same purpose. For example, the amino acid residues essential to T cell epitope function can be determined using known techniques (e.g., substitution of each residue and determination of presence or absence of T cell reactivity). Those residues shown to be essential can be modified (e.g., replaced by another amino acid whose

presence is shown to enhance T cell reactivity), as can those which are not required for T cell reactivity (e.g., by being replaced by another amino acid whose incorporation enhances T cell reactivity but does not diminish binding to relevant MHC). Another example of a modification of peptides is substitution of cysteine residues preferably with alanine, or glutamic acid, or alternatively with serine or threonine to minimize dimerization via disulfide linkages. In order to enhance stability and/or reactivity, peptides can also be modified to incorporate one or more polymorphisms in the amino acid sequence of a protein allergen resulting from natural allelic variation. Additionally, D-amino acids, non-natural amino acids or non-amino acid analogues can be substituted or added to produce a modified peptide within the scope of this invention. Furthermore, peptides can be modified using well-known polyethylene glycol (PEG) methods to produce a peptide conjugated with PEG. Modifications ofpeptides can also include reduction/alkylation (Tarr in: Methods of Protein Microcharacterization, J. E. Silver ed. Humana Press, Clifton, N.J., pp 155-194 (1986)); acylation (Tarr, supra); esterification (Tarr, supra); chemical coupling to an appropriate carrier (Mishell and Shiigi, eds, Selected Methods in Cellular Immunology, W H Freeman, San Francisco, Calif. (1980); U.S. Pat. No. 4,939,239); or mild formalin treatment (Marsh International Archives of Allergy and Applied Immunology 41: 199-215 (1971)).

Peptides may be synthesised by the Fmoc-polyamide mode of solid-phase peptide synthesis as disclosed by Lu et al (1981) J. Org. Chem. 46:3433 and references therein. Temporary N-amino group protection is afforded by the 9-fluorenylmethyloxycarbonyl (Fmoc) group. Repetitive cleavage of this highly base-labile protecting group is effected using 20% piperidine in N,N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethyl-acrylamide polymer constituted from the three monomers dimethylacrylamide (backbone-monomer), bisacryloylethylene diamine (cross linker) and acryloylsarcosine methyl ester (functionalising agent). The peptide-to-resin cleavable linked agent used is the acid-labile 4-hydroxymethyl-phenoxyacetic acid derivative. All amino acid derivatives are added as their preformed symmetrical anhydride derivatives with the exception of asparagine and glutamine, which are added using a reversed N,N-dicyclohexyl-carbodiimide/1-hydroxybenzotriazole mediated coupling procedure. All coupling and deprotection reactions are monitored using ninhydrin, trinitrobenzene sulphonic acid or isotin test procedures. Upon completion of synthesis, peptides are cleaved from the resin support with concomitant removal of side-chain protecting groups by treatment with 95% trifluoroacetic acid containing a 50% scavenger mix. Scavengers commonly used are ethanedithiol; phenol, anisole and water, the exact choice depending on the constituent amino acids of the peptide being synthesised. Trifluoroacetic acid is removed by evaporation *in vacuo,* with subsequent trituration with diethyl ether affording the crude peptide. Any scavengers present are removed by a simple extraction procedure which on lyophilisation of the aqueous phase affords the crude peptide free of scavengers. Reagents for peptide synthesis are generally available from Calbiochem-Novabiochem (UK) Ltd, Nottingham NG7 2QJ, UK. Purification may be effected by any one, or a combination of, techniques such as size exclusion chromatography, ion-exchange chromatography and (principally) reverse-phase high performance liquid chromatography. Analysis of peptides may be carried out using thin layer chromatography, reverse-phase high performance liquid chromatography, aminoacid analysis after acid hydrolysis and by fast atom bombardment (FAB) mass spectrometric analysis.

Preferably, the polypeptide antigens used herein are those wherein restriction to a MHC Class II molecule possessed by the individual can be demonstrated for the peptide and the peptide is able to induce a late phase response in an individual who possesses the said MHC Class II molecule. Such peptides, compositions of such peptides and methods of identifying such peptides are described in commonly owned International Publication WO 99/34826.

Although the peptide administered in step (1) may be a single peptide, the primary composition may conveniently comprise a plurality of T-cell-epitope containing antigens, for example, a plurality of peptides. The peptides in the composition may or may not be multiple overlapping peptides (MOPs) derived from the same polypeptide antigen. The plurality of peptides may be derived from the whole of the polypeptide antigen and therefore the peptides may span the whole of the polypeptide chain or chains of the antigen. However, they may be derived from only portions of the polypeptide antigen such that some portions of the polypeptide antigen are not represented in the plurality of peptides (for example, some peptides derived from an antigen may not be very soluble in aqueous solution and so may not be useful, and other peptides may not show restriction to MHC Class II molecules). MOPs or any peptides derived from the antigen and present in the composition can be designed by reference to the amino acid sequence of the polypeptide antigen. Typically, the MOPs are at least seven amino acid residues and more typically between around 10 to 14 amino acid residues in length. It is preferred that the peptides of MOPs have a reduced ability to bind IgE compared to longer peptides containing the same sequence. It is particularly preferred if the peptides or MOPs are substantially incapable of crosslinking IgE on the surface of a mast cell. Typically, when the MOPs overlap, the overlap is around one or two or three or four amino acid residues.

The method may be used to desensitise an individual to a polypeptide allergen. By "desensitising an individual to a polypeptide allergen" is meant inhibition or dampening of allergic tissue reactions induced by allergens in appropriately sensitised individuals. It will be appreciated that whether or not an individual is sensitive to a particular polypeptide allergen can be assessed using well known procedures such as skin prick testing with solutions of allergen extracts, induction of cutaneous late phase responses (LPRs), clinical history, allergen challenge and radio-allergosorbent test (RAST) for measurement of allergen specific IgE, and whether or not a particular individual is one who is expected to benefit from treatment may be determined by the physician based, for example, on such tests.

The method may be used to desensitise an individual to a polypeptide autoantigen. By "desensitising an individual to a polypeptide autoantigen" is meant inhibition or dampening of autologous or allogeneic tissue reactions induced by said antigens in appropriately sensitised individuals. It will be appreciated that whether or not an individual is sensitive to a particular autoantigen can be assessed using well known procedures such as clinical history, *in vitro* autoantigen challenge of peripheral blood mononuclear cells with the antigen in question, measurement of antigen-specific immunoglobulins (Ig), for example the presence in the serum of clinically significant autoantibodies or antibodies against a graft antigen, or host antigens following allograft; or the presence of visible or measurable inflammation within the tissue. For example, tissue that forms part of an allograft or the tissues of a host having received an allograft, or the central nervous system of an individual with multiple sclerosis, or insulitis in an individual with type 1 diabetes, swollen joints in an individual with rheumatoid arthritis and any other clinical test that may be used to determine autoimmune disease or graft versus host disease or host versus graft disease. Whether or not a particular individual is one who is expected to benefit from treatment may be determined by the physician based, for example, on such tests.

The composition administered in step (2) may comprise a T cell epitope of a peptide administered in step (1) and at least one T cell epitope of a different polypeptide antigen to which the individual is to be desensitised. In a preferred embodiment, the composition is a fusion of a T-cell-epitope containing peptide as defined in step (1) and a different substantially whole antigen to which the individual is to be desensitised. It may be beneficial to use a "non-anaphylactic" version of the antigen when it is an allergen. IgE recognises determinants (epitopes) on the outer surface of a whole protein. Thus, if the peptides or sequences of polypeptide to which you wish to desensitise, are hidden within the chimeric protein, for example, you may effectively sequester them from IgE, thus reducing the chance of IgE-mediated events like anaphylaxis which you may run the risk of if you were simply to administer the whole protein.

By "substantially localised" we include the meaning that upon administration or delivery to a site in the individual which has access to the immune system, at least some of the composition resides at the site for an appreciable length of time such as, for example, 24 to 48 hours and does not diffuse away or disperse to any significant extent. It will be appreciated that some of the composition may diffuse or disperse away from the site of administration or delivery but it remains substantially localised in the context of the invention if sufficient remains present at a particular site to be taken up by antigen presenting cells (APCs) and for recruited T cells to recognise both sets of epitopes in the same APC. Thus, there is preferably a "depot" produced of the composition, or local fixation at the site of administration occurs. The composition may be substantially localised by physical or chemical means. Thus, typically, the composition may be substantially localised by virtue of its molecular weight or by virtue of being present in a slow release formulation or by virtue of being conjugated to a further molecule that leads to substantial localisation at the site of administration. It will be appreciated that for many antigens, the native antigen polypeptide (such as a recombinantly produced antigen) alone is sufficient to be substantially localised when administered. However, it may be beneficial if the antigen is combined with some other agent. Typically, the composition may be a polymer of a peptide or an aggregate or an emulsion thereof. However, in each case, in the composition administered in step (2), the T cell epitope of a peptide administered in step (1) must be accessible to a T cell and the T cell epitope containing portion is kept at the site of administration, at least in sufficient amount and for a sufficient time to have the desired effect as described above.

Depot forming agents include water and mineral oil emulsions where the composition, such as a polypeptide, is solubilised in the aqueous phase prior to emulsification. Conjugates may include conjugates of the composition with a bacterial product which may stimulate uptake by an APC.

The method may be used to desensitise the individual to the polypeptide antigen from which the peptides in step (1) are derived, in which case in step (2) the individual may be administered a composition which contains the T cell epitope of a peptide administered in step (1) and further T cell epitopes of said antigen. Thus, in one embodiment a single composition is administered in step (2) which composition is the substantially whole antigen corresponding to the peptide or peptides in step (1).

In a preferred disclosure, additionally, or alternatively, the method may be used to desensitise the individual to one or more polypeptides other than the polypeptide antigen from which the peptides in step (1) are derived. Thus, in this embodiment, the composition may be one which contains a T cell epitope of a peptide administered in step (1) and which contains a T cell epitope of the one or more polypeptide antigens to which the individual is to be desensitised. Alternatively, in this embodiment, if the composition administered in step (2) does not contain a T cell epitope of the said one or more polypeptide antigens the method comprises the step of (3) further administering to the individual a composition which contains a T cell epitope of the one or more polypeptide antigens to which the individual is to be desensitised.

The composition administered in step (2) can comprises substantially whole antigen from which the peptides in step (1) are derived. In addition, the composition in step (3) can comprises substantially whole antigen to which the individual is to be desensitised.

Without being bound by any theory, we believe that the peptides administered in step (1) are able to generate a state of general hyporesponsiveness which extends not only to the antigen from which the antigen is obtained or derived, but also to other polypeptide antigens. Thus, a type of tolerance is induced by the peptide or peptides administered in step (1). While again not being bound by any theory, we believe that the presence in the composition administered in step (2) of a T cell epitope of a peptide administered in step (1) means that appropriate T cells (i.e., newly induced tolerogenic cells which are specific for it) are drawn into the environment where the composition is substantially localised. When the further composition (e.g., whole antigen to which the individual is to be desensitised in step (3)) is administered, we believe that pathogenic T cells specific for the further antigen are recruited and, at the same local site, the further-antigen-specific cells come under the influence of the tolerogenic cells and are rendered tolerant. The compositions administered in steps (2) and (3) may also contain B cell (antibody) epitopes, particularly when the compositions do not contain allergens. It is known in the art that some T cell epitopes are also B cell epitopes and that some B cell epitopes are T cell epitopes. However, although T cell epitopes are always linear determinants of a polypeptide, B cell epitopes may be non-linear, eg made up of different portions of the polypeptide. Again not being bound by any theory, we believe that antigen-specific B cells are drawn into the environment by the presence in the composition administered in step (2) and (step (3), if carried out) ofB-cell (antibody) epitopes of the polypeptide antigen, and that this is advantageous the case of desensitisation to autoimmune proteins or to transplantation allergens (such as in autoimmunity and graft-versus-host or host-versus-graft disease). It is preferred that B cell epitopes are not present, or are hidden, in the compositions administered in steps (2) or (3) in order to try to reduce IgE responses and the possibility of anaphylaxis.

In a second aspect, we provide a method of desensitising an individual to one or more polypeptide antigens the method comprising: step (1) administering to the individual a composition comprising a T-cell-epitope-containing peptide, or a course of T-cell-epitope-containing peptides, of a first antigen to which the individual has been previously exposed, in order to generate in the individual a state of hyporesponsiveness to the first antigen; then step (2) administering to the individual substantially whole antigen corresponding to the peptide or peptides in step (1); and, if the polypeptide antigen to which the individual is to be desensitised is not the substantially whole first antigen administered in step (2), the method comprises the step of (3) further administering to the individual substantially whole polypeptide antigen or antigens to which the individual is to be desensitised. Preferably, the first antigen is an allergen.

Put in another way, in a second aspect, a method is provided for desensitising an individual to one or more polypeptide antigens. The method entails, in a first step, administering to the individual a primary composition comprising a peptide antigen containing a T cell epitope, wherein the individual has been previously exposed to the antigen and the administration is carried out in a manner sufficient to generate a hyporesponsive state against the peptide antigen. Once a hyporesponsive state has been established toward the peptide antigen, or at least a shift toward desensitisation has occurred, the method entails administration of a secondary composition comprising substantially a whole antigen from which the peptide antigen was obtained or derived from. Administration of the secondary composition is carried out in such a way as to take advantage of the tolergeneic environment established by use of the primary composition, where it is now possible to establish tolerance to the whole antigen. The secondary composition can be coadministered with another, different antigen (i.e., different to both the first polypeptide antigen and the whole antigen from which it was obtained or derived), wherein it is desired to also desensitise the individual to the second antigen.

Here again, the antigen employed in step (1), that is, in the primary composition, may be any suitable antigen. Typically, it is an allergen. It is not necessary for the individual to be allergic to the antigen specified in step (1) but it may be preferred. In this regard, the individual need not be atopic and need not have generated an IgE response to the whole antigen. However, it is necessary for the individual to have been previously exposed to at least the first antigen. Typically, the individual has mounted a T cell response (Th1 or Th2) to the first antigen from which the peptides are derived, and have specific T cells (of any kind) present that may then be rendered "tolerogenic" by peptide treatment. Thus, in one embodiment, it is determined whether the individual has previously been exposed to an antigen before commencing treatment. This can be determined by measuring serum antibodies to the antigen and/or by carrying out a T cell proliferation assay and/or cytokine assay. It will be appreciated that it is preferred that the peptides administered in the first step are ones derived from a ubiquitous allergen such as house dust mite allergen or cat dander allergen or tree or grass pollen allergen in which case it may not be necessary to test the individual.

By "substantially whole antigen" or "substantially whole allergen" we include the meaning that at least 50% of the antigen or allergen molecule is present, preferably at least 70% or 80% or 90% and, most preferably, all of the antigen or allergen. The percentage of the antigen or allergen can be determined by reference to the number of amino acids in the substantially whole antigen or allergen and the number in the native antigen or allergen.

Typically, the substantially whole antigen or allergen has at least 50% of the T cell epitopes of the whole antigen or allergen, preferably at least 70% or 80% or 90% and most preferably all of the T cell epitopes of the native antigen or allergen.

In both the first and second aspects (methods) of the invention, the T-cell-epitope-containing peptide, or composition of T-cell-epitope-containing peptides, may be administered in step (1) as a single dose. However, it is preferred that the peptide or plurality of peptides is administered as a course so that an optimal state of hyporesponsiveness is achieved before administering the composition or substantially whole allergen in step (2).

In preferred embodiments, administration of the primary composition is carried out in an escalating dose regime, that is, escalating doses of the primary composition comprising the peptide or peptides can administered over a suitable period of time. Suitable escalating doses of the peptide or peptides may be determined by the person skilled in the art, for example, by carrying out symptom-free updosing which results in tolerance (i.e., reduced reactivity to whole injected antigen in the skin). The symptom tested may conveniently be an isolated late asthmatic reaction. Skin tests to whole antigen are generally a good way of assessing induction of tolerance, with or without *in vitro* T cell proliferation and/or cytokine assays.

For escalating dosage regimes, typically 5 µg of each peptide is initially administered, and then increasing doses of the primary composition are given until a cumulative dose of 90 µg has been administered. The increasing doses of peptide are typically administered with intervals of several days (e.g., 10 to 60 days, but typically 10 to 20 days) between each administration. The total cumulative dose may thus be given over a period of 8 to 16 weeks.

Accordingly, in a preferred embodiment, the suitable course of administration may be doses of 5 µg, 10 µg, 25 µg and then 50 µg of each peptide with intervals of between 3 days to 8 weeks, preferably between 10 days and 4 weeks, and more preferably around 10 to 14 days, between the doses given. The doses may start lower, such as 0.1 µg, or 1.0 µg. Conveniently, a "maintenance" dose may be administered subsequently, such as one or two or three doses of 100 µg with a dose interval of two weeks.

Typically, the lowest initial dose will be between about 0.01 to 0.1 µg, more particularly between about 0.05 to 0.1 µg. The highest initial dose may be higher for non-allergic individuals (for example in treating autoimmunity where there is no risk of anaphylaxis) and may be in the mg range, but preferably will be 100 to 500 µg.

The course of administration that gives rise to the state of hyporesponsiveness may be any suitable course. For example, in commonly owned International Publication WO 99/34826 (PCT/GB99/00080), a method is disclosed for defining the dose for therapy which involves injecting peptides into groups (cohorts) of asthmatic subjects until 50% of them develop a late asthmatic reaction (defined as a greater then 20% fall in forced expiratory volume in one second (FEV1)). From this "upper dose" it is possible to work backwards to establish the starting dose. Typically, if the 50% LAR dose is 5 µg of peptide, the initial therapy dose should be one 50^{th} of that i.e., 0.1 µg.

By "state of hyporesponsiveness to the antigen in the individual" we mean that the individual becomes hyporesponsive to the antigen, for example, as measured by a reduction in size of skin reactions to the antigen. Hyporesponsiveness can readily be determined using well-known clinical parameters. The hyporesponsive individual will contain antigen-specific T cells since their ability to proliferate in response to antigen is reduced as is their ability to secrete disease-associated cytokines such as, in the case of allergic reactions, IL-4 and IL-5 and, possibly IFN-γ. It will be appreciated that the state of hyporesponsiveness includes antigen-specific non-responsiveness i.e., tolerance.

Challenge testing, such as intradermal injection or inhaled challenge (in an asthmatic) or intraocular administration (for allergic conjunctivitis) may be used. Intradermal testing may be simplest and works on individuals with IgE-mediated allergy. For individuals who have been exposed, but are not allergic, it is preferred to use *in vitro* proliferation and/or cytokine assays to show down regulation of T cell responses.

It may be advantageous to administer, at or around the time of the administration of the secondary composition or substantially whole antigen in steps (2) and/or (3), and at a suitable dose (eg 1 ng/kg to 10 µg/kg) immunomodulating compositions, such as interleukin-10 (IL-10) or transforming growth factor-β (TGF-β) or interferon-α (IFN-α) or combination thereof may be desirable to aid the production of a hyporesponsive or tolerogenic state. A relatively high dose of IL-10 or TGF-β or IFN-α may be administered.

In relation to any of the previously disclosed methods of the invention, the polypeptide antigen to which the individual is desensitised is any one of an allergen, an autoantigen or a transplant antigen. The allergen may be any allergen disclosed herein above. In addition, the autoantigen may be any autoantigen disclosed herein above.

Thus, in a preferred embodiment of the invention, the individual is administered a course of peptides obtained or derived from the allergen to which he is to be desensitised, which leads to a state of hyporesponsiveness or tolerance in the individual to the allergen. There is a window within the state of hyporesponsiveness or tolerance which, following the first administration, takes, typically around two weeks to develop, for example between 10 and 14 days, and becomes optimal after which it is gradually lost unless further peptide or whole protein doses are administered. Further doses may improve the degree of tolerance induced as manifest by a more marked reduction in allergen reactivity following challenge test, after a course of several peptide administrations versus just one (see, for example, Figure 3). The state of hyporesponsiveness may last for different lengths of time following the administration, depending on the number of administrations of peptides, and during this time the individual is administered whole allergen corresponding to the peptides used in producing the state of hyporesponsiveness. The optimal window may be between 2 weeks and 2 months. Typically, a single administration (injection) of peptide lasts for several months in giving only 50% reactivity after four months (ie the subsequent administration (eg injection) ofpeptide(s) may give a late phase response (eg late asthmatic response) of about 50% of the original LPR, when the second administration (eg injection) is four months after the first). Typically, full reactivity (responsiveness) has returned after 8 to 12 months. However, with multiple injections and a higher overall dose of peptide, hyporesponsiveness may last longer.

Typically, a series of administrations of compositions comprising increasing doses (with or without a "maintenance" dose) of peptides are carried out over a prescribed period of time with an interval between administrations of several days (we have now shown that the optimal interval is approximately 2-8 weeks). Following peptide administration, administration of the whole protein (with or without other proteins to which the induction of hyporesponsiveness is also desirable) is carried out. This may be followed by further injections of the whole protein antigen. Finally, a challenge test may be performed in which the subject's response to challenge with the whole protein antigen is measured.

Thus, as an example and as described in more detail in the Experimental Section herein below, an individual is selected who is known to be allergic to cat dander and, in particular, the cat dander allergen, Fel d 1. The individual is administered a course of T-cell-epitope-containing peptides of the Fel d 1 allergen (for example, a composition comprising the following peptides: EICPAVKRDVDLFLTGT (SEQ ID NO. 1); LFLTGTPDEYVEQVAQY (SEQ ID NO. 2); EQVAQYKALPVVLENA (SEQ ID NO. 3); KALPVVLENARILKNCV (SEQ ID NO. 4); RILKNCVDAKMTEEDKE (SEQ ID NO. 5); KMTEEDKENALSLLDK (SEQ ID NO. 6); KENALSLLDKIYTSPL (SEQ ID NO. 7); LTKVNATEPERTAMKK (SEQ ID NO. 8); TAMKKIQDCYVENGLI (SEQ ID NO. 9); SRVLDGLVMTTISSSK (SEQ ID NO. 10); ISSSKDCMGEAVQNTV (SEQ ID NO. 11); and AVQNTVEDLKLNTLGR (SEQ ID NO. 12)).

A state of hyporesponsiveness to Fel d 1 is generated in the individual, as can be tested by, for example, skin testing the individual by intradermal injection of Fel d 1. One week after the administration of the last dose of the peptide, and while still in a state of hyporesponsiveness, the individual is administered whole Fel d 1 allergen. Typically, a small quantity of Fel d 1 is administered, for example 4 or 5 ng. Typically, this is followed by higher doses, for example 100 ng to 100 µg, and possibly more, which are typically delivered in 2 weekly intervals to allow the previously administered Fel d 1 to expand the tolerogenic T cell pool before administration of the further dose.

It will be appreciated that similar treatment regimes can be used for other antigens. If the antigen is an autoantigen it may be preferable to use higher doses, e.g., 1 to 10 mg of autoantigen.

It will be appreciated that in the methods, the whole antigen that is administered to the individual is part of the therapeutic regime and not to test sensitivity to the whole antigen. Thus, the method of desensitising the individual specifically includes the step of administering the whole antigen for the purpose of increasing the desensitivity to the antigen and not for the purpose of testing the effect of the administration of peptides alone on the sensitivity of the individual to whole antigen.

It is preferred if the method is not one which includes administration of a primary composition comprising an allergen that an individual may be seriously allergic to, for example, a primary composition comprising peptides of bee venom phospholipase A2 followed by administration of whole bee venom phospholipase A2, or a primary composition comprising peptides of peanut allergen followed by administration of whole peanut allergen.

In a more preferred disclosure, the individual is administered a course of peptide antigens to which he or she previously has been exposed and which lead to a state of hyporesponsiveness or tolerance in the individual to the antigen and then the individual is administered, while still in the state of hyporesponsiveness, the whole antigen corresponding to, that is, from which the peptide or peptides used in producing the state of hyporesponsiveness were obtained or derived from and a further, different whole antigen, e.g., an allergen. Typically, between 1 ng and 10 mg of whole antigen is administered, for example, between 10 ng and 1 mg, or between 100 ng and 100 µg, for example between 1 and 10 µg. In this way, the individual can be desensitised to the further, different whole antigen (and also to the whole antigen corresponding to the peptide even if the individual is not allergic to that whole antigen), in the sense that the individual has T cells that can recognise the whole antigen corresponding to the peptide, i.e., has "antigen-primed" T cells regardless of their Th1/Th2 phenotype.

Typically, the individual is allergic to the further, different whole antigen and it is to this antigen that the individual is to be desensitised. It will be appreciated that the method can be used to desensitise the individual to one or two or three or four or more antigens using this technique. Thus, as an example, treatment for an individual allergic to cat, house dust mite and grasses can entail administration of a primary composition comprising peptides of cat dander allergen Fel d 1, administered in a course suitable to induce a state of hyporesponsiveness to the cat dander antigen. While still in the state of hyporesponsiveness the individual is administered a secondary composition comprising whole cat allergen (Fel d 1), whole house dust mite allergen and whole grass allergen, or a plurality of secondary compositions comprising each whole allergen. By using this method the individual can be desensitised to cat allergen, house dust mite allergen and grass allergen.

It will be appreciated that it is not essential for all of the whole antigens to be administered simultaneously, although this is particularly preferred for convenience and to make best use of the tolerogenic environment. If the two different antigens are not administered simultaneously or substantially simultaneously, it is much preferred if the antigen corresponding to the peptides is administered first and the second antigen is administered at the same site as the first antigen.

It will be appreciated that it may be desirable to administer additional doses of one or more of the whole antigens. Typically, the additional doses would be administered twice weekly or monthly. Additional doses can be given if, following testing of the individual for reactivity to the antigens by e.g., antigen challenge (by measuring size of reaction) it is suggested that further doses are required.

The methods thus provide the opportunity to treat individuals who are allergic to multiple allergens in a relatively short period of time. The method is also beneficial in the treatment of individuals who are very seriously allergic to some things but more mildly allergic to others. Thus, for example, the peptide or polypepitde antigen administered in the primary composition (in step (1)) can be directed at a mild allergen and then the selected, more severe polypeptide antigen could be administered using escalating doses of that severe allergen. Thus, as an example, where the individual to be treated is mildly allergic to cat dander but is very seriously allergic to peanuts, the individual can be treated with a course of T-cell-epitope-containing peptides of cat dander allergen to induce a state of hyporesponsiveness. The individual would then have a dose of whole cat allergen at the same time as a very low dose of whole peanut allergen. The individual is subsequently administered escalating doses of whole peanut allergen together with further doses of cat allergen.

It will be appreciated that combinations of peptides and whole allergens may be selected in order to tailor the treatment of the individual depending on the needs of the individual (e.g., to which allergens the individual needs desensitising and to which allergens the individual is mildly allergic or severely allergic).

In addition, the methods, particularly the first method, can be used to desensitise the individual to an autoantigen, that is, where the selected polypeptide antigen is an autoantigen. The autoantigen may be any associated with any autoimmune disease. The following is a non-exhaustive list of autoimmune diseases and implicated autoantigens that may be used in the method of the invention: Multiple sclerosis- MBP (myelin basic protein), PLP (proteolipid protein), and/or MOG (myelin oligodendrocyte glycoprotein); Diabetes- GAD (glutamic acid decarboxylase), insulin, and/or IA-2 (a protein tyrosine phosphatase-like molecule); Rheumatoid Arthritis- collagen and/or HSP's (heat shock proteins); Thyroiditis- thyroglobulin; Systemic Lupus Erythromatosus-histone proteins and/or immunoglobulin heavy chain; Behcet's disease- Sag (S antigen from the eye), HLA-B44, HLA-B51, and/or HSP65; Coeliac Disease/Dermatitis herpetiformis- gliadin (rather than use whole gliadin, it may be useful to use a fraction of gliadin which is able to down regulate gliadin-specific T-cell proliferation. A suitable fraction may be the α fraction disclosed in Maurano et al (2001) Scand. J. Immuno/. 53:290-295); and Myasthenia gravis-acetyl choline receptor.

The methods can also be generally applied to treat Th1 type autoimmunity, such as rheumatoid arthritis, diabetes and multiple sclerosis (MS) in individuals that are not allergic. Thus, in one embodiment an individual has MS but is not allergic to cats. However, the individual will have been exposed to cat dander allergen. The individual is given a suitable course of cat dander peptides to induce a suitable hyporesponsive or tolerogenic environment to the cat dander allergen and then a secondary composition comprising cat dander peptides or substantially whole cat dander allergen is coadministered with myelin basic protein. In this way, the cat-specific T cells, present in the environment to which the myelin basic protein is injected, will down-regulate the individual's inappropriate immune response to myelin basic protein-specific cells, thereby treating the MS.

The methods can also be generally applied to treat graft or transplant rejection. For example, the transplant antigen (selected polypeptide antigen to which the individual would be desensitised to) may be a HLA-A2 molecule or other molecule encoded by an MHC gene. Approximately 50% of Caucasians express the Class I MHC molecule HLA-A2. This is a very immunogenic molecule and when a non-A2 recipient gets an A2 graft there is a strong T and B cell response to the molecule. Host T cell receptors recognize a peptide from HLA-A2 presented by their own MHC molecules and react (this is similar to Behcet's disease mentioned above where a peptide common to both the HLA-B44 and HLA-B51 molecules appears to act as an antigenic peptide). Thus, therapy with the peptide may lead to a state of successful engraftment.

Suitably, the methods may be used to desensitise to any protein that causes a pathogenic T cell response, including proteins from latex or from the mite, Trichopyton tonsurans, which cause Th1 hypersensitivity in the skin.

It will be appreciated that the methods are particularly suited for desensitising an individual to more than one polypeptide antigen simultaneously.

The route of administration used to administer any one of the primary composition, secondary composition and/or a composition comprising the first polypeptide antigen or a larger molecule containing the first polypeptide antigen can be carried out using any known technique including intradermal injection, subcutaneous injection, intramuscular injection, intravenous injection, transdermal, intranasal, oral including inhaled via the mouth, intraocular or intrathecal administration techniques.

Preferred routes of administration are intradermal, intramuscular, intravenous and subcutaneous injections, wherein intradermal or subcutaneous injection routes are particularly preferred. Another preferred route of administration is by using a transdermal particle injection technique.

It is particularly preferred that the primary compositions, secondary compositions, and any other compositions (e.g., those coadministered with the secondary compositions) are administered to substantially the same site in order to make best use of the localised, tolerogenic environment generated by administration of the polypeptide antigen from the primary composition or larger molecule containing the polypeptide antigen from the primary composition. However, the skilled artisan will understand, upon reading the instant specification, that the primary and secondary compositions can be administered to different sites, and using different administration techniques.

It will be appreciated by the skilled artisan, upon reading the instant specification, that it may be desirable to administer the secondary composition over a period of time and in a course of administration, for example, using a regime of escalating doses. In this way it is believed that the hyporesponsiveness or tolerogenic environment may be expanded. Typically, the escalating doses will fall within the range of about 1 ng to 1 mg, typically 10 ng to 100 µg, or more typically 100 ng to 10 µg.

If multiple administrations are conducted in the second step of the invention, it is preferred that the administrations are to substantially the same site in the individual. Typically, when a plurality of selected polypeptide antigens are administered in the secondary composition (or secondary compositions), the antigens are administered simultaneously, to the same site, and typically as a single combined composition. However, if multiple antigens are selected for desensitisation, it is of course possible to administer a plurlaity of different secondary compositions to two or more different sites.

Whilst it may be possible for the peptides, polypeptides, or substantially whole antigens to be presented in raw form, it is preferable to present them as a pharmaceutical formulation for use in the various compositions administered to the individual in the practice of the instant methods. A pharmaceutical formulation comprises one or more peptide, polypeptide, or substantially whole antigen as defined herein together with one or more auxiliary substances such as pharmaceutically acceptable carriers or vehicles therefor and optionally one or more other ingredients such as an excipient or stabilizer. The various carriers, vehicles, and excipients must be "acceptable" in the sense of being compatible with the other ingredients of the formulation and not deleterious to the recipient thereof. Typically, carriers or vehicles for injection, and the final formulation, are sterile and pyrogen-free.

More particularly, auxiliary substances, such as wetting or emulsifying agents, pH buffering substances and the like, may be present in the compositions of the present invention as an excipient or vehicle. These excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, although not required, that the preparation will contain a pharmaceutically acceptable excipient that serves as a stabilizer for the peptide, polypeptide, protein or other like molecules in the composition. Examples of suitable carriers that also act as stabilizers for peptides, polypeptides and proteins include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, vehicles and auxiliary substances is available in REMINGTON'S PHARMACEUTICAL SCIENCES (Mack Pub. Co., N.J. 1991).

Formulations useful in the practice of the instant methods include those suitable for oral (e.g., sublingual or inhaled), parenteral (including subcutaneous, transdermal, intradermal, intramuscular and intravenous and rectal), or transdermal administration, although the most suitable route may depend upon for example the condition and disorder of the recipient. The formulations may conveniently be presented in unit dosage form and may be prepared by any of the methods well known in the art of pharmacy. The methods disclosed herein will thus normally include the step of bringing into association a composition of the present invention as herein defined or a pharmacologically acceptable salt or solvate thereof ("active ingredient") with a carrier, vehicle or excipient which constitutes one or more accessory ingredients.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste. Formulations for inhalation may be presented in any of the ways known to be effective e.g., metered dose inhalers.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter or polyethylene glycol.

The peptides, polypeptides or substantially whole antigens/allergens of the invention may also be administered intranasally, by inhalation, orally or via injection at a dose of from 0.0001 to 1 µg/kg per dose. Preferred are doses in the region of 10 ng to 10 mg per human individual, such as 10 µg to 1 mg or 100 µg to 1 mg, advantageously about 80 µg.

Preferred unit dosage formulations are those containing an effective dose of the active ingredient as herein described, or an appropriate fraction thereof. It will be appreciated that when a course of administration of different doses of the peptide or polypeptides in step (1), that is, in the primary composition, or the composition or compositions used in step (2), that is, in the secondary composition or other compositions are to be administered, unit dosage formulations for each dose in the course may be prepared. Thus, typically, for a course of peptides or polypeptides to be administered, a unit dosage of the peptide is available for each, typically escalating, dose.

In some preferred embodiments, one or more of the primary composition, secondary composition and any further composition (e.g., the composition comprising the first polypeptide antigen or a larger molecule containing the first polypeptide antigen) can be formulated as a particulate (powdered) composition. More particularly, formulation of particles comprising the peptide, polypeptide or whole antigen can be carried out using standard pharmaceutical formulation chemistries. For example, the antigen molecules can be combined with one or more pharmaceutically acceptable excipient or vehicle to provide a suitable formulated composition.

The formulated compositions can then be prepared as particles using standard techniques, such as by simple evaporation (air drying), vacuum drying, spray drying, freeze drying (lyophilization), spray-freeze drying, spray coating, precipitation, supercritical fluid particle formation, and the like. If desired, the resultant particles can be densified using the techniques described in International Publication No. WO 97/48485.

These methods can be used to obtain nucleic acid particles having a size ranging from about 0.01 to about 250 µm, preferably about 10 to about 150 µm, and most preferably about 20 to about 60 µm; and a particle density ranging from about 0.1 to about 25 g/cm³, and a bulk density of about 0.5 to about 3.0 g/cm³, or greater.

Similarly, particles of selected adjuvants having a size ranging from about 0.1 to about 250 µm, preferably about 0.1 to about 150 µm, and most preferably about 20 to about 60 µm; a particle density ranging from about 0.1 to about 25 g/cm³, and a bulk density of preferably about 0.5 to about 3.0 g/cm³, and most preferably about 0.8 to about 1.5 g/cm³ can be obtained.

Single unit dosages or multidose containers, in which the particles may be packaged prior to use, can comprise a hermetically sealed container enclosing a suitable amount of the particles comprising the antigen of interest. The particulate compositions can be packaged as a sterile formulation, and the hermetically sealed container can be designed to preserve sterility of the composition until use in the methods of the invention. If desired, the containers can be adapted for direct use in a needleless syringe system. Such containers can take the form of capsules, foil pouches, sachets, cassettes, and the like. Appropriate needleless syringes are described herein and are otherwise known in the art.

The container in which the particles are packaged can further be labelled to identify the composition and provide relevant dosage information. In addition, the container can be labelled with a notice in the form prescribed by a governmental agency, for example the Food and Drug Administration, wherein the notice indicates approval by the agency under Federal law of the manufacture, use or sale of the antigen, adjuvant (or vaccine composition) contained therein for human administration.

Following their formation, the particulate composition (*e*.*g*., powder) can be delivered transdermally to the individual's tissue using a suitable transdermal particle injection technique. Various particle acceleration devices suitable for transdermal particle injection are known in the art, and will find use in the practice of the invention. A particularly preferred transdermal particle injection system employs a needleless syringe to fire solid drug-containing particles in controlled doses into and through intact skin and tissue. See, e.g., U.S. Patent No. 5,630,796 to Bellhouse et al. which describes a needleless syringe (also known as "the PowderJect needleless syringe device"). Other needleless syringe configurations are known in the art and are described herein. Preferably, the particulate compositions will be delivered using a needleless syringe system such as those described in International Publication Nos. WO 94/24263, WO 96/04947, WO 96/12513, and WO 96/20022. Delivery of particles from such needleless syringe systems is typically practised with particles having an approximate size generally ranging from 0.1 to 250 µm, preferably ranging from about 10-70 µm. Particles larger than about 250 µm can also be delivered from the devices, with the upper limitation being the point at which the size of the particles would cause untoward damage to the skin cells. The actual distance which the delivered particles will penetrate a target surface depends upon particle size (e.g., the nominal particle diameter assuming a roughly spherical particle geometry), particle density, the initial velocity at which the particle impacts the surface, and the density and kinematic viscosity of the targeted skin tissue. In this regard, optimal particle densities for use in needleless injection generally range between about 0.1 and 25 g/cm³, preferably between about 0.9 and 1.5 g/cm³, and injection velocities generally range between about100 and 3,000 m/sec, or greater. With appropriate gas pressure, particles having an average diameter of 10-70 µm can be accelerated through the nozzle at velocities approaching the supersonic speeds of a driving gas flow.

If desired, these needleless syringe systems can be provided in a preloaded condition containing a suitable dosage of the particles comprising the peptide, polypeptide or whole antigen of interest. The loaded syringe can be packaged in a hermetically sealed container, which may further be labelled as described above.

The powdered compositions are administered to the individual to be treated in a manner compatible with the dosage formulation, and in an amount that will be prophylactically and/or therapeutically effective. The amount of the composition to be delivered, generally in the range of from 0.5 µg/kg to 100 µg/kg of nucleic acid molecule per dose, depends on the subject to be treated. Doses for physiologically active peptides and proteins generally range from about 0.1 µg to about 20 mg, preferably 10 µg to about 3 mg. The exact amount necessary will vary depending on the age and general condition of the individual to be treated, the severity of the condition being treated, the particular preparation delivered, the site of administration, as well as other factors. An appropriate effective amount can be readily determined by one of skill in the art.

In a third aspect, we provide a therapeutic system (or, as it may be termed, a kit of parts) for desensitising an individual to one or more polypeptide antigens. The system comprises (1) a T-cell-containing peptide, or a plurality of T-cell-epitope-containing peptides, of an antigen and (2) a composition which contains the T cell epitope of a peptide as defined in (1) and further contains a T cell epitope of the one or more polypeptide antigens to which the individual is to be desensitised wherein the composition is capable of remaining substantially at the site of its administration.

Put in another way, in a third aspect, we provide a therapeutic system for desensitising an individual to one or more polypeptide antigens. The system comprises a primary composition comprising a first polypeptide antigen containing a T cell epitope, and a secondary composition comprising the first polypeptide antigen and at least one second, different antigen to which the individual is to be desensitised.

Typically, compositions capable of remaining at the site of administration are, or are formulated to be able to localise at the site of administration. Suitable compositions and formulations are described above in relation to the methods of the invention. Conveniently, the secondary composition comprises a substantially whole antigen corresponding to the peptide or peptides present in the primary composition. Preferably the polypeptide antigen is an allergen. Typically, the secondary composition comprises a T cell epitope of the first polypeptide antigen and at least one T cell epitope of a different polypeptide antigen. The secondary composition may comprise a fusion of a T-cell-epitope-containing peptide and a different substantially whole polypeptide antigen.

In a fourth aspect, we provide a therapeutic system (or, as it may be termed, a kit of parts) for desensitising an individual to one or more polypeptide antigens each of which contains a T cell epitope. The system comprises (1) one or more peptide antigens containing T cell epitope, (2) a composition which contains a T cell epitope of a peptide defined in (1), and (3) a composition which contains a T cell epitope of a different polypeptide antigen wherein the compositions defined in (2) and (3) are capable of localising at the site of their administration. Typically, the composition defined in (2) is the substantially whole antigen corresponding to the peptide or peptides as defined in (1) and the composition defined in (3) is a substantially whole antigen. Preferably, the antigen is an allergen.

In a fifth aspect, we provide a therapeutic system (or, as it may be termed, a kit of parts) for desensitising an individual to one or more polypeptide antigens, the system comprising one or more peptide antigen containing a T cell epitope and a first substantially whole antigen corresponding to the peptide antigen or antigens. Typically, the system further comprises a second substantially whole polypeptide antigen that is different to the first substantially whole allergen. Preferably, the antigen is an allergen.

Thus, the invention includes various therapeutic systems (or kits of parts) for desensitising an individual to a selected antigen (e.g., an allergen). The systems contain a peptide antigen as defined herein above and substantially whole antigen, wherein both components are to be used in a treatment regime for desensitising the individual to the antigen. Typically, the kit of parts includes instructions for using the two components in a therapeutic regime. Suitably, the peptide antigen is one which, when administered to a suitable individual gives rise to a state of hyporesponsiveness in the individual to the antigen from which it is derived. An example of this type of therapeutic system is one containing a Fel d 1 peptide such as one or more of the following peptides: EICPAVKRDVDLFLTGT (SEQ ID NO. 1); LFLTGTPDEYVEQVAQY (SEQ ID NO. 2); EQVAQYKALPVVLENA (SEQ ID NO. 3); KALPVVLENARILKNCV (SEQ ID NO. 4); RILKNCVDAKMTEEDKE (SEQ ID NO. 5); KMTEEDKENALSLLDK (SEQ ID NO. 6); KENALSLLDKIYTSPL (SEQ ID NO. 7); LTKVNATEPERTAMKK (SEQ ID NO. 8); TAMKKIQDCYVENGLI (SEQ ID NO. 9); SRVLDGLVMTTISSSK (SEQ ID NO. 10); ISSSKDCMGEAVQNTV (SEQ ID NO. 11); AVQNTVEDLKLNTLGR (SEQ ID NO. 12); and peptides substantially homologous to any one or more of SEQ ID NOs 1-12, and whole Fel d 1. Thus, the Fel d 1 peptide and the whole Fel d 1 are to be used therapeutically in a treatment regime. This type of system may optionally contain whole allergen (e.g., whole Fel d 1) to establish the effectiveness of the desensitisation using the other components.

The invention also includes a therapeutic system (or kit of parts) which contains a course of peptides of an antigen (e.g., an allergen) as defined and substantially whole antigen. The system may include a course of peptides and a course of substantially whole antigen. The course of peptides of an antigen is one which, when administered to a suitable individual, gives rise to a state of hyporesponsiveness in the individual to the antigen from which it is obtained or derived from. Typically, as described above, the course of peptides may be a course of peptides of escalating dose. In one embodiment, the therapeutic system may contain a single peptide antigen containing a T cell epitope but present separately packaged in a series of escalating concentrations or in escalating unit dosages for administration in escalating doses to the individual. In a further embodiment, the system may contain different peptide antigens either separately packaged or packaged together (e.g., present in the same sterile and pyrogen free aqueous solution). Suitably, in some embodiments, the plurality of peptides are packaged in a series of escalating concentrations or escalating unit dosages for administration in escalating doses to the individual. Other suitable combinations may be readily devised by the person skilled in the art given the teachings herein.

The allergen may also be packaged in a series of escalating concentrations or in escalating unit dosages for administration in escalating doses to the individual.

Thus, as an example, a therapeutic system for desensitising an individual to cat dander contains any one or more peptides selected from EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12), and peptides substantially homologous to any one or more of SEQ ID NOs 1-12. separately packaged in unit dosages of 5 µg, 10 µg, 25 µg and 50 µg and whole Fel d 1 antigen separately packaged in unit dosages of 1 ng to 50 µg. Suitable instructions may be provided to indicate to the physician how to administer the peptides in order to give an optimal state ofhyporesponsiveness to Fel d 1 before administration of the whole Fel d 1 allergen. Similar therapeutic systems and kits may be devised for desensitisation to other antigens.

In a preferred embodiment, the invention includes a therapeutic system for desensitising an individual to multiple antigens (e.g., multiple allergens). The system contains a peptide antigen to which the individual has been exposed previously, substantially whole antigen corresponding to the peptide, that is, from which the peptide antigen was obtained or derived, and one or more substantially whole antigens to which the individual is to be desensitised. Typically, the therapeutic system contains the peptide, peptides, or course of peptides as described above and the antigen corresponding to the peptide, or peptides. However, in addition, the therapeutic system contains the one or more whole antigens to which the individual is to be desensitised.

Thus, in one embodiment the therapeutic system is for desensitising the individual to peanut allergen. The individual has been exposed to cat dander allergen (but may not be allergic to cat dander) but is allergic to peanut allergen. The system contains a course of Fel d 1 peptides for producing a state of hyporesponsiveness, whole Fel d 1, and course of whole peanut allergen of escalating dosages.

In another embodiment the therapeutic system is for desensitising the individual to cat dander, house dust mites and grass pollen. The individual is allergic to cat dander allergen, house dust mite allergen and grass pollen. The system contains a course of Fel d 1 peptides for producing a state of hyporesponsiveness, whole Fel d 1 and whole house dust mite allergen and whole grass pollen allergen.

In a sixth aspect of the invention, we provide for the use of a T-cell epitope-containing peptide of an antigen (e.g., an allergen) to which the individual has been exposed, in the manufacture of a medicament for generating in the individual a state of hyporesponsiveness to the antigen to allow desensitisation to one or more polypeptide antigens each of which contains a T cell epitope. Preferably, the antigen is an allergen.

In a seventh aspect, we provide for the use of a composition which contains a T cell epitope of one or more polypeptide antigens to which the individual is to be desensitised, in the manufacture of a medicament for administration to an individual who has been administered a T-cell-epitope-containing peptide, or a course of T-cell-epitope-containing peptides, of an antigen, such as an allergen, to which the individual has been exposed, in order to generate in the individual a state of hyporesponsiveness to the antigen and, if the composition does not contain a T cell epitope of the peptide or peptides administered, the individual has further been administered a composition which contains said T cell epitope, wherein the compositions are substantially localised at the site of administration.

In an eighth aspect, we provide for the use of a T-cell-epitope-containing peptide of an antigen, preferably an allergen, to which the individual has been exposed, in the manufacture of a medicament for generating in the individual a state of hyporesponsiveness to the antigen to allow for desensitisation to one or more substantially whole allergens.

In a ninth aspect, we provide for the use of a substantially whole first antigen, preferably an allergen, to which an individual has been exposed, in the manufacture of a medicament for desensitising the individual to one or more polypeptide antigens wherein the individual has been administered a T-cell-epitope-containing peptide, or a course of T-cell-epitope-containing peptides, of the first antigen and subsequently the individual is administered substantially whole antigen to which the individual is to be desensitised.

In a tenth aspect, we provide for the use of a substantially whole antigen, preferably an allergen, to which an individual has been exposed in the manufacture of a medicament for desensitising the individual to the antigen wherein the individual has been administered a T-cell-epitope-containing peptide, or a course of T-cell-epitope-containing peptides, of the antigen.

### C. Experimental

Below are examples of specific embodiments for carrying out the present invention. The examples are offered for illustrative purposes only, and are not intended to limit the scope of the present invention in any way.

Efforts have been made to ensure accuracy with respect to numbers used (*e*.*g*., amounts, temperatures, etc.), but some experimental error and deviation should, of course, be allowed for.
**Figure 1****.** *Administration of peptides followed by whole protein reduces the cutaneous early phase reaction to Fel d 1*. In a placebo-controlled, double blind clinical trial, subjects were injected intradermally with whole Fel d 1 protein and the size of the reaction at 15 minutes measured (baseline). A series of injections were administered of peptides derived from the sequence of Fel d 1. The dose of peptides started at 5 µg of each peptide and increased until a cumulative dose of 90 µg had been administered. Approximately 2-4 weeks later the whole protein was injected (whole protein). 3-6 months later whole protein challenge was performed and the magnitude of the skin reaction at 15 minutes defined (outcome).
**Figure 2****.** *Administration of peptides followed by whole protein reduces the cutaneous late-phase reaction to Fel d 1*. In a placebo-controlled, double blind clinical trial, subjects were injected intradermally with whole Fel d 1 protein and the size of the reaction at 6 hours measured (baseline). A series of injections were administered of peptides derived from the sequence of Fel d 1. The dose of peptides started at 5 µg of each peptide and increased until a cumulative dose of 90 µg had been administered. Approximately 2-4 weeks later the whole protein was injected (whole protein). 3-6 months later whole protein challenge was performed and the magnitude of the skin reaction at 6 hours defined (outcome).
**Figure 3****.** Peptides from the cat allergen Fel d 1 were administered intradermally at 2 weekly intervals in the following dose schedule: 0.1 µg, 1.0 µg, 5.0 µg, 10.0 µg, 25.0 µg. No isolated late asthmatic reactions were observed (n = 8 subjects; pooled data). EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12).

### Example 1: Desensitisation to Fel d 1

In this example, in a double-blind, placebo-controlled study, 16 subjects were injected intradermally with a mixture of the peptides: EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12) (starting at 5 µg of each peptide and increasing in dose until a cumulative dose of 90 µg was achieved) derived from the sequence of the major cat allergen Fel d 1. A control group of 8 individuals received diluent alone (placebo). Subsequently (after approximately one month) they were injected intradermally with the whole protein. Finally after 3-6 months their response to intradermal challenge with the whole protein was assessed in terms of the size (in mm²) of the skin reaction measured at 15 minutes and 6 hours post injection. The reactions to the injected proteins were compared to the reaction to equivalent protein challenges performed before the peptide injection.

Figure 1 shows that injection ofpeptides followed by protein leads to a statistically significant reduction in the size of the skin response to challenge with the whole protein at 15 minutes.

Figure 2 shows that injection of peptide alone is able to cause a significant reduction in the size of the reaction to the whole protein injection, but that the effect of the peptide injections and the protein injection is even greater than the peptide injection alone, since the combination of the two markedly reduce the size of the reaction to the 3-6 month "outcome" injection measured 6 hours after protein challenge.

### Example 2: Desensitisation to house dust mite allergen, Der p 1

In this example, subjects are injected intradermally with a mixture of peptides: EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12), and peptides substantially homologous to any one or more of SEQ ID NOs 1-12. (starting at 5 µg of each peptide and increasing in dose until a cumulative dose of 90 µg is achieved) derived from the sequence of the major cat allergen Fel d 1. A control group receives diluent alone (placebo). Subsequently (after approximately one month) they are injected intradermally with the whole Fel d 1 protein which is mixed with the house dust mite protein allergen Der p 1. Finally after 3-6 months their response to intradermal challenge with the whole Fel d 1 protein and the whole Der p 1 protein is assessed in terms of the size (in mm²) of the skin reaction measured at 15 minutes and 6 hours post injection. The reactions to the injected proteins are compared to the reaction to equivalent protein challenges performed before the peptide injection.

Skin reaction to both Fel d 1 and to Der p1 are expected to be reduced at 15 minutes after injection and/or 6 hours after injection.

### Example 3: Desensitisation to house dust mite allergen, Der p 1

In this example, subjects are injected intradermally with a mixture of peptides: EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12), and peptides substantially homologous to any one or more of SEQ ID NOs 1-12. (starting at 5 µg of each peptide and increasing in dose until a cumulative dose of 90 µg is achieved) derived from the sequence of the major cat allergen Fel d 1. A control group receives diluent alone (placebo). Subsequently (after approximately one month) they are injected intradermally with an emulsion containing the Fel d 1 peptides administered previously together with a peptide from the house dust mite protein allergen Der p 1. Finally after 3-6 months their response to intradermal challenge with the whole Fel d 1 protein and the whole Der p 1 protein is assessed in terms of the size (in mm²) of the skin reaction measured at 15 minutes and 6 hours post injection. The reactions to the injected proteins are compared to the reaction to equivalent protein challenges performed before the peptide injection.

Skin reaction to both Fel d 1 and to Der p1 are expected to be reduced at 15 minutes after injection and/or 6 hours after injection.

### Example 4: Desensitisation to myelin basic protein (MBP)

In this example, subjects are injected intradermally with a mixture of peptides EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12), and peptides substantially homologous to any one or more of SEQ ID NOs 1-12. (starting at 5 µg of each peptide and increasing in dose until a cumulative dose of 90 µg is achieved) derived from the sequence of the major cat allergen Fel d 1. A control group receives diluent alone (placebo). Subsequently (after approximately one month) they are injected intradermally with the whole Fel d 1 protein which is mixed with the autoantigen myelin basic protein (MBP). Finally after 3-6 months their response to intradermal challenge with the whole Fel d 1 protein is assessed in terms of the size (in mm²) of the skin reaction measured at 15 minutes and 6 hours post injection. The induction of hyporesponsiveness to MBP is measured by assessment of symptom scores for example using the "expanded disability status scale" (Kurtze JF. Rating neurological impairment in multiple sclerosis: an expanded disability status scale. (EDSS). Neurology 33; 1444-52 (1983) and/or the "Scripps neurological rating scale" Sharrack B & Hughes RA. Clinical scales for multiple sclerosis. J. Neurol. Sci. 135; 1-9 (1996) and the use of magnetic resonance imaging (MRI) of the brain. Additionally, *in vitro* T cell responses to MBP are expected to reduce in terms of proliferative capacity and production of IFNγ. The reaction to the injected Fel d 1 protein is compared to the reaction to equivalent protein challenge performed before the peptide injection.

Skin reaction to Fel d 1 is reduced at 15 minutes after injection and/or 6 hours after injection. Furthermore, the symptoms scores recorded with the instruments listed above are expected to show an improvement associated with a reduction in reactivity to MBP.

### Example 5: Desensitisation to both cat and dog allergen

In this example, an individual with cat-induced allergic asthma and also with allergic sensitisation to dog allergen(s) was injected, intradermally with a mixture of peptides from the major cat allergen, Fel d 1. The peptides were specifically EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12). The peptides were injected in incremental divided doses at approximately 2 week intervals. The doses were administered in a 100 microlitre volume and contained the following concentrations of each of the 12 peptides: 1 µg, 5 µg, 10 µg, 25 µg, 50 µg, 100 µg and 100 µg in that order.

At various times during the treatment, the individual was also injected intradermally with whole cat dander extract 30 biological units (BU; purchased from ALK, Horsholm, Denmark) and with dog dander extract (30BU) via the same route.

The magnitude of the early-phase (15 minutes) and late-phase (6 hours) allergic skin response to both allergen extracts was measured before and 4 weeks after the course of peptide injections. The magnitude of the reactions was as follows:

**Table 1**

| Allergen | EPR (mm²) | | LPR (mm²) | |
|---|---|---|---|---|
| | Before | After | Before | After |
| Cat dander | 75 | 128 | 488 | 0 |
| | | | | |
| Dog dander | 61 | 55.3 | 644 | 0 |

In-addition to the changes in skin late-phase reactivity to the two allergens, the clinical efficacy of the peptide treatment with respect to symptoms was confirmed by improvement in nasal symptom scores following nasal allergen challenge with cat dander extract (dog allergen not done).

The results demonstrate that treatment with peptides derived from one protein can be used to induce hyporesponsiveness not only to the parent protein but to another protein, provided the whole proteins are co-administered during or shortly after administration of a course of peptides.

## Claims

1. Use of
(i) a polypeptide comprising a whole first polypeptide antigen sequence which comprises a T cell epitope or a polypeptide comprising a portion of the first polypeptide antigen sequence, which portion comprises a T cell epitope, or
(ii) a polypeptide comprising a whole second polypeptide antigen sequence or a polypeptide comprising a portion of the second polypeptide antigen sequence, which portion contains a T cell epitope,
for the manufacture of a medicament for desensitising an individual to the second polypeptide antigen by a method comprising:
(a) administering a primary composition comprising the polypeptide comprising the whole first polypeptide antigen sequence or the polypeptide comprising the portion of the first polypeptide antigen sequence to the individual in a manner sufficient to generate a hyporesponsive state against the first polypeptide antigen sequence; and
(b) administering a secondary composition to the individual, wherein said secondary composition comprises the polypeptide comprising the whole second antigen sequence or the polypeptide comprising the portion of the second polypeptide sequence
whereby the hyporesponsive state generated in step (a) and administration of the secondary composition are sufficient to desensitise the individual to the second polypeptide antigen,
and wherein the first polypeptide antigen is different from the second polypeptide antigen, and wherein the individual has previously been exposed to the first or second polypeptide antigen.

2. Use according to claim 1 wherein the individual has previously been exposed to the first polypeptide antigen, and wherein the secondary composition comprises the polypeptide comprising the whole second antigen sequence or the polypeptide comprising the portion of the second polypeptide sequence and is coadministered with the polypeptide comprising the whole first polypeptide antigen sequence or the polypeptide comprising the portion of the first polypeptide antigen sequence, whereby the hyporesponsive state generated in step (a) and said coadministration are sufficient to desensitise the individual to the second polypeptide antigen.

3. Use of claim 1 wherein a plurality of secondary compositions are administered to the individual in step (b) and each secondary composition contains a different polypeptide antigen, thereby desensitising the individual to a plurality of polypeptide antigens.

4. Use of any one of the preceding claims wherein the individual is allergic to the first polypeptide antigen or the second polypeptide antigen.

5. Use of any one of claims 1 to 4 wherein the first polypeptide antigen is an allergen.

6. Use of any one of claims 1 to 5 wherein the first polypeptide antigen or the selected polypeptide antigen is an aero allergen.

7. Use of claim 6, wherein the aero allergen is selected from the group consisting of a cat dander allergen, a dog dander allergen, a house dust mite allergen, a pollen allergen, a grass allergen, and a food allergen.

8. Use of claim 7, wherein the cat dander allergen is Fel d 1.

9. Use of any one of claims 1 to 8 wherein the primary composition comprises a plurality of first polypeptide antigens each containing a T cell epitope.

10. Use of claim 9, wherein the primary composition comprises one or more Fel d 1 peptides selected from the group consisting of EICPAVKRDVDLFLTGT (SEQ ID NO. 1), LFLTGTPDEYVEQVAQY (SEQ ID NO. 2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO. 8), TAMKKIQDCYVENGLI (SEQ ID NO. 9), SRVLDGLVMTTISSSK (SEQ ID NO. 10), ISSSKDCMGEAVQNTV (SEQ ID NO. 11), AVQNTVEDLKLNTLGR (SEQ ID NO. 12), and peptides substantially homologous to any one or more of SEQ ID NOS. 1-12.

11. Use of any one of claims 1 to 10 wherein step (a) comprises a series of administrations of the primary composition.

12. Use of claim 11, wherein the primary composition is administered to the individual in a series of escalating doses of the first polypeptide antigen carried out over a period of time.

13. Use of claims 1 to 12 wherein the second polypeptide antigen is an allergen.

14. Use of claim 13, wherein the second polypeptide antigen is an allergen from any one or more of the following sources: latex; plants such as grass, tree, and ragweed; pollens; fungi and moulds; foods; stinging insects including wasps; the chirnomidae (non-biting midges); spiders and mites; flies such as housefly, fruit fly, sheep blow fly and screw worm fly; grain weevil; silkworm; honeybee; non-biting midge larvae; bee moth larvae; mealworm; cockroach; larvae of *Tenibrio molitor* beetle; and mammals such as cat, dog, horse, cow, pig, sheep, rabbit, rat, guinea pig, mice and gerbil.

15. Use of claim 14, wherein the allergen is selected from the group consisting of Der p 1; Der p 2; Der p 3; Der p 4; Der p 5; Der p 6; Der p 7; Der p 9; Der f 1; Der f 2; Der f 3; Der f 4; Der f 7; Fel d 1 chain 1 or 2; Hev b 1; Hev b 3; Lol p 1; Lol p 2a; Lol p 3; Lol p 5a; Lol p 5b; Lol p isoform 9; Lol p 11; Ole e 1; Par j P2; Par j P5; Par j P8; Par j P9; Par j 1; Phl p 1; Phl p 2; Phl p 5; Phl p 5b; Phl p 5a; VES V 5; VES M 1; VES V 1; VES V 2; VES VI; Bet v 1; Bet v 2; Bet v 3; Bet v4; Que a I; Car b I; Aln g I; Rubisco; Ara h 1; Amb a 1; Amb a 2; Amb a 1.3; Amb a 1.2; Amb a 1.1; Cry j IB precursor, Cry j IA precursor, Cry j II precursor; Cry j II protein; Cry j I precursor; Can f 1; Can f 2; serum albumin fragment; Equ c1; Equ c 2; Eur m 1; POA P 9; Cr p1; Cr p2; Bla g 2; Bla g 4; and Bla g 5.

16. Use of any one of claims 1 to 12 wherein the second polypeptide antigen is an autoantigen.

17. Use of claim 16, wherein the autoantigen is associated with any one of the following autoimmune disorders: Multiple Sclerosis; Diabetes; Rheumatoid Arthritis; Thyroiditis; Systemic Lupus Erythematosus; Behcet's Disease; Coeliac Disease; or Myasthenia gravis.

18. Use of claim 17, wherein the autoantigen is selected from the group consisting of: myelin basic protein (MBP); proteolipid protein (PLP); myelin oligodendrocyte glycoprotein (MOG); glutamic acid decarboxylase (GAD); insulin; IA-2 (a protein phosphatase-like molecule); collagen; heat shock proteins (HSP's); thyroglobulin; histone proteins; immunoglobulin heavy chain; S antigen from the eye (Sag); HLA-B44; HLA B51; HSP65; gliadin; and acetyl choline receptor.

19. Use of any one of claims 1 to 12 wherein the second polypeptide antigen is from a transplant antigen.

20. Use of any one of claims 1 to 19 wherein the secondary composition is coadministered with the polypeptide comprising the whole first polypeptide antigen sequence or the polypeptide comprising the portion of the first polypeptide antigen sequence and said coadministration entails administration of two separate compositions.

21. Use of claim 20, wherein the secondary composition is administered to a first site and the composition comprising the polypeptide comprising the whole first polypeptide antigen sequence or the polypeptide comprising the portion of the first polypeptide antigen sequence is administered to a second site.

22. Use of claim 20, wherein the secondary composition and the composition comprising the polypeptide comprising the whole first polypeptide antigen sequence or the polypeptide comprising the portion of the first polypeptide antigen sequence are administered to the same site.

23. Use of any one of claims 1 to 22 wherein step (b) entails administration of a plurality of secondary compositions, each said secondary composition comprising a different polypeptide antigen, whereby the individual is desensitised to more than one polypeptide antigen.

24. Use of claim 23, wherein the plurality of secondary compositions are combined.

25. Use of claim 24, wherein the plurality of secondary compositions are further combined with the polypeptide comprising the whole first polypeptide antigen sequence or the polypeptide comprising the portion of the first polypeptide antigen sequence.

26. Use of claim 24, wherein the plurality of secondary compositions and the composition comprising the polypeptide comprising the whole first polypeptide antigen sequence or the polypeptide comprising the portion of the first polypeptide antigen sequence are administered to the same site.

27. Use of claim 24, wherein the secondary compositions are administered to a first site and the composition comprising the polypeptide comprising the whole first polypeptide antigen sequence or the polypeptide comprising the portion of the first polypeptide antigen sequence is administered to a second site.

28. Use of any one of claims 1 to 19 wherein in step (b) the polypeptide comprising the whole first polypeptide antigen sequence or the polypeptide comprising the portion of the first polypeptide antigen sequence is administered to the individual to an administration site and remains substantially localised at said administration site.

29. Use of claim 28, wherein the polypeptide comprising the whole first polypeptide antigen sequence or the polypeptide comprising the portion of the first polypeptide antigen sequence is administered in a slow release formulation or in an oil and water emulsion.

30. Use of any one of claims 1 to 29 wherein at least one of said primary composition, secondary composition, or a composition comprising the polypeptide comprising the whole first polypeptide antigen sequence or the polypeptide comprising the portion of the first polypeptide antigen sequence is administered using intradermal injection, subcutaenous injection, intramuscular injection, intravenous injection, transdermal, intranasal, oral, intraocular, or intrathecal administration technique.

31. Use of claim 30, wherein at least one of said primary composition, secondary composition, or a composition comprising the polypeptide comprising the whole first polypeptide antigen sequence or the polypeptide comprising the portion of the first polypeptide antigen sequence comprises an aqueous or liquid carrier or vehicle.

32. Use of claim 30, wherein at least one of said primary composition, secondary composition, or a composition comprising the polypeptide comprising the whole first polypeptide antigen sequence or the polypeptide comprising the portion of the first polypeptide antigen sequence is provided in dry powdered form.

33. Use of claim 32 wherein said dry powdered composition is administered using a transdermal particle injection technique.

34. Products containing
(i) a polypeptide comprising a whole first antigen sequence which comprises a T cell epitope or a polypeptide comprising a portion of the first polypeptide antigen sequence, which portion comprises a T cell epitope, and
(ii) a polypeptide comprising a whole second antigen sequence or a polypeptide comprising a portion of the second polypeptide antigen sequence, which portion contains a T cell epitope, as a combined preparation for simultaneous, separate or sequential use in a method of desensitising the individual to the second polypeptide antigen comprising steps (a) and (b) as defined in claim 1, and wherein the first polypeptide antigen is different from the second polypeptide antigen, and wherein the individual has previously been exposed to the first or second polypeptide antigen.

35. Products according to claim 34 wherein the primary or secondary composition is capable of remaining substantially at the site of its administration.

36. Use according to claim 1 wherein the primary and secondary composition are coadministered in the form of a single composition.

37. Use according to claim 1 wherein the primary and secondary composition are administered in the form of separate compositions.

## Patentansprüche

1. Verwendung von
(i) einem Polypeptid, umfassend eine gesamte erste Polypeptidantigensequenz, welche ein T-Zell-Epitop umfasst, oder einem Polypeptid, umfassend einen Teil der ersten Polypeptidantigensequenz, wobei dieser Teil ein T-Zell-Epitop umfasst, oder
(ii) einem Polypeptid, umfassend eine gesamte zweite Polypeptidantigensequenz, oder einem Polypeptid, umfassend einen Teil der zweiten Polypeptidantigensequenz, wobei der Teil ein T-Zell-Epitop enthält,
zur Herstellung eines Medikaments zur Desensibilisierung eines Individuums gegenüber dem zweiten Polypeptidantigen durch ein Verfahren, umfassend:
(a) Verabreichen einer primären Zusammensetzung, die das Polypeptid, umfassend die gesamte erste Polypeptidantigensequenz, oder das Polypeptid, umfassend den Teil der ersten Polypeptidantigensequenz, umfasst, an das Individuum in einer Weise, die ausreichend ist, um einen hyporeaktiven Zustand gegenüber der ersten Polypeptidantigensequenz zu erzeugen; und
(b) Verabreichen einer sekundären Zusammensetzung an das Individuum, wobei die sekundäre Zusammensetzung das Polypeptid, umfassend die gesamte zweite Antigensequenz, oder das Polypeptid, umfassend den Teil der zweiten Polypeptidsequenz, umfasst,
wodurch der hyporeaktive Zustand, der in Stufe (a) erzeugt wurde, und die Verabreichung der sekundären Zusammensetzung ausreichend sind, um das Individuum gegenüber dem zweiten Polypeptidantigen zu desensibilisieren,
und wobei das erste Polypeptidantigen von dem zweiten Polypeptidantigen verschieden ist und wobei das Individuum früher gegenüber dem ersten oder zweiten Polypeptidantigen exponiert war.

2. Verwendung nach Anspruch 1, wobei das Individuum früher gegenüber dem ersten Polypeptidantigen exponiert war und wobei die sekundäre Zusammensetzung das Polypeptid, umfassend die gesamte zweite Antigensequenz, oder das Polypeptid, umfassend den Teil der zweiten Polypeptidsequenz, umfasst und mit dem Polypeptid, umfassend die gesamte erste Polypeptidantigensequenz, oder dem Polypeptid, umfassend den Teil der ersten Polypeptidantigensequenz, co-verabreicht wird, wodurch der hyporeaktive Zustand, der in Stufe (a) erzeugt wurde, und die Co-Verabreichung ausreichend sind, um das Individuum gegenüber dem zweiten Polypeptidantigen zu desensibilisieren.

3. Verwendung nach Anspruch 1, wobei eine Vielzahl sekundärer Zusammensetzungen an das Individuum in Stufe (b) verabreicht wird und jede sekundäre Zusammensetzung ein verschiedenes Polypeptidantigen enthält, wodurch das Individuum gegenüber einer Vielzahl von Polypeptidantigenen desensibilisiert wird.

4. Verwendung gemäß einem der vorhergehenden Ansprüche, wobei das Individuum allergisch gegen das erste Polypeptidantigen oder das zweite Polypeptidantigen ist.

5. Verwendung gemäß einem der Ansprüche 1 bis 4, wobei das erste Polypeptidantigen ein Allergen ist.

6. Verwendung nach einem der Ansprüche 1 bis 5, wobei das erste Polypeptidantigen oder das ausgewählte Polypeptidantigen ein Aeroallergen ist.

7. Verwendung nach Anspruch 6, wobei das Aeroallergen ausgewählt ist aus der Gruppe, bestehend aus einem Katzenschuppen-Allergen, einem Hundeschuppen-Allergen, einem Hausstaubmilben-Allergen, einem Pollenallergen, einem Gräserallergen und einem Lebensmittelallergen.

8. Verwendung nach Anspruch 7, wobei das Katzenschuppen-Allergen Fel d 1 ist.

9. Verwendung nach einem der Ansprüche 1 bis 8, wobei die primäre Zusammensetzung eine Vielzahl von ersten Polypeptidantigenen umfasst, von denen jedes ein T-Zell-Epitop enthält.

10. Verwendung nach Anspruch 9, wobei die primäre Zusammensetzung ein oder mehrere Fel d 1-Peptid(e) umfasst, ausgewählt aus der Gruppe, bestehend aus EICPAVKRDVDLFLTGT (SEQ ID NO: 1), LFLTGTPDEYVEQVAQY (SEQ ID NO: 2), EQVAQYKALPVVLENA (SEQ ID NO: 3), KALPVVLENARILKNCV (SEQ ID NO: 4), RILKNCVDAKMTEEDKE (SEQ ID NO: 5), KMTEEDKENALSLLDK (SEQ ID NO: 6), KENALSLLDKIYTSPL (SEQ ID NO: 7), LTKVNATEPERTAMKK (SEQ ID NO: 8), TAMKKIQDCYVENGLI (SEQ ID NO: 9), SRVLDGLVMTTISSSK (SEQ ID NO: 10), ISSSKDCMGEAVQNTV (SEQ ID NO: 11), AVQNTVEDLKLNTLGR (SEQ ID NO: 12) und Peptiden, die im Wesentlichen homolog zu einem beliebigen oder mehreren von SEQ ID NO: 1-12 sind.

11. Verwendung nach einem der Ansprüche 1 bis 10, wobei die Stufe (a) eine Reihe von Verabreichungen der primären Zusammensetzung umfasst.

12. Verwendung nach Anspruch 11, wobei die primäre Zusammensetzung an das Individuum in einer Reihe von zunehmenden Dosen des ersten Polypeptidantigens, durchgeführt über eine Zeitdauer, verabreicht wird.

13. Verwendung gemäß den Ansprüchen 1 bis 12, wobei das zweite Polypeptidantigen ein Allergen ist.

14. Verwendung gemäß Anspruch 13, wobei das zweite Polypeptidantigen ein Allergen aus einer beliebigen oder mehreren der folgenden Quellen ist: Latex, Pflanzen, wie Gras, Baum und Ambrosia, Pollen, Pilze und Schimmelpilze, Lebensmittel, stechende Insekten, einschließlich Wespen, den Chironomidae ("chirnomidae") (nichtstechende Mücken), Spinnen und Milben, Fliegen, wie Stubenfliege, Fruchtfliege, Schafschmeißfliege und Schraubenwurmfliege, Kornkäfer, Seidenraupe, Honigbiene, nichtstechende Mückenlarven, Bienenmottenlarven, Mehlwurm, Schabe, Larve des *Tenibrio molitor*-Käfers und Säuger, wie Katze, Hund, Pferd, Kuh, Schwein, Schaf, Kaninchen, Ratte, Meerschweinchen, Mäuse und Wüstenrennmaus.

15. Verwendung gemäß Anspruch 14, wobei das Allergen ausgewählt ist aus der Gruppe, bestehend aus Der p 1, Der p 2, Der p 3, Der p 4, Der p 5, Der p 6, Der p 7, Der p 9, Der f 1, Der f 2, Der f 3, Der f 4, Der f 7, Fel d 1-Kette 1 oder 2, Hev b 1, Hev b 3, Lol p 1, Lol p 2a, Lol p 3, Lol p 5a, Lol p 5b, Lol p-Isoform 9, Lol p 11, Ole e 1, Par j P2, Par j P5, Par j P8, Par j P9, Par j 1, Phl p 1, Phl p 2, Phl p 5, Phl p 5b, Phl p 5a, VES V 5, VES M 1, VES V 1, VES V 2, VES VI, Bet v 1, Bet v 2, Bet v 3, Bet v 4, Que a I, Car b I, Aln g I, Rubisco, Ara h 1, Amb a 1, Amb a 2, Amb a 1.3, Amb a 1.2, Amb a 1.1, Cry j IB-Vorläufer, Cry j IA-Vorläufer, Cry j II-Vorläufer, Cry j II-Protein, Cry j I-Vorläufer, Can f 1, Can f 2, Serumalbuminfragment, Equ c 1, Equ c 2, Eur m 1, POA P 9, Cr p 1, Cr p 2, Bla g 2, Bla g 4 und Bla g 5.

16. Verwendung gemäß einem der Ansprüche 1 bis 12, wobei das zweite Polypeptidantigen ein Autoantigen ist.

17. Verwendung gemäß Anspruch 16, wobei das Autoantigen assoziiert ist mit einer der folgenden Autoimmunstörungen: multiple Sklerose, Diabetes, rheumatoide Arthritis, Thyreoiditis, systemischer Lupus erythematodes, Behcet-Erkrankung, Zöliakie oder Myasthenia gravis.

18. Verwendung nach Anspruch 17, wobei das Autoantigen ausgewählt ist aus der Gruppe, bestehend aus basischem Myelinprotein (MBP), Proteolipidprotein (PLP), Myelin-Oligodentrozyten-Glycoprotein (MOG), Glutaminsäuredecarboxylase (GAD), Insulin, IA-2 (ein Proteinphosphatase-artiges Molekül), Kollagen, Hitzeschockproteinen (HSPs), Thyroglobulin, Histonproteinen, schwerer Kette von Immunglobulin, S-Antigen aus dem Auge (Sag), HLA-B44, HLA B51, HSP65, Gliadin und Acetylcholinrezeptor.

19. Verwendung gemäß einem der Ansprüche 1 bis 12, wobei das zweite Polypeptidantigen aus einem Transplantatantigen ist.

20. Verwendung gemäß einem der Ansprüche 1 bis 19, wobei die sekundäre Zusammensetzung mit dem Polypeptid, umfassend die gesamte erste Polypeptidantigensequenz, oder dem Polypeptid, umfassend den Teil der ersten Polypeptidsequenz, co-verabreicht wird und die Co-Verabreichung eine Verabreichung zweier separater Zusammensetzungen bedingt.

21. Verwendung gemäß Anspruch 20, wobei die sekundäre Zusammensetzung an eine erste Stelle verabreicht wird und die Zusammensetzung, umfassend das Polypeptid, umfassend die gesamte erste Polypeptidantigensequenz, oder das Polypeptid, umfassend den Teil der ersten Polypeptidantigensequenz, an eine zweite Stelle verabreicht wird.

22. Verwendung gemäß Anspruch 20, wobei die sekundäre Zusammensetzung und die Zusammensetzung, umfassend das Polypeptid, umfassend die gesamte erste Polypeptidantigensequenz, oder das Polypeptid, umfassend den Teil der ersten Polypeptidantigensequenz, an die gleiche Stelle verabreicht werden.

23. Verwendung gemäß einem der Ansprüche 1 bis 22, wobei die Stufe (b) die Verabreichung einer Vielzahl von sekundären Zusammensetzungen bedingt, wobei jede sekundäre Zusammensetzung ein unterschiedliches Polypeptidantigen umfasst, wodurch das Individuum gegenüber mehr als einem Polypeptidantigen desensibilisiert wird.

24. Verwendung gemäß Anspruch 23, wobei die Vielzahl der sekundären Zusammensetzungen kombiniert wird.

25. Verwendung gemäß Anspruch 24, wobei die Vielzahl der sekundären Zusammensetzungen weiterhin mit dem Polypeptid, umfassend die gesamte erste Polypeptidantigensequenz, oder dem Polypeptid, umfassend den Teil der ersten Polypeptidantigensequenz, kombiniert wird.

26. Verwendung gemäß Anspruch 24, wobei die Vielzahl der sekundären Zusammensetzungen und die Zusammensetzung, umfassend das Polypeptid, umfassend die gesamte erste Polypeptidantigensequenz, oder das Polypeptid, umfassend den Teil der ersten Polypeptidantigensequenz, an die gleiche Stelle verabreicht werden.

27. Verwendung gemäß Anspruch 24, wobei die sekundären Zusammensetzungen an eine erste Stelle verabreicht werden, und die Zusammensetzung, umfassend das Polypeptid, umfassend die gesamte erste Polypeptidantigensequenz oder das Polypeptid, umfassend den Teil der ersten Polypeptidantigensequenz, an eine zweite Stelle verabreicht wird.

28. Verwendung gemäß einem der Ansprüche 1 bis 19, wobei in Stufe (b) das Polypeptid, umfassend die gesamte erste Polypeptidantigensequenz, oder das Polypeptid, umfassend den Teil der ersten Polypeptidantigensequenz, an das Individuum an eine Verabreichungsstelle verabreicht wird und im Wesentlichen lokalisiert an dieser Verabreichungsstelle verbleibt.

29. Verwendung gemäß Anspruch 28, wobei das Polypeptid, umfassend die gesamte erste Polypeptidantigensequenz, oder das Polypeptid, umfassend den Teil der ersten Polypeptidantigensequenz, in einer Formulierung mit langsamer Freisetzung oder in einer Öl-und-Wasser-Emulsion verabreicht wird.

30. Verwendung gemäß einem der Ansprüche 1 bis 29, wobei wenigstens eine aus der primären Zusammensetzung, der sekundären Zusammensetzung oder einer Zusammensetzung, umfassend das Polypeptid, umfassend die gesamte erste Polypeptidantigensequenz, oder das Polypeptid, umfassend den Teil der ersten Polypeptidantigensequenz, unter Verwendung von intradermaler Injektion, subkutaner Injektion, intramuskulärer Injektion, intravenöser Injektion, einer transdermalen, intranasalen, oralen, intraokularen oder intrathekalen Verabreichungstechnik verabreicht wird.

31. Verwendung gemäß Anspruch 30, wobei wenigstens eine aus der primären Zusammensetzung, der sekundären Zusammensetzung oder einer Zusammensetzung, umfassend das Polypeptid, umfassend die gesamte erste Polypeptidantigensequenz, oder das Polypeptid, umfassend den Teil der ersten Polypeptidantigensequenz, einen wässrigen oder flüssigen Träger oder ein wässriges oder flüssiges Vehikel umfasst.

32. Verwendung gemäß Anspruch 30, wobei wenigstens eine aus der primären Zusammensetzung, der sekundären Zusammensetzung oder einer Zusammensetzung, umfassend das Polypeptid, umfassend die gesamte erste Polypeptidantigensequenz, oder das Polypeptid, umfassend den Teil der ersten Polypeptidantigensequenz, in Trockenpulverform bereitgestellt wird.

33. Verwendung gemäß Anspruch 32, wobei die Trockenpulverzusammensetzung unter Verwendung einer transdermalen Partikelinjektionstechnik verabreicht wird.

34. Produkte, enthaltend
(i) ein Polypeptid, umfassend eine gesamte erste Antigensequenz, welche ein T-Zell-Epitop umfasst, oder ein Polypeptid, umfassend einen Teil der ersten Polypeptidantigensequenz, wobei der Teil ein T-Zell-Epitop umfasst, und
(ii) ein Polypeptid, umfassend eine gesamte zweite Antigensequenz, oder ein Polypeptid, umfassend einen Teil der zweiten Polypeptidantigensequenz, wobei der Teil ein T-Zell-Epitop enthält, als kombinierte Präparation zur simultanen, getrennten oder sequentiellen Verwendung in einem Verfahren zur Desensibilisierung des Individuums gegenüber dem zweiten Polypeptidantigen, umfassend die Stufen (a) und (b), wie in Anspruch 1 definiert, und wobei das erste Polypeptidantigen von dem zweiten Polypeptidantigen verschieden ist und wobei das Individuum früher gegenüber dem ersten oder dem zweiten Polypeptidantigen exponiert war.

35. Produkte gemäß Anspruch 34, wobei die primäre oder die sekundäre Zusammensetzung zum im Wesentlichen Verbleiben an der Stelle ihrer Verabreichung befähigt ist.

36. Verwendung gemäß Anspruch 1, wobei die primäre und die sekundäre Zusammensetzung in der Form einer einzelnen Zusammensetzung co-verabreicht werden.

37. Verwendung gemäß Anspruch 1, wobei die primäre und die sekundäre Zusammensetzung in der Form getrennter Zusammensetzungen verabreicht werden.

## Revendications

1. Utilisation de
(i) un polypeptide comprenant une séquence entière d'un premier antigène polypeptidique qui comprend un épitope de lymphocyte T ou un polypeptide comprenant une portion de la séquence du premier antigène polypeptidique, laquelle portion comprend un épitope de lymphocyte T, ou
(ii) un polypeptide comprenant une séquence entière d'un second antigène polypeptidique ou un polypeptide comprenant une portion de la séquence du second antigène polypeptidique, laquelle portion contient un épitope de lymphocyte T,
pour la fabrication d'un médicament destiné à désensibiliser un individu au second antigène polypeptidique par une méthode comprenant :
(a) l'administration d'une composition primaire comprenant le polypeptide comprenant la séquence entière du premier antigène polypeptidique ou le polypeptide comprenant la portion de la séquence du premier antigène polypeptidique à un individu de manière suffisante pour générer un état d'hyporéponse à la séquence du premier antigène polypeptidique ; et
(b) l'administration d'une composition secondaire à l'individu, dans laquelle ladite composition secondaire comprend le polypeptide comprenant la séquence entière du second antigène ou le polypeptide comprenant la portion de la séquence du second antigène polypeptidique
ce par quoi l'état d'hyporéponse généré à l'étape (a) et l'administration de la composition secondaire sont suffisants pour désensibiliser l'individu au second antigène polypeptidique,
et dans laquelle le premier antigène polypeptidique est différent du second antigène polypeptidique et dans laquelle l'individu a précédemment été exposé au premier ou au second antigène polypeptidique.

2. Utilisation selon la revendication 1 dans laquelle l'individu a précédemment été exposé au premier antigène polypeptidique et dans laquelle la composition secondaire comprend le polypeptide comprenant la séquence entière du second antigène ou le polypeptide comprenant la portion de la séquence du second polypeptide et est co-administrée avec le polypeptide comprenant la séquence entière du premier antigène polypeptidique ou le polypeptide comprenant la portion de la séquence du premier antigène polypeptidique, ce par quoi l'état d'hyporéponse généré à l'étape (a) et ladite co-administration sont suffisants pour désensibiliser l'individu au second antigène polypeptidique.

3. Utilisation selon la revendication 1, dans laquelle une pluralité de compositions secondaires sont administrées à l'individu à l'étape (b) et chaque composition secondaire contient un antigène polypeptidique différent, désensibilisant ainsi l'individu à une pluralité d'antigènes polypeptidiques.

4. Utilisation de l'une quelconque des revendications précédentes dans laquelle l'individu est allergique au premier antigène polypeptidique ou au second antigène polypeptidique.

5. Utilisation de l'une quelconque des revendications 1 à 4 dans laquelle le premier antigène polypeptidique est un allergène.

6. Utilisation de l'une quelconque des revendications 1 à 5 dans laquelle le premier antigène polypeptidique ou l'antigène polypeptidique choisi est un aéro-allergène.

7. Utilisation de la revendication 6, dans laquelle l'aéro-allergène est choisi parmi le groupe constitué d'un allergène de squames de chat, un allergène de squames de chien, un allergène d'acarien, un allergène de pollen, un allergène de graminée et un allergène alimentaire.

8. Utilisation de la revendication 7, dans laquelle l'allergène de squames de chat est Fel d 1.

9. Utilisation de l'une quelconque des revendications 1 à 8 dans laquelle la composition primaire comprend une pluralité de premiers antigènes polypeptidiques contenant chacun un épitope de lymphocyte T.

10. Utilisation de la revendication 9, dans laquelle la composition primaire comprend un ou plusieurs peptides Fel d 1 choisis dans le groupe constitué de EICPAVKRDVDLFLTGT (SEQ ID NO.1), LFLTGTPDEYVEQVAQY (SEQ ID NO.2), EQVAQYKALPVVLENA (SEQ ID NO. 3), KALPVVLENARILKNCV (SEQ ID NO. 4), RILKNCVDAKMTEEDKE (SEQ ID NO. 5), KMTEEDKENALSLLDK (SEQ ID NO. 6), KENALSLLDKIYTSPL (SEQ ID NO. 7), LTKVNATEPERTAMKK (SEQ ID NO.8), TAMKKIQDCYVENGLI (SEQ ID NO. 9), SRVLDGLVMTTISSSK (SEQ ID NO. 10), ISSSKDCMGEAVQNTV (SEC ID NO.11), AVQNTVEDLKLNTLGR (SEQ ID NO. 12),
et des peptides sensiblement homologues à l'une quelconque ou plusieurs parmi SEQ ID NO 1-12.

11. Utilisation de l'une quelconque des revendications 1 à 10 dans laquelle l'étape (a) comprend une série d'administrations de la composition primaire.

12. Utilisation de revendication 11, dans laquelle la composition primaire est administrée à l'individu en une série de doses croissantes du premier antigène polypeptidique dans un intervalle de temps.

13. Utilisation des revendications 1 à 12 dans laquelle le second antigène polypeptidique est un allergène.

14. Utilisation de la revendication 13, dans laquelle le second antigène polypeptidique est un allergène de l'une quelconque ou de plusieurs des sources suivantes : latex ; végétaux tels que graminée, arbre et ambroisie ; pollens ; champignons et moisissures ; aliments ; insectes piqueurs incluant les guêpes ; Chirnomidae (« moucherons non piqueurs ») ; araignées et papillons nocturnes ; mouches telles que mouche domestique, drosophile, mouche verte (*Lucilla*) et lucilie bouchère ; calandre des grains ; ver à soie ; abeille ; larves de Chirnomidae ; larves de teigne de la ruche ; larves du ver de la farine ; blatte ; larves de ténébrion meunier ; et mammifères tels que chat, chien, cheval, vache, porc, mouton, lapin, rat, cobaye, souris et gerbille.

15. Utilisation de la revendication 14, dans laquelle l'allergène est choisi dans le groupe constitué de Der p 1 ; Der p 2 ; Der p 3 ; Der p 4 ; Der p 5 ; Der p 6 ; Der p 7 ; Der p 9 ; Der f 1 ; Der f 2 ; Der f 3 ; Der f 4 ; Der f 7 ; chaîne 1 ou 2 de Fel d 1 ; Hev b 1 ; Hev b 3 ; Lol p 1 ; Lol p 2a ; Lol p 3 ; Lol p 5a ; Lol p 5b ; Lol p isoforme 9 ; Lol p 11 ; Ole e 1 ; Par j P 2 ; Par j P 5 ; Par j P 8 ; Par j P 9 ; Par j 1 ; Phl p 1 ; Phl p 2 ; Phl p 5 ; Phl p 5b ; Phl p 5a ; VES V 5 ; VES M 1 ; VES V 1 ; VES V 2 ; VES VI ; Bet v 1 ; Bet v 2 ; Bet v 3 ; Bet v 4 ; Que a I ; Car b I ; Aln g I ; Rubisco ; Ara h I ; Amb a 1 ; Amb a 2 ; Amb a 1.3 ; Amb a 1.2 ; Amb a 1.1 ; précurseur de Cry j IB ; précurseur de Cry j IA ; précurseur de Cry j II ; protéine Cry j II ; précurseur de Cry j I ; Can f 1 ; Can f 2 ; fragment de sérum-albumine ; Equ c1 ; Equ c2 ; Eur m 1 ; POA P 9 ; Cr p1 ; Cr p2 ; Bla g2 ; Bla g4 ; et Bla g5.

16. Utilisation de l'une quelconque des revendications 1 à 12 dans laquelle le second antigène polypeptidique est un auto-antigène.

17. Utilisation de la revendication 16, dans laquelle l'auto-antigène est associé à l'un quelconque des troubles auto-immuns suivants : sclérose en plaques ; polyarthrite rhumatoïde ; thyroïdite ; lupus érythémateux systémique ; maladie de Behçet ; maladie coeliaque ; ou myasthénie grave.

18. Utilisation de la revendication 17, dans laquelle l'auto-antigène est choisi dans le groupe constitué de : protéine basique de la myéline (MBP) ; protéine protéolipidique (PLP) ; glycoprotéine de myéline et d'oligodendrocyte (MOG) ; décarboxylase de l'acide glutamique (GAD) ; insuline ; IA-2 (molécule de type protéine phosphatase) ; collagène ; protéines de choc thermique (HSP) ; thyroglobuline ; protéines d'histones ; chaîne lourde d'immunoglobuline ; antigène S rétinien (Sag) ; HLA-B44 ; HLA B51 ; HSP65 ; gliadine ; et récepteur de l'acétylcholine.

19. Utilisation de l'une quelconque des revendications 1 à 12 dans laquelle le second antigène polypeptidique provient d'un antigène de transplant.

20. Utilisation de l'une quelconque des revendications 1 à 19 dans laquelle la composition secondaire est co-administrée avec le polypeptide comprenant la séquence entière du premier antigène polypeptidique ou le polypeptide comprenant la portion de la séquence du premier antigène polypeptidique et ladite co-administration comporte l'administration de deux compositions séparées.

21. Utilisation de la revendication 20, dans laquelle la composition secondaire est administrée à un premier site et la composition comprenant le polypeptide comprenant la séquence entière du premier antigène polypeptidique ou le polypeptide comprenant la portion de la séquence du premier antigène polypeptidique est administrée à un second site.

22. Utilisation de la revendication 20, dans laquelle la composition secondaire et la composition comprenant le polypeptide comprenant la séquence entière du premier antigène polypeptidique ou le polypeptide comprenant la portion de la séquence du premier antigène polypeptidique sont administrées au même site.

23. Utilisation de l'une quelconque des revendications 1 à 22 dans laquelle l'étape (b) comporte l'administration d'une pluralité de compositions secondaires, chaque dite composition secondaire comprenant un antigène polypeptidique différent, ce par quoi l'individu est désensibilisé à plus d'un antigène polypeptidique.

24. Utilisation de la revendication 23, dans laquelle la pluralité de compositions secondaires sont combinées.

25. Utilisation de la revendication 24, dans laquelle la pluralité de compositions secondaires sont en outre combinées avec le polypeptide comprenant la séquence entière du premier antigène polypeptidique ou le polypeptide comprenant la portion de la séquence du premier antigène polypeptidique.

26. Utilisation de la revendication 24, dans laquelle la pluralité de compositions secondaires et la composition comprenant le polypeptide comprenant la séquence entière du premier antigène polypeptidique ou le polypeptide comprenant la portion de la séquence du premier antigène polypeptidique sont administrées au même site.

27. Utilisation de la revendication 24, dans laquelle les compositions secondaires sont administrées à un premier site et la composition comprenant le polypeptide comprenant la séquence entière du premier antigène polypeptidique ou le polypeptide comprenant la portion de la séquence du premier antigène polypeptidique est administrée au second site.

28. Utilisation de l'une quelconque des revendications 1 à 19 dans laquelle à l'étape (b) le polypeptide comprenant la séquence entière du premier antigène polypeptidique ou le polypeptide comprenant la portion de la séquence du premier antigène polypeptidique est administré à l'individu à un site d'administration et reste sensiblement localisé audit site d'administration.

29. Utilisation de la revendication 28, dans laquelle le polypeptide comprenant la séquence entière du premier antigène polypeptidique ou le polypeptide comprenant la portion de la séquence du premier antigène polypeptidique est administré dans une formulation à libération retardée ou dans une émulsion huile et eau.

30. Utilisation de l'une quelconque des revendications 1 à 29 dans laquelle au moins une parmi ladite composition primaire, composition secondaire, ou une composition comprenant le polypeptide comprenant la séquence entière du premier polypeptide antigénique ou le polypeptide comprenant la portion de la séquence du premier antigène polypeptidique est administrée en utilisant une injection intradermique, injection sous-cutanée, injection intramusculaire, injection intraveineuse, méthode d'administration transdermique, intranasale, orale, intra-oculaire ou intrathécale.

31. Utilisation de la revendication 30, dans laquelle au moins une parmi ladite composition primaire, composition secondaire, ou une composition comprenant le polypeptide comprenant la séquence entière du premier antigène polypeptidique ou le polypeptide comprenant la portion de la séquence du premier antigène polypeptidique comprend un porteur ou véhicule aqueux ou liquide.

32. Utilisation de la revendication 30, dans laquelle au moins une parmi ladite composition primaire, composition secondaire, ou une composition comprenant le polypeptide comprenant la séquence entière du premier antigène polypeptidique ou le polypeptide comprenant la portion de la séquence du premier antigène polypeptidique est fournie sous forme de poudre sèche.

33. Utilisation de la revendication 32 dans laquelle ladite composition de poudre sèche est administrée en utilisant une technique d'injection transdermique de particules.

34. Produits contenant
(i) un polypeptide comprenant une séquence entière d'un premier antigène qui comprend un épitope de lymphocyte T ou un polypeptide comprenant une portion de la séquence du premier antigène polypeptidique, laquelle portion comprend un épitope de lymphocyte T, et
(ii) un polypeptide comprenant une séquence entière d'un second antigène ou un polypeptide comprenant une portion de la séquence du second antigène polypeptidique, laquelle portion contient un épitope de lymphocyte T, en tant que préparation combinée pour une utilisation simultanée, séparée ou séquentielle dans une méthode de désensibilisation de l'individu au second antigène polypeptidique comprenant les étapes (a) et (b) telles que définies dans la revendication 1, et dans laquelle le premier antigène polypeptidique est différent du second antigène polypeptidique et dans laquelle l'individu a précédemment été exposé au premier ou au second antigène polypeptidique.

35. Produits selon la revendication 34 dans lesquels la composition primaire ou secondaire est capable de rester sensiblement au site de son administration.

36. Utilisation selon la revendication 1 dans laquelle les compositions primaire et secondaire sont co-administrées sous la forme d'une seule composition.

37. Utilisation selon la revendication 1 dans laquelle les compositions primaire et secondaire sont administrées sous la forme de compositions séparées.
